# EUROPEAN PATENT SPECIFICATION

(11) **EP 3 512 533 B1**
(45) Date of publication and mention of the grant of the patent: **16.09.2020**
(21) Application number: 17726866.1
(22) Date of filing: 18.05.2017
(51) Int. Cl.: A61K 35/761, A61K 38/47, A61P 35/00, A61P 35/04

(54) **ONCOLYTIC VIRAL VECTORS FOR USE IN THE TREATMENT OF RETINOBLASTOMA**
ONKOLYTISCHE VIRUSVEKTOREN ZUR VERWENDUNG BEI DER BEHANDLUNG VON RETINOBLASTOM
VECTEURS VIRAUX ONCOLYTIQUES POUR UNE UTILISATION DANS LE TRAITEMENT D'UN RÉTINOBLASTOME

(30) Priority: 17.11.2016 ES 201631473
(43) Date of publication of application: 24.07.2019
(73) Proprietor: VCN Biosciences SL, 08022 Barcelona (ES); Hospital Sant Joan de Deu, 08950 Esplugues de Llobregat Barcelona (ES)
(72) Inventor: CASCALLO PIQUERAS, Manuel Maria, 08790 Gelida (Barcelona) (ES); BAZAN PEREGRINO, Miriam, 08028 Barcelona (ES); MONTERO CARCABOSO, Angel, 08174 Sant Cugat del Valles (Barcelona) (ES); CHANTADA, Guillermo Luis, Buenos Aires 1428 (AR)
(74) Representative: Durán-Corretjer, S.L.P.
(86) International application number: PCT/EP2017/061961
(87) International publication number: WO 2018/091151

(56) References cited:
- EP-A1- 2 428 229
- JI X ET AL: "Oncolytic adenovirus delivering herpes simplex virus thymidine kinase suicide gene reduces the growth of human retinoblastoma in an in vivo mouse model", EXPERIMENTAL EYE RESEARCH, ACADEMIC PRESS LTD, LONDON, vol. 89, no. 2, 1 August 2009 (2009-08-01) , pages 193-199, XP026336934, ISSN: 0014-4835, DOI: 10.1016/J.EXER.2009.03.007 [retrieved on 2009-03-28]
- XIN SONG ET AL: "Combined Treatment with an Oncolytic Adenovirus and Antitumor Activity of Vincristine against Retinoblastoma Cells", INTERNATIONAL JOURNAL OF MOLECULAR SCIENCES, vol. 13, no. 12, 27 August 2012 (2012-08-27), pages 10736-10749, XP055394331, DOI: 10.3390/ijms130910736
- GANESH S: "Intratumoral coadmisinistration of hyaluronidase inzyme and oncolytic adenoviruses inhances virus potency in metastatic tumor models", CLINICAL CANCER RESE, THE AMERICAN ASSOCIATION FOR CANCER RESEARCH, US, vol. 14, no. 12, 1 January 2008 (2008-01-01), pages 3933-3941, XP009153332, ISSN: 1078-0432

## Description

The present invention relates to the sector of medicine, more specifically the sector of oncology.

Retinoblastoma is the most common malignant intraocular tumour in children, with an incidence of 1/17,000 newborns. It develops in the retina, usually growing therebelow towards the vitreous cavity, from cells that have variants predisposed to cancer in both copies of the Rb1 gene, whether by inherited or acquired mutation. It is a disease that normally occurs before the age of five years and can be sporadic or hereditary, if there is a germ line mutation of the Rb1 gene.

Depending on its presentation, retinoblastoma can be unifocal or multifocal and unilateral (representing 65% of cases) or bilateral (representing the remaining 35% - with germ line mutation of the Rb1 gene). Unilateral retinoblastoma only occurs in one of the patient's eyes, and the average age at the time of diagnosis is 24 months, whereas bilateral retinoblastoma affects both eyes, with an average age at the time of diagnosis of 15 months.

Current treatment of retinoblastoma is aimed primarily at the patient's survival and secondly at preservation of sight. The optimum treatment is usually complex and involves experts from multiple disciplines of medicine, such as ophthalmologists, paediatric oncologists and radiation oncologists, amongst others. There are various therapeutic options, including especially: systemic or local ocular chemotherapy, radiotherapy, cryotherapy, laser therapy and surgery (enucleation of the affected eye). The choice of therapeutic regimen depends on many factors, such as the stage of tumour development, whether the tumour is unifocal or multifocal and unilateral or bilateral, the site and size of the tumour, amongst others. Because of the impact on the eye and its functions, the preferred therapy or treatment of choice is normally chemotherapy. There is, however, the problem that high-dose chemotherapy is highly toxic and in a percentage of cases the tumour develops resistance to said treatment. In these cases, the only therapeutically viable option to preserve the patient's life is currently enucleation.

In addition, although retinoblastoma as such is a very curable disease, there is a fundamental risk of the development of metastases, secondary malignancies or trilateral retinoblastoma. In these cases, the prognosis is less favourable because there are no clear and effective therapeutic options for treatment.

Gene therapy and virotherapy use viruses for therapeutic purposes against cancer. In gene therapy the virus is modified so as to prevent it from replicating and to serve as a vehicle or vector for therapeutic genetic material. In contrast, virotherapy uses viruses that replicate and propagate selectively in the tumour cells. In virotherapy the tumour cell dies through the cytopathic effect caused by the replication of the virus inside it rather than through the effect of a therapeutic gene. The preferential replication in a tumour cell is called oncotropism and tumour lysis is called oncolysis. Strictly speaking, viruses that replicate selectively in tumours are called oncolytic, although in a wider meaning the word oncolytic can be applied to any replicating virus that is able to lyse tumour cells, even non-selectively. In this description the term oncolytic is used with both meanings.

Cancer virotherapy far predates gene therapy. The first observations of tumours being cured with viruses date back to the beginning of the last century. As early as 1912 De Pace achieved tumour regression after inoculating the rabies virus into cervical carcinomas. Since then, many types of virus have been injected into tumours to treat them. There are viruses that have natural oncotropism, such as the autonomous parvovirus, the vesicular stomatitis virus and the reovirus. Other viruses can be genetically manipulated so that they replicate selectively in tumours. For example, the herpes simplex virus (HSV) has been made oncotropic by eliminating the ribonucleotide reductase gene, a dispensable enzymatic activity in actively proliferating cells such as tumour cells. Adenovirus, however, has been the virus most frequently used in both virotherapy and gene therapy for cancer because of its low pathogenicity and high ability to infect tumour cells.

Fifty-one serotypes of human adenoviruses have been identified, classified into six groups, differentiated from A to F.

Human adenovirus serotype 5 (Ad5), which belongs to group C, is a virus formed by an icosahedral protein capsid containing a 36-kilobase linear deoxyribonucleic acid (DNA). In adults, Ad5 infection is usually asymptomatic and in children it causes common cold and conjunctivitis. Ad5 generally infects epithelial cells - the cells of the bronchial epithelium during a natural infection. It enters the cell by means of the interaction of the fibre, a viral protein that extends like an antenna from the twelve vertices of the capsid, with a cell protein involved in intercellular adhesion, called the Coxsackie-Adenovirus Receptor (CAR). When viral DNA reaches the interior of the nucleus it starts systematically transcribing the early genes (E1 to E4) of the virus. The first viral genes to be expressed correspond to early region genes 1A (E1A). E1A binds to the cell protein of the retinoblastoma to release E2F and thus activate the transcription of other viral genes such as E2, E3 and E4 and the cellular genes that activate the cell cycle. E1B, for its part, binds to the p53 protein, to activate the cell cycle and prevent apoptosis of the infected cell. E2 encodes for virus replication proteins; E3 encodes proteins that inhibit the antiviral immune response; E4 encodes for proteins that transport viral RNA. Expression of the early genes leads to replication of the viral DNA, and once this is replicated, the major late promoter is activated, leading to expression of a messenger ribonucleic acid (RNA) transcript which, by cutting and differential splicing, generates all the RNAs that encode for the structural proteins that form the capsid.

There are two important aspects to be considered in relation to the design of oncolytic adenoviruses: selectivity and potency. To achieve selectivity towards the tumour cell, three strategies were used: elimination of viral functions necessary for replication in normal cells but dispensable in tumour cells; control of the viral genes that initiate replication by tumour-selective promoters; and modification of the viral capsid proteins involved in infection of the host cell. By means of these genetic modifications, a considerable level of selectivity has been achieved, with a tumour-cell replicative capacity some 10,000 times greater than the replicative capacity in the normal cell. In relation to oncolytic potency, various genetic modifications for increasing this have also been described. These modifications include: a) increased viral release, for example by deletion of E1B19K, overexpression of E3-11.6K (ADP), or relocation of the protein E3/19K in the plasma membrane; and b) the insertion of a therapeutic gene into the oncolytic adenovirus genome to generate an "armed oncolytic adenovirus". In this case, the therapeutic gene would have to mediate the death of the uninfected carcinogenic cells by activating a prodrug having a bystander effect (in other words, it kills the uninfected neighbouring cells), activating the immune system against the tumour, inducing apoptosis, inhibiting angiogenesis, or removing the extracellular matrix, among other things. In these cases, the form and time of therapeutic gene expression will be critical for the end result of the therapeutic approach.

However, the major obstacles encountered by adenoviral therapy in its clinical application are the immune response, whether pre-existing or generated by the first or subsequent injections of adenovirus, and the difficulty of adenoviruses being efficiently distributed throughout the tumour mass, i.e. not infecting solely the surface cells of the tumour but also reaching those located inside it. This latter difficulty has also been described for other anti-cancer drugs such as doxorubicin, Taxol [paclitaxel], vincristine and methotrexate and is thought to be related to the extracellular matrix of the tumour (involved in the resistance of tumour cells to chemotherapy drugs, BP Toole et al., "Hyaluronan: a constitutive regulator of chemoresistance and malignancy in cancer cells", Seminars in Cancer Biology 2008, vol. 18, pp. 244-50). Tumour cells and stromal cells in the tumour produce and assemble a matrix of collagens, proteoglycans and other molecules that hinder the transport of macromolecules within the tumour. Hyaluronic acid is one of the main components of said extracellular matrix involved in the resistance of tumour cells to therapeutic drugs. Hyaluronic acid is overexpressed in a wide variety of malignant tissues, and in many cases the levels of said acid are a prognostic factor for tumour progression. The interaction of hyaluronic acid with the receptors CD44 and RHAMM increases tumour survival and invasion. Moreover, hyaluronic acid can promote tumour metastases by inducing cell migration and adhesion, and protecting against the immune system.

On the other hand, the inhibition of interactions between hyaluronic acid and the tumour cells, reversing resistance to a large number of drugs, has been described. Various works have indicated that hyaluronidases, enzymes responsible for the degradation of hyaluronic acid, increase the activity of various types of chemotherapy in patients with melanoma, Kaposi's sarcoma, head and neck cancer and hepatic metastases from the colon. The mechanism of action of hyaluronidases is as yet unknown, but is generally attributed to a decrease in cell adhesion barriers, reduced interstitial pressure and improved penetration of the anticancer drug into the tumour, rather than its inhibitory effects on the signalling pathways related to cell survival.

Hyaluronidase enzymes are a family of enzymes responsible for degrading hyaluronic acid. In the human species six genes that encode for hyaluronidase enzymes, having different properties and locations, have been located to date. The isoforms Hyal1 and Hyal2 are found in most tissues, Hyal1 being the predominant form in human plasma. Hyal3 is located in the bone marrow and testicles, but its function is not well characterised. The hyaluronidase enzyme PH20 is highly expressed in the testicles and is involved in the process by which the oocyte is fertilised by the spermatozoon. The hyaluronidase enzyme PH20 is anchored to the plasma membrane and the inner acrosomal membrane of the spermatozoa and gives the spermatozoon the ability to penetrate the extracellular matrix of the cumulus cells (which are rich in hyaluronic acid) and reach the zona pellucida of the oocyte. During the acrosome reaction, some of the hyaluronidase enzymes anchored in the spermatozoon membrane are processed enzymatically to give rise to a soluble form of the protein, which is released from the acrosomal membrane. The membrane protein PH20 is the only enzyme in the mammalian hyaluronidase family with activity at neutral pH.

In view of the above, there is a need to have alternative therapies, more effective than the current ones, to treat retinoblastoma. In addition, said alternative treatments are particularly necessary when the tumour develops resistance to chemotherapy at the maximum permitted doses and/or the doses to be used show excessively high levels of toxicity, to avoid enucleating one or both eyes (for cases when the various alternative treatments with chemotherapy do not have positive effects). New therapeutic alternatives are also required to allow the risk of the patient developing metastases, secondary malignancies or trilateral retinoblastoma to be eliminated or reduced.

After extensive and exhaustive experiments, the inventors of the present invention have discovered, surprisingly, that it is possible to use genetically modified oncolytic adenoviruses to treat retinoblastoma and that said oncolytic adenoviruses allow the risk of metastases, secondary malignancies and/or trilateral retinoblastoma to be eliminated or reduced. The results obtained by the inventors of the present invention prove surprising for various reasons, including the fact that the aforementioned modified oncolytic adenovirus is not effective in infecting cell lines derived from some tumours yet is so for cell lines derived from retinoblastoma; and, furthermore, in in-vivo treatments, even though the adenovirus is injected into a theoretically semi-closed or isolated system (the vitreous cavity), the adenovirus manages to reduce the ability of the tumour cells to form metastases in comparison with the effect observed when using conventional chemotherapy. In other words, an effect is observed outside the eye which is totally unexpected and superior to that observed in conventional therapies of the prior art.

As used in the present document, "oncolytic adenovirus" and its plural refer to adenoviruses capable of replicating themselves or being replication-competent in the tumour cell. Said oncolytic adenoviruses are differentiated from non-replicating adenoviruses because the latter are unable to replicate themselves in the target cell.

According to a first aspect, the present invention discloses a composition comprising an oncolytic adenovirus for use for treating retinoblastoma, wherein the aforementioned oncolytic adenovirus comprises:
- a sequence encoding a hyaluronidase enzyme inserted into its genome; and
- replication machinery specific for tumour cells.

Said composition is injected by any route ensuring that the necessary amount of the adenovirus reaches the interior of the eyeball, preferably by intraocular injection, even more preferably intravitreal injection. Intraocular or intravitreal injections have the advantage that they are performed into an organ, the eye, which provides an immunoprivileged environment, facilitating a lower immune response, or no response, to the oncolytic adenovirus comprised in the composition. This situation makes it possible to ensure, to a large extent, that the action of the oncolytic adenovirus will remain confined to the eye, since if the adenovirus should ever leave said organ it could be neutralised by the immune system. Therefore, in a preferred embodiment, the aforementioned composition takes an appropriate form for intravitreal or intraocular administration or injection.

Another possible route of administration for the composition provided for in the present invention is intratumoral injection.

In a preferred embodiment, the treatment of retinoblastoma takes place in a mammal, preferably a human, more preferably a paediatric human patient.

In another preferred embodiment, the retinoblastoma treated is a retinoblastoma resistant to conventional chemotherapy and/or radiotherapy treatment; even more preferably said retinoblastoma is refractory or the result of a relapse and is resistant to conventional chemotherapy and/or radiotherapy treatment.

The oncolytic adenovirus used in the present invention is an oncolytic adenovirus with replication machinery and a capsid that allows infection and replication in human cancer cells. In a preferred embodiment, said oncolytic adenovirus is preferably generated from adenoviruses that infect humans, for example from an adenovirus of serotype 1 to 51, or combinations thereof (hybrid recombinant of two or more different serotypes of adenovirus) of human adenoviruses. In the most preferred embodiment, the oncolytic adenovirus used in the present invention is generated from a human adenovirus serotype 5.

It should be noted that the hyaluronidase enzyme contributes to ensuring greater or easier dispersion and penetration of the oncolytic adenovirus, thus ensuring that said adenovirus can reach and infect a larger number of retinoblastoma cells.

Furthermore, in one embodiment the hyaluronidase enzyme is a mammalian testicular hyaluronidase enzyme, more preferably a human one (GenBank Gene ID: 6677), also known as SPAM1 or spermatozoon adhesion molecule 1 or PH20. In one embodiment, the sequence of said enzyme has the sequence corresponding to the membrane-binding carboxy-terminal domain deleted from it, in order for the enzyme to be soluble. When this carboxy-terminal domain is deleted, the resulting enzyme is secreted into the extracellular environment.

In a preferred embodiment, the sequence encoding a hyaluronidase enzyme inserted into the oncolytic adenovirus genome is SEQ ID NO: 1, from which nucleotides 1471 to 1527, corresponding to the carboxy-terminal domain, have been deleted.

It is envisaged that the hyaluronidase enzyme sequence is inserted at any point in the oncolytic adenovirus genome as long as it allows said adenovirus to replicate selectively in tumour or cancer cells and as long as the hyaluronidase enzyme is expressed and produced effectively and functionally in the required quantities. Preferably, the hyaluronidase enzyme sequence is inserted into the genome of the oncolytic adenovirus after the nucleotide sequence of the adenovirus fibre.

Clearly, the expression of the hyaluronidase enzyme is controlled by a promoter operational in the retinoblastoma cells to be treated. Preferably, the expression of said enzyme is controlled by a promoter operational in animal cells. Preferably, the promoter is selected from the group consisting of the cytomegalovirus promoter, the adenovirus major late promoter, the SV40 promoter, the herpes simplex virus thymidine kinase promoter, the RSV promoter, the EF1-α promoter, the beta-actin promoter, the human IL-2 promoter, the human IL-4 promoter, the IFN promoter, the E2F promoter, the human GM-CSF promoter or combinations thereof. The promoter that regulates the expression of the enzyme can be present naturally in the adenovirus, as is the case with the adenovirus major late promoter. The promoter can also be inserted together with the sequence encoding the enzyme. In a preferred embodiment, the promoter is the adenovirus major late promoter and this is already located in the oncolytic adenovirus genome. In this last embodiment it is not necessary to introduce the promoter together with the hyaluronidase enzyme sequence, but rather the latter is introduced into the oncolytic adenovirus genome in such a way that it remains under the control of said promoter.

It is envisaged that the oncolytic adenovirus comprises additional sequences that allow promotion or optimisation of the protein translation of the sequence encoding the hyaluronidase enzyme. Said sequences can be inside or outside the hyaluronidase enzyme gene. For example, said additional sequences are selected from the group consisting of a sequence of cutting and splicing that allows RNA to be processed, IRES (internal ribosome entry site) sequences, the picornavirus sequence 2A or combinations thereof.

The tumour cell-specific replication machinery comprised by the oncolytic virus used in the present invention is machinery that, as explained above, causes the oncolytic adenovirus to replicate itself in a specific form in tumour cells and not in healthy or normal cells. Said machinery can take different forms if, as a result, it provides oncolytic adenoviruses with a replicative capacity solely in the retinoblastoma (tumour) cells - in other words, adenoviruses with selective replication in which their replication machinery requires the virus to enter the cancer or tumour cells in order for the virus to replicate itself. In this respect, the oncolytic adenovirus used can have modifications in its genome sequence that give it selective replication in tumour cells.

In one embodiment this is achieved with the incorporation of a specific tissue promoter or a specific tumour promoter, where said promoter controls the expression of one or more genes in the group E1a, E1b, E2 and E4. In particular, the promoter is selected from the group consisting of the E2F promoter, the telomerase hTERT promoter, the tyrosinase promoter, the prostate-specific antigen (PSA) promoter, the alpha-fetoprotein promoter, the COX-2 promoter, as well as artificial promoters formed by various transcription factor binding sites such as binding sites for hypoxia-inducible factor (HIF-1), the ETS transcription factor, the tumour cytotoxic factor (TCF), the E2F transcription factor or the Sp1 transcription factor. Preferably, the promoter controls the expression of E1a.

In a preferred embodiment, the specific replication machinery for tumour cells is defective replication machinery that can be complemented in tumour cells with both defective copies of the Rb1 gene. In other words, this is replication machinery that is mutated or modified such that when it enters healthy (non-tumour) cells it is not expressed or is expressed in such a way that it does not lead to viral replication. Instead, when the oncolytic adenovirus enters a tumour cell with the characteristics mentioned above, the replication machinery acquires sufficient complementation (the tumour cell performs the functions lacking in the virus) for the virus to be able to be replicated (complete its cycle, producing viruses and leading to lysis of the tumour cell). In this embodiment, one option for achieving said selective replication in tumour cells with both defective copies of the Rb1 gene is the elimination of early functions of E1A that block the retinoblastoma (RB) pathway. Other viral genes that interact directly with the retinoblastoma protein, such as E4 and E4orf6/7 respectively, are candidates for deletion or truncation in order to achieve selective replication in tumour cells with both defective copies of the Rb1 gene. In the most preferred embodiment, the oncolytic adenovirus used in the present invention is characterised by the deletion Δ24, which affects the interaction of E1a with the retinoblastoma protein, and the insertion of four sites binding to E2F-1 and one site binding to Sp1 into the endogenous E1a promoter to control the expression of E1a. Said DNA sequence corresponds to SEQ ID NO: 2 in the attached list of sequences.

Other modifications that comply with the above, i.e. allow specific replication of oncolytic adenoviruses in retinoblastoma tumour cells, are also provided for and included in the present invention.

The two strategies explained for achieving selective replication in retinoblastoma are not mutually exclusive.

Furthermore, it is envisaged that the oncolytic adenovirus used in the present invention comprises one or more modifications in the capsid allowing increased affinity of said oncolytic adenovirus for the cancer or tumour cells (modifications in its capsid to increase its infectivity with regard to the cancer or tumour cells or to direct it to a receptor present in said cancer or tumour cells). In this way, the proportion of oncolytic adenoviruses infecting cancer or tumour cells is increased compared with those infecting healthy cells, and the latter adenoviruses can even reach a negligible amount compared with the former.

In one embodiment, the oncolytic adenovirus used in the present invention has been modified genetically so as to include, in the adenovirus capsid proteins, ligands that increase infectivity or direct the virus to a receptor in the cancer or tumour cell. Directing the adenovirus to the tumour can also be achieved with bifunctional ligands that bind to the virus on one side and to the tumour receptor on the other. In a preferred embodiment, the oncolytic adenovirus used in the present invention has the capsid modified to increase its infectivity or to direct it better to the target tumour cell, such that the binding domain KKTK of the heparan sulfates present in the adenovirus fibre has been replaced by the domain RGDK. Said modification relates to positions 91 to 94 of the adenovirus fibre, taking the standard sequence of the adenovirus serotype 5 fibre as the reference. The sequence SEQ ID NO: 9 shows the complete sequence of the adenovirus type 5 fibre protein with the modified version in its heparan sulfate binding domain (modification RGDK).

Furthermore, it is also possible to modify the capsid of the viruses for other purposes, for example to increase the persistence of the oncolytic adenovirus in blood and thus increase the possibilities of said adenovirus reaching tumour nodules (particularly if said nodules or tumour foci are disseminated). For example, the capsid can be covered with polymers such as polyethylene glycol.

Therefore, in the most preferred embodiment, the oncolytic adenovirus used in the present invention is generated from a human adenovirus serotype 5 and comprises:
- a sequence encoding a hyaluronidase enzyme inserted into its genome, more preferably the human testicular hyaluronidase enzyme sequence (PH20, SEQ ID NO: 1) from which the sequence corresponding to the membrane-binding carboxy-terminal domain has been deleted in order for the enzyme to be soluble.
- deficient replication machinery that can be complemented in tumour cells by both defective copies of the Rb1 gene; more preferably said machinery comprises the deletion Δ24, which affects the interaction of E1a with the Rb protein, and the insertion of four binding sites to E2F-1 and one binding site to Sp1 into the endogenous E1a promoter to control the expression of E1a;
- modification of the adenovirus capsid to increase the infectivity of said virus or to direct said adenovirus better to the target tumour cell; more preferably the binding domain KKTK of the heparan sulfates present in the adenovirus fibre has been replaced by the domain RGDK.

Preferably, the oncolytic virus sequence of the present invention corresponds to SEQ ID NO: 3.

The oncolytic adenovirus used in the present invention can have inserted into its genome other genes in common use in the field of gene therapy for cancer to increase the cytotoxicity of oncolytic adenoviruses on tumour cells, for example the thymidine kinase gene, the cytosine deaminase gene, proapoptotic genes, immunostimulants, tumour suppressors or prodrug activators.

To construct the oncolytic adenovirus to be used in the present invention (i.e. as explained above), use is made of any of the methods of construction of genetically modified adenovirus known in the field of gene therapy and virotherapy using adenoviruses. The most commonly used method is based on first constructing the desired genetic modification in a plasmid containing the adenoviral region to be modified, and then carrying out homologous recombination in bacteria with a plasmid containing the rest of the viral genome.

The oncolytic adenovirus used in the present invention is propagated and amplified in cell lines normally used in the field of gene therapy and virotherapy such as the lines HEK-293 (Reference number: ATCC CRL-1573) and A549 (Reference number: ATCC CCL185). A preferred method of propagation of said adenovirus is by infection of a cell line permitting the replication of the adenovirus. The lung adenocarcinoma line A549 is an example of a line with such characteristics. Propagation takes place, for example, as follows: the A549 cells are grown on plastic cell culture plates and are infected using 100 virus particles per cell. Two days later, the cytopathic effect reflecting virus production can be observed as a clustering of the cells. The cells are collected and stored in tubes. After centrifuging at 1000 g for 5 minutes, the cell pellet is frozen and thawed three times, to lyse the cells. The resulting cell extract is centrifuged at 1000 g for 5 minutes and the supernatant, in which the viruses are located, is loaded over a caesium chloride gradient and centrifuged for 1 hour at 35,000 g. The virus band obtained from the gradient is loaded again over another caesium chloride gradient and centrifuged for 16 hours at 35,000 g. The virus band is collected and dialysed opposite PBS-10% glycerol. The virus dialysate is aliquoted and stored at -80 ºC. The number of plaque-forming units and particles is quantified following standard protocols known in the prior art. Phosphate-buffered saline (PBS) with 5% glycerol is a standard formulation for storing adenoviruses. However, new formulations improving virus stability have been described. The methods for purifying the adenovirus containing the hyaluronidase gene for use in cancer treatment are the same as those described for other adenoviruses and adenoviral vectors used in the prior art in virotherapy and gene therapy for cancer.

It will be understood that the composition used in the present invention is used in a pharmaceutically acceptable presentation. This means that said composition may additionally comprise pharmaceutically acceptable excipients.

The dose of oncolytic adenovirus required can be determined by the person skilled in the art, taking various parameters into account, for example the volume of the vitreous cavity, the volume of the retinoblastoma to be treated or the age and weight of the patient to be treated. It will be understood that the dose must be a therapeutically effective quantity of oncolytic adenovirus in order to produce a positive therapeutic effect on the retinoblastoma, "positive therapeutic effect" meaning the maintenance or reduction of retinoblastoma volume.

Obviously, the person skilled in the art will adapt the composition depending on the particular method of administration, taking particular account of the route of administration to be used.

The composition can also be administered in a therapeutic regimen that includes the administration of one or more other anti-tumour agents (such as one or more chemotherapy drugs) and/or one or more of the other aforementioned therapies conventionally used for treating retinoblastoma. The composition used in the present invention in relation to these other therapeutic agents and or therapies can be administered prior to, at the same time as or after the same. When the composition used in the present invention is administered at the same time as one or more other anti-tumour agents, said one or more other anti-tumour agents can be included in the composition used in the present invention or can be administered as separate compositions.

Alternatively, the composition used in the present invention, comprising the oncolytic adenovirus as explained above, can be used alone in a therapeutic regimen for treating retinoblastoma, i.e. without using other anti-tumour agents and/or other conventional therapies.

According to a second aspect, the present invention discloses a composition comprising an oncolytic adenovirus for use for preventing, removing or reducing metastases, secondary malignancies and or trilateral retinoblastoma associated with retinoblastoma, wherein the aforementioned oncolytic adenovirus comprises:
- a sequence encoding a hyaluronidase enzyme inserted into its genome; and
- replication machinery specific for tumour cells.

Said composition is injected by any route ensuring that the necessary amount of the adenovirus reaches the interior of the eyeball, preferably by intraocular injection, even more preferably intravitreal injection. Therefore, in a preferred embodiment, the aforementioned composition takes an appropriate form for intravitreal or intraocular administration or injection.

Another possible route of administration for the composition provided for in the present invention is intratumoral injection.

In a preferred embodiment, the prevention, removal or reduction of metastases, secondary malignancies and/or trilateral retinoblastoma associated with retinoblastoma takes place in a mammal, preferably a human, more preferably a paediatric human patient.

In another preferred embodiment, the retinoblastoma treated is a retinoblastoma resistant to conventional chemotherapy and/or radiotherapy treatment; even more preferably said retinoblastoma is refractory or the result of a relapse and is resistant to conventional chemotherapy and/or radiotherapy treatment.

The oncolytic adenovirus used is the same as has been explained previously.

The composition and the form in which said composition is used or can be administered is also the same as has been explained previously.

According to a third aspect, the present invention relates to a composition comprising an oncolytic adenovirus for use thereof in the treatment of retinoblastoma, wherein the aforementioned oncolytic adenovirus comprises:
- a sequence encoding a hyaluronidase enzyme inserted into its genome; and
- replication machinery specific for tumour cells.

Said composition is injected by any route ensuring that the necessary amount of the adenovirus reaches the interior of the eyeball, preferably by intraocular injection, even more preferably intravitreal injection. Therefore, in a preferred embodiment, the aforementioned composition takes an appropriate form for intravitreal or intraocular administration or injection.

Another possible route of administration for the composition provided for in the present invention is intratumoral injection.

In a preferred embodiment, the treatment of retinoblastoma takes place in a mammal, preferably a human, more preferably a paediatric human patient.

In another preferred embodiment, the retinoblastoma treated is a retinoblastoma resistant to conventional chemotherapy and/or radiotherapy treatment; even more preferably said retinoblastoma is refractory or the result of a relapse and is resistant to conventional chemotherapy and/or radiotherapy treatment.

The oncolytic adenovirus used is the same as has been explained previously.

The composition and the form in which said composition is used or can be administered is also the same as has been explained previously.

According to a fourth aspect, the present invention relates to a composition comprising an oncolytic adenovirus for use thereof in the prevention, removal or reduction of metastases, secondary malignancies and/or trilateral retinoblastoma associated with retinoblastoma, wherein the aforementioned oncolytic adenovirus comprises:
- a sequence encoding a hyaluronidase enzyme inserted into its genome; and
- replication machinery specific for tumour cells.

Said composition is injected by any route ensuring that the necessary amount of the adenovirus reaches the interior of the eyeball, preferably by intraocular injection, even more preferably intravitreal injection. Therefore, in a preferred embodiment, the aforementioned composition takes an appropriate form for intravitreal or intraocular administration or injection.

Another possible route of administration for the composition provided for in the present invention is intratumoral injection.

In a preferred embodiment, the prevention, removal or reduction of metastases, secondary malignancies and/or trilateral retinoblastoma associated with retinoblastoma takes place in a mammal, preferably a human, more preferably a paediatric human patient.

In another preferred embodiment, the retinoblastoma treated is a retinoblastoma resistant to conventional chemotherapy and/or radiotherapy treatment; even more preferably said retinoblastoma is refractory or the result of a relapse and is resistant to conventional chemotherapy and/or radiotherapy treatment.

The oncolytic adenovirus used is the same as has been explained previously.

The composition and the form in which said composition is used or can be administered is also the same as has been explained previously.

According to a fifth aspect, the present disclosure relates to a method for treating retinoblastoma in a patient requiring the same, comprising the administration of a therapeutically effective amount of a composition comprising an oncolytic adenovirus, wherein the aforementioned oncolytic adenovirus comprises:
- a sequence encoding a hyaluronidase enzyme inserted into its genome; and
- replication machinery specific for tumour cells.

Said composition is injected by any route ensuring that the necessary amount of the adenovirus reaches the interior of the eyeball, preferably by intraocular injection, even more preferably intravitreal injection. Therefore, in a preferred embodiment, the aforementioned composition takes an appropriate form for intravitreal or intraocular administration or injection.

Another possible route of administration for the composition provided for in the present invention is intratumoral injection.

In a preferred embodiment, the patient requiring treatment of retinoblastoma is a mammal, preferably a human, more preferably a paediatric human patient.

In another preferred embodiment, the retinoblastoma treated is a retinoblastoma resistant to conventional chemotherapy and/or radiotherapy treatment; even more preferably said retinoblastoma is refractory or the result of a relapse and is resistant to conventional chemotherapy and/or radiotherapy treatment.

The oncolytic adenovirus used is the same as has been explained previously.

The composition and the form in which said composition is used or can be administered is also the same as has been explained previously.

According to a final aspect, the present disclosure relates to a method for preventing, removing or reducing metastases, secondary malignancies and/or trilateral retinoblastoma associated with retinoblastoma in a patient requiring the same, comprising the administration of a therapeutically effective amount of a composition comprising an oncolytic adenovirus, wherein the aforementioned oncolytic adenovirus comprises:
- a sequence encoding a hyaluronidase enzyme inserted into its genome; and
- replication machinery specific for tumour cells.

Said composition is injected by any route ensuring that the necessary amount of the adenovirus reaches the interior of the eyeball, preferably by intraocular injection, even more preferably intravitreal injection. Therefore, in a preferred embodiment, the aforementioned composition takes an appropriate form for intravitreal or intraocular administration or injection.

Another possible route of administration for the composition provided for in the present invention is intratumoral injection.

In a preferred embodiment, the patient requiring prevention, removal or reduction of metastases, secondary malignancies and/or trilateral retinoblastoma associated with retinoblastoma is a mammal, preferably a human, more preferably a paediatric human patient.

In another preferred embodiment, the retinoblastoma treated is a retinoblastoma resistant to conventional chemotherapy and/or radiotherapy treatment; even more preferably said retinoblastoma is refractory or the result of a relapse and is resistant to conventional chemotherapy and/or radiotherapy treatment.

The oncolytic adenovirus used is the same as has been explained previously.

The composition and the form in which said composition is used or can be administered is also the same as has been explained previously.

The inventors of the present invention have therefore managed to resolve the problems present in the prior art, since they have been able to ascertain that the use of oncolytic adenoviruses having the characteristics mentioned above makes it possible to provide an alternative therapeutic method to those currently available that allows enucleation of the eye to be avoided, and to provide a method that prevents, reduces or avoids the development of metastases, secondary malignancies or trilateral retinoblastoma associated with retinoblastoma.

For better understanding, the present invention is described in greater detail below with reference to the attached figures, which are presented by way of example, and with reference to illustrative but nonlimiting examples. Said examples are carried out in cell cultures (in vitro) and in animal models (mouse and rabbit), both recognised and accepted as reliable models representative of retinoblastoma in the scientific world, on the basis of which a person skilled in the art will recognise the direct application of the methods and treatments of the present invention in humans.

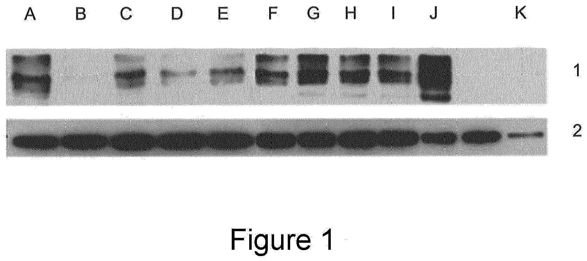

Fig. 1 shows the results of the Western blot mentioned in example 2. In said Western blot the expression of the protein E1A is analysed in the various cell lines analysed. Retinal explants were used as a general control and, as a control of expression, the presence of tubulin was measured. In this figure, row 1 shows the expression of E1A and row 2 the expression of tubulin. With regard to the various columns, column A relates to the primary cell line HSJD-RBT1; column B relates to the primary cell line HSJD-RBVS1; column C relates to the primary cell line HSJD-RBT2; column D relates to the primary cell line HSJD-RBVS2, column E relates to the primary cell line HSJD-RBVS3; column F relates to the primary cell line HSJD-RBT5; column G relates to the primary cell line HSJD-RBT7; column H relates to the primary cell line HSJD-RBT8; column I relates to the primary cell line HSJD-RBVS8; column J relates to the cell line Y79; and column K relates to retinal explants.

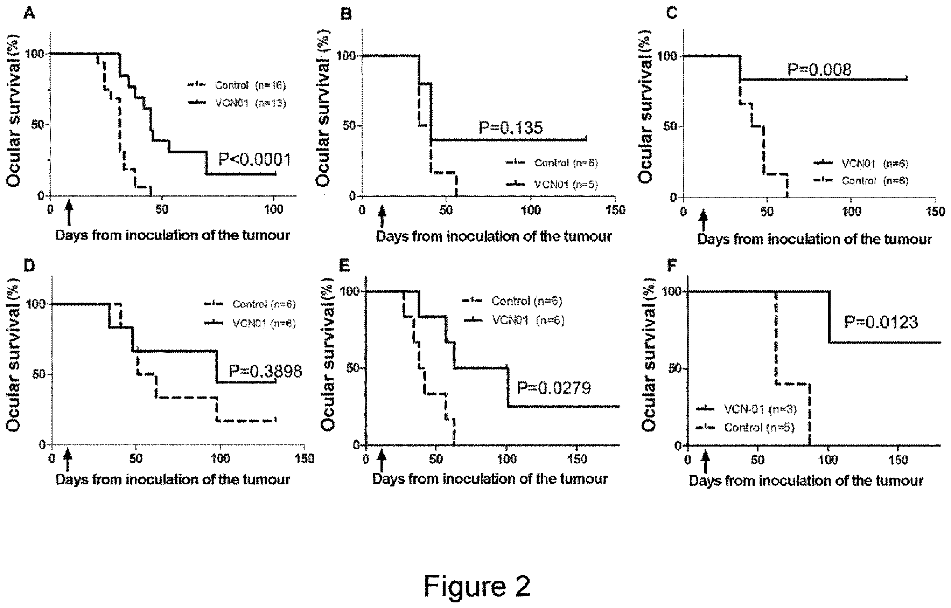

Fig. 2 shows in graph form the survival of treated eyes and control eyes according to the Kaplan-Meier method for the study described in example 3. Fig. 2A relates to the results obtained by generating orthotopic tumours in mice using the cell line Y79; Fig. 2B relates to the results obtained by generating orthotopic tumours in mice using the primary cell line HSJD-RBT2; Fig. 2C relates to the results obtained by generating orthotopic tumours in mice using the primary cell line HSJD-RBT5; Fig. 2D relates to the results obtained by generating orthotopic tumours in mice using the primary cell line HSJD-RBVS1; Fig. 2E relates to the results obtained by generating orthotopic tumours in mice using the primary cell line HSJD-RBT7; and Fig. 2F relates to the results obtained by generating orthotopic tumours in mice using the primary cell line HSJD-RBVS8.

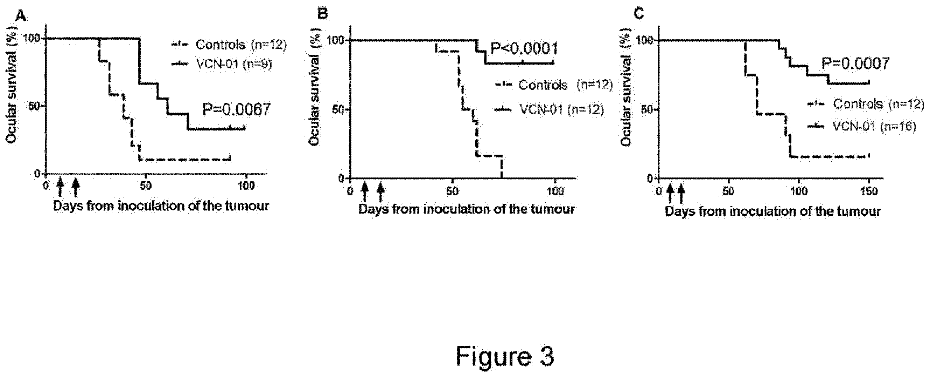

Fig. 3 shows in graph form the survival of treated eyes and control eyes according to the Kaplan-Meier method for the study described in example 4. Fig. 3A relates to the results obtained by generating orthotopic tumours in mice using the cell line Y79; Fig. 3B relates to the results obtained by generating orthotopic tumours in mice using the primary cell line HSJD-RBT-002; and Fig. 3C relates to the results obtained by generating orthotopic tumours in mice using the primary cell line HSJD-RBT-005.

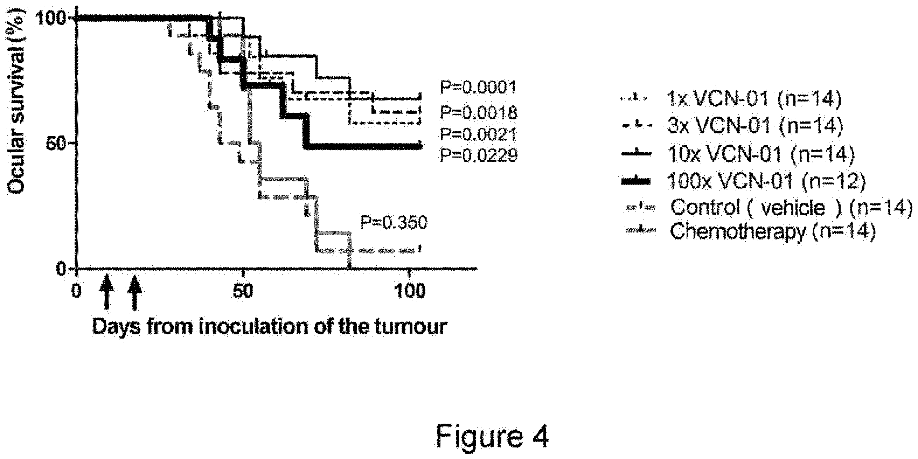

Fig. 4 shows in graph form the survival of treated eyes and control eyes according to the Kaplan-Meier method for the study described in example 5.

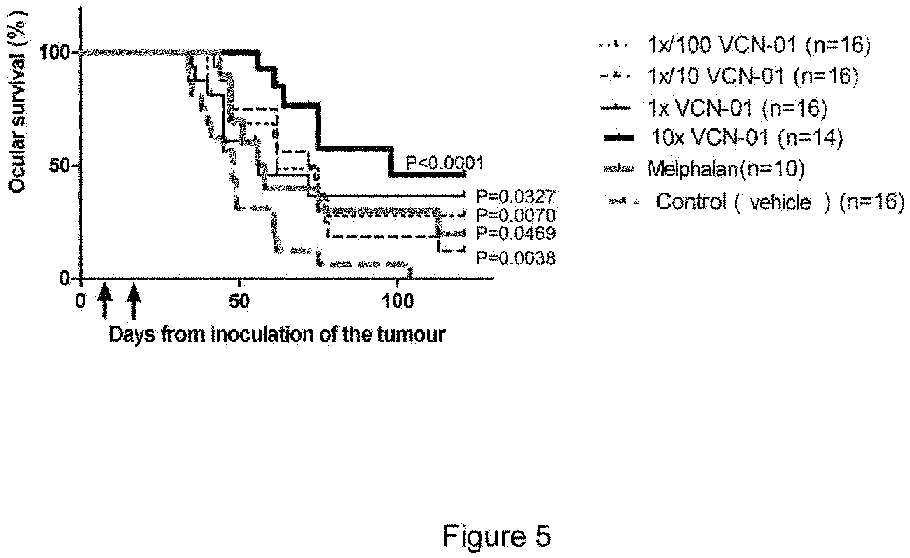

Fig. 5 shows in graph form the survival of treated eyes and control eyes according to the Kaplan-Meier method for the study described in example 6.

### Example 1. Generation of the oncolytic adenovirus VCN-01

The cDNA of hyaluronidase PH20 was obtained by PCR amplification (polymerase chain reaction amplification technique) of the various exons of the protein from the genome of the cell line A549 and subsequent binding of these exons with specific oligonucleotides that contain the target of the restriction enzyme Mfel. The resulting fragment was digested with Mfel and cloned by ligation in the shuttle plasmid, pNKFiberRGD (containing the sequence of the adenovirus fibre modified with RGD), to generate the plasmid pNKFiberPH20. The cDNA corresponding to the PH20 protein cloned in the plasmid pNKFiberPH20 is shown in SEQ ID NO: 1. SEQ ID NO: 1 shows the nucleotides coding for the PH20 protein from the start of its transcription (ATG) to position 1470. The nucleotide sequence of region 1471 to 1527 encodes for the hydrophobic tail of the protein, which is responsible for anchoring it to the membrane. Said sequence was deleted. After the position 1470 the translation stop codon TAA was added.

To generate the plasmid pAdwtRGD-PH20, the adenovirus fibre gene of the plasmid pVK50cau (which contains the complete Ad5 sequence with a Swal target in the fibre) was replaced by homologous recombination in yeast, according to procedures known in the prior art, by the fibre gene followed by the hyaluronidase PH20 gene of the plasmid pNKFiberPH20 digested with Notl/Kpnl.

The adenoviral plasmid pICOVIR17 was used to generate the adenovirus ICOVIR17 (SEQ ID NO: 17). To generate this plasmid, the adenovirus fibre gene of the plasmid pICOVIR15 was replaced by homologous recombination in yeast by the fibre gene followed by the hyaluronidase PH20 gene of the plasmid pAdwtRGD-PH20 digested with Spel/Pacl.

The adenovirus ICOVIR15 (SEQ ID NO: 4) comes from the adenovirus AdA24RGD, characterised by containing the deletion Δ24 in the sequence coding for the protein E1a. This deletion affects the interaction of E1a with the retinoblastoma protein. AdΔ24RGD also has the insertion of the RGD peptide in the adenovirus fibre, to increase the infectivity of the virus. These two modifications were described in K. Suzuki et al., "A conditionally replicative adenovirus with enhanced infectivity shows improved oncolytic potency", Clin Cancer Res 2001, vol. 7, pp. 120-6. Starting from AdA24RGD, four binding sites to E2F-1 and one binding site to Sp1 were inserted into the endogenous E1a promoter to control the expression of E1a. In this way ICOVIR15 was obtained. This insertion was carried out by replacing the sequence 419-422 of the genome by the sequence with the four binding sites to E2F-1 and one binding site to Sp1, such that the final sequence is that shown in SEQ ID NO: 4. To do this, a single cleavage site BsiWI was created by mutagenesis directed to the E1A promoter of pEndK/Spe (J. E. Carette et al., "Conditionally replicating adenoviruses expressing short hairpin RNAs silence the expression of a target gene in cancer cells", Cancer Res 2004, vol. 64, pp. 2663-7). The binding site to Sp1 was introduced into the pEndK/Spe plasmid with the site BsiWI by ligation of said plasmid digested with BsiWI with the oligonucleotides Sp1F (SEQ ID NO: 5 - 5'-GTACGTCGACCACAAACCCCGCCCAGCGTCTTGTCATTGGCGTCGACGCT-3') and Sp1R (SEQ ID NO: 6 - 5'-GTACAGCGTCGACGCCAATGACAAGACGCTGGGCGGGGTTTGTGG TCGAC-3') paired together. The binding sites to E2F were introduced by means of binding the oligonucleotides E2FF2 (SEQ ID NO: 7 - 5'-GTACGTCGGCGGCTCGTGGC TCTTTCGCGGCAAAAAGGATTTGGCGCGTAAAAGTGGTTCGAA-3') and E2FR2 (SEQ ID NO: 8 - 5'-GTACTTCGAACCACTTTTACGCGCCAAATCCTTTTTGCCGCGAAAGAGCCAC GAGCCGCCGAC-3') paired together, to create the plasmid pEndK415Sp1 E2F2. Next, the CAU sequence, which contains the elements required for plasmid replication in yeast - a centromere, the autonomous recombination sequence ARS and the selection marker URS3 - was introduced by homologous recombination in yeast, to create the plasmid pEndK415Sp1 E2F2CAU. Finally, homologous recombination in yeast of the plasmid pEndK415Sp1 E2F2CAU, digested with Kpnl, with the adenoviral genome of the adenovirus AdA24RGD, was carried out to construct pICOVIR15cau. ICOVIR15 was obtained by transfection of Pacl digestion of pICOVIR15cau to HEK293 cells (ATCC reference number CRL-1573).

The adenovirus ICOVIR17, which contains the same modifications as ICOVIR15, plus the insertion of the hyaluronidase gene after the adenovirus fibre gene, was generated by digestion with Pacl of the pICOVIR17 plasmid and transfection into HEK293 cells.

The adenovirus VCN-01 was generated using the adenoviral plasmid pICOVIR17RGDK. In this plasmid the native adenovirus serotype 5 fibre gene has been replaced by a modified version in its heparan sulfate binding domain (amino acids ⁹¹KKTK⁹⁴ of the polypeptide sequence replaced by ⁹¹RGDK⁹⁴). The plasmid pICOVIR17RGDK was constructed by homologous recombination in yeast between the partial digestion product of pICOVIR17 with Ndel and digestion with EcoRI of the plasmid pBSattKKT (containing the modified version of the adenovirus fibre described in N. Bayó-Puxan et al. "Replacement of adenovirus type 5 fiber shaft heparan sulfate proteoglycan-binding domain with RGD for improved tumor infectivity and targeting". Human Gene Therapy 2009, vol. 20, pp 1214-21).

The sequence SEQ ID NO: 9 shows the complete sequence of the adenovirus serotype 5 fibre protein with the modified version in its heparan sulfate binding domain (modification RGDK). The adenovirus ICOVIR17 contains a version of the adenovirus fibre gene into which the peptide RGD-4C has been inserted (Cys-Asp-Cys-Arg-Gly-Asp-Cys-Phe-Cys; CDCRGDCFC; SEQ ID NO: 10) into the HI region of the knob domain of the protein (hypervariable loop of the adenovirus capsid, evolutionarily non-conserved and highly exposed). VCN-01 is completely analogous to ICOVIR17 apart from the fibre gene, since in this case the VCN-01 fibre differs only from the native form of human adenovirus serotype 5 in that the amino acids ⁹¹KKTK⁹⁴ have been replaced by the peptide with a high affinity for integrins ⁹¹RGDK⁹⁴, in the shaft domain of the protein.

### Example 2. Cytotoxicity in primary cultures of retinoblastoma refractory to conventional treatment with chemotherapy and/or radiotherapy.

First, IC50 was studied in various primary cultures obtained from retinoblastomas showing resistance to conventional chemotherapy and/or radiotherapy treatment. The cell line Y79 (ATCC reference number HTB-18) was also analysed.

To obtain the various primary cultures, tumour samples were first taken from enucleated eyes of patients with retinoblastoma at the Sant Joan de Deu Hospital (HSJD, Barcelona), under a protocol approved by the corresponding Ethics Committee and with an Informed Consent. The specimens were collected from two possible sources: solid tumour tissue in the retina (retinoblastoma tumour; RBT) or from the tumour seeds in the vitreous body (retinoblastoma vitreous seedings; RBVS). To obtain cell suspensions of the RBT specimens, these were disaggregated using a mixture of collagenase (5 mg/mL) and DNAse (40 Kunitz units/mL) (Sigma, St Louis, MO) at 37°C for 5 min; cells from RBVS samples were collected by centrifugation. After two washes with PBS, the tumour cells were cultured as tumour-spheres floating in a serum-free neural stem cell medium.

To determine the IC₅₀ values of VCN-01, tests were carried out with each line on 96-well plates seeded with 5000 cells per well. After being cultured for 24 hours, the cells were exposed to increasing doses of VCN-01 for 11-14 days in triplicate. At this point the metabolic activity of each well was determined using the reagent MTS (Promega, Fitchburg, WI, USA) and according to procedures known in the prior art. Based on these values and using Graphpad Prism 5 software (La Jolla, CA, USA), the concentration of VCN-01 that was causing a 50% decrease in cell proliferation (IC₅₀ value) was calculated.

**Table 1. IC₅₀ results obtained in primary cultures of retinoblastoma refractory to conventional chemotherapy and/or radiotherapy treatment; and in the cell line Y79.**

| **Cell model** | **IC₅₀ (TU/cell) (95% confidence interval)** |
|---|---|
| HSJD-RBT-1 | 5.47 (4.81-6.21) |
| HSJD-RBVS-1 | >100 (not sensitive) |
| HSJD-RBT-2 | 8.01 (5.69-11.29) |
| HSJD-RBVS-3 | 2.97 (1.60-5.50) |
| HSJD-RBT-5 | 0.0825 (0.0489-0.139) |
| HSJD-RBT-7 | 8.82 (4.29-18.14) |
| HSJD-RBT-8 | 27.29 (21.73-34.03) |
| Y79 | 0.0809 (0.0662-0.0988) |

As can be seen in Table 1, VCN-01 demonstrated great cytotoxicity in all the cell lines tested except for one (HSJD-RBVS1), from which it can be deduced that not only can said oncolytic adenovirus correctly infect a large number of primary cell lines derived from retinoblastoma but that it also has high cytotoxic activity in them.

Furthermore, expression of the adenoviral protein E1A was analysed 24 hours from infection of the cell lines by Western blotting (said expression indicated that the defective replication machinery of VCN-01 was correctly complemented by the machinery of the corresponding tumour cell). As can be seen in Fig. 1, expression of the protein E1A was observed in all the cell lines mentioned above apart from the cell line HSJD-RBVS1, which agrees or correlates with the IC₅₀ results obtained and shown in Table 1.

### Example 3. Anti-tumour activity in various orthotopic models of human retinoblastoma in mice treated with an injection of VCN-01 or vehicle.

Female athymic mice (Hsd:Athymic Nude-Foxn1nu) aged 6 weeks were injected with 200,000 cells of the corresponding primary cell line or the cell line Y79 in 2 µL Matrigel, into the vitreous cavity of both eyes. On the eighth day, an intravitreal injection of the 100x dose of VCN-01 (2 µL with a total content of 3x10⁹ virus particles per eye) was applied to the right eyes of each animal. The contralateral (left) eyes were injected by the intravitreal route with the vehicle in which VCN-01 (20 mM Tris pH 8.0 25 mM NaCl, 2.5% glycerol) had been diluted.

The distribution of mice in each of the experimental groups was as indicated in Table 2.

**Table 2. Experimental groups and number of mice per study group in example 3.**

| **Group (cell line with which the orthotopic tumour is generated in the eyes of the mice)** | **Number of mice in the treated group** |
|---|---|
| Cell line Y79 | 17 |
| HSJD-RBT2 | 6 |
| HSJD-RBT5 | 6 |
| HSJD-RBVS1 | 6 |
| HSJD-RBT7 | 6 |
| HSJD-RBVS8 | 5 |

In this case, what was studied was the survival of the treated and control eyes, i.e. the number of days until the size of the retinoblastoma required enucleation of said eye.

As can be seen in Fig. 2, eyes treated with the adenovirus VCN-01 survive in greater numbers and for a longer time in all the groups analysed (that is to say, irrespective of the cell line used for implanting the orthotopic tumour in the mouse eye, a significant improvement was observed when applying treatment with the adenovirus VCN-01).

### Example 4. Anti-tumour activity in various orthotopic models of human retinoblastoma in mice treated with two injections of VCN-01 or vehicle.

Female athymic mice (Hsd:Athymic Nude-Foxn1nu) aged 6 weeks were injected with 200,000 cells of the corresponding primary cell line or the cell line Y79 in 2 µL Matrigel, into the vitreous cavity of each eye. On the eighth day, an intravitreal injection of the 100x dose of VCN-01 (2 µL with a total content of 3x10⁹ virus particles) was applied to both eyes of the mice in the treated group, and the mice of the other group (control group) were given an intravitreal injection of vehicle (20 mM Tris pH 8.0, 25 mM NaCl, 2.5% glycerol) into each eye. On the twenty-second day, the therapeutic procedure was repeated, i.e. an intravitreal injection of the 100x dose of VCN-01 (2 µL with a total content of 3x10⁹ virus particles) was applied to both eyes of the mice in the treated group, and the mice of the other group (control group) were given an intravitreal injection of the same vehicle also into both eyes.

The distribution of mice in each of the experimental groups was as indicated in Table 3.

**Table 3. Experimental groups and number of mice per study group in example 4.**

| **Group (cell line with which the orthotopic tumour is generated in the eyes of the mice)** | **Number of mice in the control group** | **Number of mice in the treated group** |
|---|---|---|
| Cell line Y79 | 6 | 6 |
| HSJD-RBT2 | 6 | 6 |
| HSJD-RBT5 | 6 | 6 |

In this case too, the survival of the treated and control eyes was studied, i.e. the number of days until the size of the retinoblastoma required enucleation of said eye.

As can be seen in Fig. 3, eyes treated with the adenovirus VCN-01 survive in greater numbers and for a longer time in all the groups analysed (that is to say, irrespective of the cell line used for implanting the orthotopic tumour in the mouse eye, a significant improvement was observed when applying treatment with the adenovirus VCN-01).

### Example 5. Dose escalation study of VCN-01 in mice.

Female athymic mice (Hsd:Athymic Nude-Foxn1nu) aged 6 weeks were injected with 200,000 cells of the primary cell line HSJD-RBT2 (day 0) in 2 µL Matrigel. Next, said mice were treated according to the following groups and the following regimen:

**Table 4. Groups and treatment days in the study in example 5. The days are indicated by taking day 0 to be the day when the cells of the primary line of retinoblastoma are injected or implanted to generate the orthotopic tumours.**

| **Group (treatment applied)** | **Number of mice in the group** | **Injection days (for VCN-01 and vehicle) / Chemotherapy days** |
|---|---|---|
| Vehicle (control group; 20 mM Tris pH 8.0 25 mM NaCl, 2.5% glycerol) | 7 | 8 and 22 |
| Systemic chemotherapy (Carboplatin/Etoposide) (6 mg/kg etoposide and 34 mg/kg carboplatin) | 7 | 8, 29 and 50 |
| VCN-01 dose 100x (3x10⁹ virus particles per eye) | 6 | 8 and 22 |
| VCN-01 dose 10x (3x10° virus particles per eye) | 7 | 8 and 22 |
| VCN-01 dose 3x (1x10⁸ virus particles per eye) | 7 | 8 and 22 |
| VCN-01 dose 1x (3x10⁷ virus particles per eye) | 7 | 8 and 22 |

The parameter analysed was the survival of the treated and control eyes, i.e. the number of days until the size of the retinoblastoma required enucleation of said eye.

The results obtained are summarised in Fig. 4. As can be seen in said figure, all the tested doses of VCN-01 gave significantly better results for eye survival than the control or chemotherapy treatment groups. In addition, it was possible to ascertain, surprisingly, that the dose that offered greatest survival of the eye for a longer time was 10x.

### Example 6. Dose escalation study of VCN-01 in mice.

Female athymic mice (Hsd:Athymic Nude-Foxn1nu) aged 6 weeks were injected with 200,000 cells of the primary cell line HSJD-RBT2 (day 0) in 2 µL Matrigel. Next, said mice were treated according to the following groups and the following regimen:

**Table 5. Groups and treatment days in the study in example 5. The days are indicated by taking day 0 to be the day when the cells of the primary line of retinoblastoma are injected or implanted to generate the orthotopic tumours.**

| **Group (treatment applied)** | **Number of mice in the group** | **Injection days (for VCN-01 and vehicle) / Chemotherapy days** |
|---|---|---|
| Vehicle (control group; 20 mM Tris pH 8.0 25 mM NaCl, 2.5% glycerol) | 8 | 8 and 22 |
| Intravitreal chemotherapy (Melphalan; 0.033 µg in 2 µL per eye) | 5 | 8 and 22 |
| VCN-01 HIGH dose (3x10° virus particles per eye) (10x VCN-01) | 7 | 8 and 22 |
| VCN-01 LOW dose (3x10⁷ virus particles per eye) (1x VCN-01) | 8 | 8 and 22 |
| VCN-01 VERY LOW dose (3x10° virus particles per eye) (1x/10 VCN-01) | 8 | 8 and 22 |
| VCN-01 ULTRA LOW dose (3x10⁵ virus particles per eye) (1x/100 VCN-01) | 8 | 8 and 22 |

The parameter analysed was the survival of the treated and control eyes, i.e. the number of days until the size of the retinoblastoma required enucleation of said eye.

The results obtained are summarised in Fig. 5. As can be seen in said figure, all the doses revealed a superior result compared with the control group. Furthermore, the HIGH and LOW doses also demonstrated a superior effect at the end of treatment than the group treated with intravitreal chemotherapy.

Although the invention has been described in relation to preferred embodiments, these should not be considered to limit the invention, which is to be defined by the broadest interpretation of the following claims.

### Example 7. Analysis of the influence of VCN-01 on brain metastases in retinoblastoma in mice.

Extraocular extension of retinoblastoma to the central nervous system (CNS) is fatal for patients.

To carry out this study an orthotopic model of retinoblastoma was used. Use was made of brains from the mice in Examples 5 and 6, female athymic nude mice (Hsd:Athymic Nude-Foxn1^{nu} strain - Harlan Laboratories, Gannat, France), aged 6 weeks, which received an injection into the posterior segment of each eye, of 2x10⁵ cells HSJD-RBT2 (primary culture of cells mentioned above and obtained from the eye of a retinoblastoma patient resistant to chemotherapy).

**Table 6. Brains analysed in the study in Example 7, with details of the treatment applied to each of the mice from which the brains were extracted.**

| Group | Treatment | Dose | Route of administration | Number of brains |
|---|---|---|---|---|
| 1 | Vehicle (negative control) | 2 µL per eye | Intravitreal | 14 |
| 2 | Systemic chemotherapy (positive control) | 6 mg/kg etoposide and 34 mg/kg carboplatin | Intraperitoneal | 5 |
| 3 | Reference dose of VCN-01 (100x VCN-01) | 3x10⁹ virus particles in 2 µL per eye (5.7x10⁸ TU) | Intravitreal | 6 |
| 4 | High dose of VCN-01 (10x VCN-01) | 3x10° virus particles in 2 µL per eye (5.7x10⁷ TU) | Intravitreal | 11 |
| 5 | Intermediate dose of VCN-01 (3x VCN-01) | 1x10° virus particles in 2 µL per eye (1.9x10⁷ TU) | Intravitreal | 7 |
| 6 | Low dose of VCN-01 (1x VCN-01) | 3x10⁷ virus particles in 2 µL per eye (5.7x1 0⁶ TU) | Intravitreal | 14 |
| 7 | Very low dose of VCN-01 (1x/10 VCN-01) | 3x10° virus particles in 2 µL per eye (5.7x10⁵ TU) | Intravitreal | 6 |
| 8 | Ultra-low dose of VCN-01 (1x/100 VCN-01) | 3x10° virus particles in 2 µL per eye (5.7x10⁴ TU) | Intravitreal | 5 |

The therapeutic regimen applied to each of the groups mentioned in Table 6 (by way of reference, the cell injection day was -7) was:
- Group 1 (Vehicle): injection on days 1 and 15.
- Group 2 (Systemic chemotherapy): injection of etoposide on days 1, 2 and 3 and injection of carboplatin on day 1. The same administration regimen was repeated on days 22, 23 and 24; and 43, 44 and 45.
- Groups 3 to 8 (administration of different doses of VCN-01). Injection on days 1 and 15.

The presence of human retinoblastoma cells in the brains mentioned in Table 6 was evaluated. Said evaluation was performed by measuring the amount of CRX messenger ribonucleic acid (mRNA) by means of real-time polymerase chain reaction. For this, total ribonucleic acid (RNA) from the brains of the mice was extracted using TRIzol (Life Technologies, Waltham, MA, USA). Next, complementary deoxyribonucleic acid (cDNA) was synthesised with the M-MLV reverse transcriptase system (Life Technologies, Waltham, MA, USA) using 1 µg of the RNA extracted. The amount of CRX mRNA was measured using the common protocol known in the prior art for the real-time polymerase chain reaction; specifically, a reaction was prepared with a volume of 10 µL, using the SYBR® Green PCR Master Mix (Life Technologies, Waltham, MA, USA); the primers and probe shown in Table 7 were used; and the 7500 Sequence Detection System (Applied Biosytems, Foster City, CA, USA) was used to detect the signals in the various cycles of the real-time polymerase chain reaction (first stage of 10 min at 95ºC, followed by 40 cycles of 15 s at 95ºC and 1 min at 60ºC).

As a control, for normalisation of CRX gene expression, the mRNA of the TATA box binding protein (TBP) was used. Its expression was measured in accordance with the protocol indicated above and known in the prior art, and using the primers and probe indicated in Table 7 for that purpose.

**Table 7. Primers and probe used in the real-time polymerase chain reaction to measure the expression of CRX and TBP.**

| Type | Gene | Sequence | SEQ ID NO: |
|---|---|---|---|
| Forward primer | CRX | 5'-AGGTGGCTCTGAAGATCAATCTG-3' | 11 |
| Reverse primer | CRX | 5'-TTAGCCCTCCGGTTCTTGAA-3' | 12 |
| Probe | CRX | 5'-FAM-CTGAGTCCAGGGTTC-MGB-3' | 13 |
| Forward primer | TBP | 5'-GAACATCATGGATCAGAACAACAG-3' | 14 |
| Reverse primer | TBP | 5'-ATTGGTGTTCTGAATAGGCTGTG-3' | 15 |
| Probe | TBP | 5'-FAM-CTGCCACCTTACGCTCAGGGCTTGG-TAMRA-3' | 16 |

The signals obtained were processed by the 2^{ΔΔCt} method, in order to determine the positive brain samples and the negative brain samples. The results obtained are summarised in table 8

**Table 8. Results obtained in the analysis of retinoblastoma brain metastases for the various groups of mice analysed in Example 7.**

| Group | Treatment | Number of available brains analysed | Number of brains with metastasis |
|---|---|---|---|
| 1 | Vehicle (negative control) | 14 | 6 |
| 2 | Systemic chemotherapy (positive control) | 5 | 4 |
| 3 | Reference dose of VCN-01 | 6 | 0 |
| 4 | High dose of VCN-01 | 11 | 0 |
| 5 | Intermediate dose of VCN-01 | 7 | 0 |
| 6 | Low dose of VCN-01 | 14 | 3 |
| 7 | Very low dose of VCN-01 | 6 | 1 |
| 8 | Ultra-low dose of VCN-01 | 5 | 0 |

As can be deduced from Table 8, only four of the 49 mice treated with the various doses of VCN-01 (8%) showed signs of metastasis in the CNS (i.e. human retinoblastoma cells could be detected in the brains of said mice), while 43% of the negative controls and 80% of the mice treated with systemic chemotherapy had metastases in the CNS. None of the mice treated with an intermediate, high and reference dose of VCN-01 developed metastases.

It can therefore be concluded that, in a surprising manner, VCN-01 prevented the spread of retinoblastoma to the CNS.

## Claims

1. Composition comprising an oncolytic adenovirus for use in the treatment of retinoblastoma in the prevention, removal or reduction of metastases, secondary malignancies and/or trilateral retinoblastoma associated with retinoblastoma, **characterised in that** said oncolytic adenovirus comprises:
- a sequence encoding a hyaluronidase enzyme inserted into its genome; and
- replication machinery specific for tumour cells.

2. Composition comprising an oncolytic adenovirus for use according to claim 1, **characterised in that** the composition is administered by intraocular or intravitreal injection.

3. Composition comprising an oncolytic adenovirus for use according to either claim 1 or claim 2, **characterised in that** the retinoblastoma is a retinoblastoma resistant to conventional chemotherapy and/or radiotherapy treatment.

4. Composition comprising an oncolytic adenovirus for use according to claim 3, **characterised in that** the retinoblastoma resistant to conventional chemotherapy and/or radiotherapy treatment is refractory or the result of a relapse.

5. Composition comprising an oncolytic adenovirus for use according to any one of claims 1 to 4, **characterised in that** the oncolytic adenovirus is generated from a human adenovirus serotype 5.

6. Composition comprising an oncolytic adenovirus for use according to any one of claims 1 to 5, **characterised in that** the hyaluronidase enzyme is the human hyaluronidase enzyme PH20.

7. Composition comprising an oncolytic adenovirus for use according to claim 6, **characterised in that** the aforementioned sequence that encodes a hyaluronidase enzyme is SEQ ID NO: 1, from which nucleotides 1471 to 1527, corresponding to the carboxy-terminal domain, have been deleted.

8. Composition comprising an oncolytic adenovirus for use according to any one of claims 1 to 7, **characterised in that** the replication machinery specific for tumour cells is defective replication machinery that can be complemented in tumour cells by both defective copies of the Rb1 gene.

9. Composition comprising an oncolytic adenovirus for use according to claim 8, **characterised in that** the defective replication machinery that can be complemented in tumour cells by both defective copies of the Rb1 gene comprises the deletion Δ24 in the sequence coding for the E1a protein and the insertion of four binding sites to E2F-1 and one binding site to Sp1 into the endogenous promoter of E1a to control the expression of E1a.

10. Composition comprising an oncolytic adenovirus for use according to any one of claims 2 to 9, **characterised in that** the oncolytic adenovirus used in the present invention has the capsid modified such that the binding domain ⁹¹KKTK⁹⁴ of the heparan sulfates present in the adenovirus fibre has been replaced by the domain ⁹¹RGDK⁹⁴.

## Patentansprüche

1. Zusammensetzung, umfassend ein onkolytisches Adenovirus zur Verwendung bei der Behandlung von Retinoblastom bei der Prävention, dem Entfernen oder der Verringerung von Metastasen, sekundären bösartigen Tumoren und/oder einem trilateralen Retinoblastom, das mit dem Retinoblastom assoziiert ist, **dadurch gekennzeichnet, dass** das onkolytische Adenovirus das Folgende umfasst:
- eine Sequenz, die für ein Hyaluronidase-Enzym kodiert, das in sein Genom inseriert ist; und
- eine Replikationsmaschinerie, die für Tumorzellen spezifisch ist.

2. Zusammensetzung, umfassend ein onkolytisches Adenovirus, zur Verwendung nach Anspruch 1, **dadurch gekennzeichnet, dass** die Zusammensetzung durch intraokulare oder intravitreale Injektion verabreicht wird.

3. Zusammensetzung, umfassend ein onkolytisches Adenovirus, zur Verwendung nach Anspruch 1 oder Anspruch 2, **dadurch gekennzeichnet, dass** das Retinoblastom ein Retinoblastom ist, das gegen konventionelle Chemotherapie- und/oder Strahlentherapiebehandlung resistent ist.

4. Zusammensetzung, umfassend ein onkolytisches Adenovirus, zur Verwendung nach Anspruch 3, **dadurch gekennzeichnet, dass** das Retinoblastom, das gegen konventionelle Chemotherapie- und/oder Strahlentherapiebehandlung resistent ist, refraktär oder das Ergebnis eines Rückfalls ist.

5. Zusammensetzung, umfassend ein onkolytisches Adenovirus, zur Verwendung nach einem der Ansprüche 1 bis 4, **dadurch gekennzeichnet, dass** das onkolytische Adenovirus aus einem menschlichen Adenovirus vom Serotyp 5 hergestellt ist.

6. Zusammensetzung, umfassend ein onkolytisches Adenovirus, zur Verwendung nach einem der Ansprüche 1 bis 5, **dadurch gekennzeichnet, dass** das Hyaluronidase-Enzym das menschliche Hyaluronidase-Enzym PH20 ist.

7. Zusammensetzung, umfassend ein onkolytisches Adenovirus, zur Verwendung nach Anspruch 6, **dadurch gekennzeichnet, dass** die vorgenannte Sequenz, die für ein Hyaluronidase-Enzym kodiert, SEQ ID NO: 1 ist, aus der die Nukleotide 1471 bis 1527, die der carboxyterminalen Domäne entsprechen, deletiert wurden.

8. Zusammensetzung, umfassend ein onkolytisches Adenovirus, zur Verwendung nach einem der Ansprüche 1 bis 7, **dadurch gekennzeichnet, dass** die Replikationsmaschinerie, die für Tumorzellen spezifisch ist, eine defekte Replikationsmaschinerie ist, die in Tumorzellen durch beide defekten Kopien des Rb1-Gens komplementiert werden kann.

9. Zusammensetzung, umfassend ein onkolytisches Adenovirus, zur Verwendung nach Anspruch 8, **dadurch gekennzeichnet, dass** die defekte Replikationsmaschinerie, die in Tumorzellen durch die beiden defekten Kopien des Rb1-Gens komplementiert werden kann, die Deletion Δ24 in der Sequenz, die für das E1a-Protein kodiert, und die Insertion von vier Bindungsstellen für E2F-1 und einer Bindungsstelle für Sp1 in den endogenen Promotor von E1a umfasst, um die Expression von E1a zu regulieren.

10. Zusammensetzung, umfassend ein onkolytisches Adenovirus, zur Verwendung nach einem der Ansprüche 2 bis 9, **dadurch gekennzeichnet, dass** bei dem in der vorliegenden Erfindung verwendeten onkolytischen Adenovirus das Kapsid so modifiziert ist, dass die Bindungsdomäne ⁹¹KKTK⁹⁴ der Heparansulfate, die in der Adenovirusfaser vorliegt, durch die Domäne ⁹¹RGDK⁹⁴ ersetzt worden ist.

## Revendications

1. Composition comprenant un adénovirus oncolytique pour une utilisation dans le traitement d'un rétinoblastome lors de la prévention, de l'enlèvement ou de la réduction de métastases, de malignités secondaires et/ou de rétinoblastome trilatéral associé avec un rétinoblastome, **caractérisée en ce que** ledit adénovirus comprend :
- une séquence encodant un enzyme de hyaluronidase insérée dans son génome, et
- un mécanisme de réplication spécifique pour les cellules tumorales.

2. Composition comprenant un adénovirus oncolytique pour une utilisation selon la revendication 1, **caractérisée en ce que** la composition est administrée par injection intraoculaire ou intravitréenne.

3. Composition comprenant un adénovirus oncolytique pour une utilisation selon l'une ou l'autre de la revendication 1 ou de la revendication 2, **caractérisée en ce que** le rétinoblastome est un rétinoblastome résistant à la chimiothérapie conventionnelle et/ou au traitement de radiothérapie.

4. Composition comprenant un adénovirus oncolytique pour une utilisation selon la revendication 3, **caractérisée en ce que** le rétinoblastome résistant à la chimiothérapie conventionnelle et/ou au traitement de radiothérapie est réfractaire ou le résultat d'une récidive.

5. Composition comprenant un adénovirus oncolytique pour une utilisation selon l'une quelconque des revendications 1 à 4, **caractérisée en ce que** l'adénovirus est généré à partir d'un adénovirus humain de sérotype 5.

6. Composition comprenant un adénovirus oncolytique pour une utilisation selon l'une quelconque des revendications 1 à 5, **caractérisée en ce que** l'enzyme de hyaluronidase est l'enzyme de hyaluronidase humain PH20.

7. Composition comprenant un adénovirus oncolytique pour une utilisation selon la revendication 6, **caractérisée en ce que** la séquence mentionnée précédemment qui encode un enzyme de hyaluronidase est SEQ ID NO :1, de laquelle les nucléotides 1471 à 1527, correspondant au domaine carboxy-terminal, ont été supprimés.

8. Composition comprenant un adénovirus oncolytique pour une utilisation selon l'une quelconque des revendications 1 à 7, **caractérisée en ce que** le mécanisme de réplication pour des cellules tumorales est un mécanisme incapable de se répliquer qui peut être complémenté dans les cellules tumorales par les deux copies défectueuses du gène Rb1.

9. Composition comprenant un adénovirus oncolytique pour une utilisation selon la revendication 8, **caractérisée en ce que** le mécanisme incapable de se répliquer qui peut être complémenté dans des cellules tumorales par les deux copies défectueuses du gène Rb1 comprend la suppression Δ24 dans la séquence codant pour la protéine E1a et l'insertion de quatre sites de liaison à E2F-1 et d'un site de liaison à Sp1 dans le promoteur endogène de E1a pour contrôler l'expression de E1a.

10. Composition comprenant un adénovirus oncolytique pour une utilisation selon l'une quelconque des revendications 2 à 9, **caractérisée en ce que** l'adénovirus oncolytique utilisé dans la présente invention comprend une modification du capside de telle sorte que le domaine de liaison ⁹¹KKTK⁹⁴ des sulfates d'héparine présent dans la fibre de l'adénovirus a été remplacé par le domaine ⁹¹RGDK⁹⁴.


### SEQUENCE LISTING

<110> VCN Biosciences SL
<120> Use of viral vectors in the treatment of retinoblastoma
<130> P 1500081
<160> 17
<170> PatentIn version 3.5
<210> 1
   <211> 1530
   <212> DNA
   <213> Artificial Sequence
<220>
   <223> Complete PH20 cDNA sequence comprising the carboxy-terminal domain. (from ATG until stop codon, both included)
<400> 1
   atgggagtgc taaaattcaa gcacatcttt ttcagaagct ttgttaaatc aagtggagta 60
   tcccagatag ttttcacctt ccttctgatt ccatgttgct tgactctgaa tttcagagca 120
   cctcctgtta ttccaaatgt gcctttcctc tgggcctgga atgccccaag tgaattttgt 180
   cttggaaaat ttgatgagcc actagatatg agcctcttct ctttcatagg aagcccccga 240
   ataaacgcca ccgggcaagg tgttacaata ttttatgttg atagacttgg ctactatcct 300
   tacatagatt caatcacagg agtaactgtg aatggaggaa tcccccagaa gatttcctta 360
   caagaccatc tggacaaagc taagaaagac attacatttt atatgccagt agacaatttg 420
   ggaatggctg ttattgactg ggaagaatgg agacccactt gggcaagaaa ctggaaacct 480
   aaagatgttt acaagaatag gtctattgaa ttggttcagc aacaaaatgt acaacttagt 540
   ctcacagagg ccactgagaa agcaaaacaa gaatttgaaa aggcagggaa ggatttcctg 600
   gtagagacta taaaattggg aaaattactt cggccaaatc acttgtgggg ttattatctt 660
   tttccggatt gttacaacca tcactataag aaacccggtt acaatggaag ttgcttcaat 720
   gtagaaataa aaagaaatga tgatctcagc tggttgtgga atgaaagcac tgctctttac 780
   ccatccattt atttgaacac tcagcagtct cctgtagctg ctacactcta tgtgcgcaat 840
   cgagttcggg aagccatcag agtttccaaa atacctgatg caaaaagtcc acttccggtt 900
   tttgcatata cccgcatagt ttttactgat caagttttga aattcctttc tcaagatgaa 960
   cttgtgtata catttggcga aactgttgct ctgggtgctt ctggaattgt aatatgggga 1020
   accctcagta taatgcgaag tatgaaatct tgcttgctcc tagacaatta catggagact 1080
   atactgaatc cttacataat caacgtcaca ctagcagcca aaatgtgtag ccaagtgctt 1140
   tgccaggagc aaggagtgtg tataaggaaa aactggaatt caagtgacta tcttcacctc 1200
   aacccagata attttgctat tcaacttgag aaaggtggaa agttcacagt acgtggaaaa 1260
   ccgacacttg aagacctgga gcaattttct gaaaaatttt attgcagctg ttatagcacc 1320
   ttgagttgta aggagaaagc tgatgtaaaa gacactgatg ctgttgatgt gtgtattgct 1380
   gatggtgtct gtatagatgc ttttctaaaa cctcccatgg agacagaaga acctcaaatt 1440
   ttctacaatg cttcaccctc cacactatct gccacaatgt tcattgttag tattttgttt 1500
   cttatcattt cttctgtagc gagtttgtaa 1530
<210> 2
   <211> 1406
   <212> DNA
   <213> Artificial Sequence
<220>
   <223> Sequence of the modified endogenous promoter of E1a + encoding Ela-Delta24 region in VCN-01 (comprising the four binding sites to E2F-1, the binding site to Sp1 and the encoding region of Ela-Delta24)
<400> 2
   aataagagga agtgaaatct gaataatttt gtgttactca tagcgcgtaa tatttgtcta      60
   gggccgcggg gactttgacc gtttacgtgg agactcgccc aggtgttttt ctcaggtgtt     120
   ttccgcgtac gtcggcggct cgtggctctt tcgcggcaaa aaggatttgg cgcgtaaaag     180
   tggttcgagt acgtcggcgg ctcgtggctc tttcgcggca aaaaggattt ggcgcgtaaa     240
   agtggttcga agtacgtcga ccacaaaccc cgcccagcgt cttgtcattg gcgtcgacgc     300
   tgtacggggt caaagttggc gttttattat tatagtcagc tgacgtgtag tgtatttata     360
   cccggtgagt tcctcaagag gccactcttg agtgccagcg agtagagttt tctcctccga     420
   gccgctccga caccgggact gaaaatgaga catattatct gccacggagg tgttattacc     480
   gaagaaatgg ccgccagtct tttggaccag ctgatcgaag aggtactggc tgataatctt     540
   ccacctccta gccattttga accacctacc cttcacgaac tgtatgattt agacgtgacg     600
   gcccccgaag atcccaacga ggaggcggtt tcgcagattt ttcccgactc tgtaatgttg     660
   gcggtgcagg aagggattga cttactcact tttccgccgg cgcccggttc tccggagccg     720
   cctcaccttt cccggcagcc cgagcagccg gagcagagag ccttgggtcc ggtttctatg     780
   ccaaaccttg taccggaggt gatcgatcca cccagtgacg acgaggatga agagggtgag     840
   gagtttgtgt tagattatgt ggagcacccc gggcacggtt gcaggtcttg tcattatcac     900
   cggaggaata cgggggaccc agatattatg tgttcgcttt gctatatgag gacctgtggc     960
   atgtttgtct acagtaagtg aaaattatgg gcagtgggtg atagagtggt gggtttggtg    1020
   tggtaatttt ttttttaatt tttacagttt tgtggtttaa agaattttgt attgtgattt    1080
   ttttaaaagg tcctgtgtct gaacctgagc ctgagcccga gccagaaccg gagcctgcaa    1140
   gacctacccg ccgtcctaaa atggcgcctg ctatcctgag acgcccgaca tcacctgtgt    1200
   ctagagaatg caatagtagt acggatagct gtgactccgg tccttctaac acacctcctg    1260
   agatacaccc ggtggtcccg ctgtgcccca ttaaaccagt tgccgtgaga gttggtgggc    1320
   gtcgccaggc tgtggaatgt atcgaggact tgcttaacga gcctgggcaa cctttggact    1380
   tgagctgtaa acgccccagg ccataa                                         1406
<210> 3
   <211> 37607
   <212> DNA
   <213> Artificial Sequence
<220>
   <223> Complete sequence of VCN-01 (ICOVIR-17K)
<400> 3
   catcatcaat aatatacctt attttggatt gaagccaata tgataatgag ggggtggagt 60
   ttgtgacgtg gcgcggggcg tgggaacggg gcgggtgacg tagtagtgtg gcggaagtgt 120
   gatgttgcaa gtgtggcgga acacatgtaa gcgacggatg tggcaaaagt gacgtttttg 180
   gtgtgcgccg gtgtacacag gaagtgacaa ttttcgcgcg gttttaggcg gatgttgtag 240
   taaatttggg cgtaaccgag taagatttgg ccattttcgc gggaaaactg aataagagga 300
   agtgaaatct gaataatttt gtgttactca tagcgcgtaa tatttgtcta gggccgcggg 360
   gactttgacc gtttacgtgg agactcgccc aggtgttttt ctcaggtgtt ttccgcgtac 420
   gtcggcggct cgtggctctt tcgcggcaaa aaggatttgg cgcgtaaaag tggttcgagt 480
   acgtcggcgg ctcgtggctc tttcgcggca aaaaggattt ggcgcgtaaa agtggttcga 540
   agtacgtcga ccacaaaccc cgcccagcgt cttgtcattg gcgtcgacgc tgtacggggt 600
   caaagttggc gttttattat tatagtcagc tgacgtgtag tgtatttata cccggtgagt 660
   tcctcaagag gccactcttg agtgccagcg agtagagttt tctcctccga gccgctccga 720
   caccgggact gaaaatgaga catattatct gccacggagg tgttattacc gaagaaatgg 780
   ccgccagtct tttggaccag ctgatcgaag aggtactggc tgataatctt ccacctccta 840
   gccattttga accacctacc cttcacgaac tgtatgattt agacgtgacg gcccccgaag 900
   atcccaacga ggaggcggtt tcgcagattt ttcccgactc tgtaatgttg gcggtgcagg 960
   aagggattga cttactcact tttccgccgg cgcccggttc tccggagccg cctcaccttt 1020
   cccggcagcc cgagcagccg gagcagagag ccttgggtcc ggtttctatg ccaaaccttg 1080
   taccggaggt gatcgatcca cccagtgacg acgaggatga agagggtgag gagtttgtgt 1140
   tagattatgt ggagcacccc gggcacggtt gcaggtcttg tcattatcac cggaggaata 1200
   cgggggaccc agatattatg tgttcgcttt gctatatgag gacctgtggc atgtttgtct 1260
   acagtaagtg aaaattatgg gcagtgggtg atagagtggt gggtttggtg tggtaatttt 1320
   ttttttaatt tttacagttt tgtggtttaa agaattttgt attgtgattt ttttaaaagg 1380
   tcctgtgtct gaacctgagc ctgagcccga gccagaaccg gagcctgcaa gacctacccg 1440
   ccgtcctaaa atggcgcctg ctatcctgag acgcccgaca tcacctgtgt ctagagaatg 1500
   caatagtagt acggatagct gtgactccgg tccttctaac acacctcctg agatacaccc 1560
   ggtggtcccg ctgtgcccca ttaaaccagt tgccgtgaga gttggtgggc gtcgccaggc 1620
   tgtggaatgt atcgaggact tgcttaacga gcctgggcaa cctttggact tgagctgtaa 1680
   acgccccagg ccataaggtg taaacctgtg attgcgtgtg tggttaacgc ctttgtttgc 1740
   tgaatgagtt gatgtaagtt taataaaggg tgagataatg tttaacttgc atggcgtgtt 1800
   aaatggggcg gggcttaaag ggtatataat gcgccgtggg ctaatcttgg ttacatctga 1860
   cctcatggag gcttgggagt gtttggaaga tttttctgct gtgcgtaact tgctggaaca 1920
   gagctctaac agtacctctt ggttttggag gtttctgtgg ggctcatccc aggcaaagtt 1980
   agtctgcaga attaaggagg attacaagtg ggaatttgaa gagcttttga aatcctgtgg 2040
   tgagctgttt gattctttga atctgggtca ccaggcgctt ttccaagaga aggtcatcaa 2100
   gactttggat ttttccacac cggggcgcgc tgcggctgct gttgcttttt tgagttttat 2160
   aaaggataaa tggagcgaag aaacccatct gagcgggggg tacctgctgg attttctggc 2220
   catgcatctg tggagagcgg ttgtgagaca caagaatcgc ctgctactgt tgtcttccgt 2280
   ccgcccggcg ataataccga cggaggagca gcagcagcag caggaggaag ccaggcggcg 2340
   gcggcaggag cagagcccat ggaacccgag agccggcctg gaccctcggg aatgaatgtt 2400
   gtacaggtgg ctgaactgta tccagaactg agacgcattt tgacaattac agaggatggg 2460
   caggggctaa agggggtaaa gagggagcgg ggggcttgtg aggctacaga ggaggctagg 2520
   aatctagctt ttagcttaat gaccagacac cgtcctgagt gtattacttt tcaacagatc 2580
   aaggataatt gcgctaatga gcttgatctg ctggcgcaga agtattccat agagcagctg 2640
   accacttact ggctgcagcc aggggatgat tttgaggagg ctattagggt atatgcaaag 2700
   gtggcactta ggccagattg caagtacaag atcagcaaac ttgtaaatat caggaattgt 2760
   tgctacattt ctgggaacgg ggccgaggtg gagatagata cggaggatag ggtggccttt 2820
   agatgtagca tgataaatat gtggccgggg gtgcttggca tggacggggt ggttattatg 2880
   aatgtaaggt ttactggccc caattttagc ggtacggttt tcctggccaa taccaacctt 2940
   atcctacacg gtgtaagctt ctatgggttt aacaatacct gtgtggaagc ctggaccgat 3000
   gtaagggttc ggggctgtgc cttttactgc tgctggaagg gggtggtgtg tcgccccaaa 3060
   agcagggctt caattaagaa atgcctcttt gaaaggtgta ccttgggtat cctgtctgag 3120
   ggtaactcca gggtgcgcca caatgtggcc tccgactgtg gttgcttcat gctagtgaaa 3180
   agcgtggctg tgattaagca taacatggta tgtggcaact gcgaggacag ggcctctcag 3240
   atgctgacct gctcggacgg caactgtcac ctgctgaaga ccattcacgt agccagccac 3300
   tctcgcaagg cctggccagt gtttgagcat aacatactga cccgctgttc cttgcatttg 3360
   ggtaacagga ggggggtgtt cctaccttac caatgcaatt tgagtcacac taagatattg 3420
   cttgagcccg agagcatgtc caaggtgaac ctgaacgggg tgtttgacat gaccatgaag 3480
   atctggaagg tgctgaggta cgatgagacc cgcaccaggt gcagaccctg cgagtgtggc 3540
   ggtaaacata ttaggaacca gcctgtgatg ctggatgtga ccgaggagct gaggcccgat 3600
   cacttggtgc tggcctgcac ccgcgctgag tttggctcta gcgatgaaga tacagattga 3660
   ggtactgaaa tgtgtgggcg tggcttaagg gtgggaaaga atatataagg tgggggtctt 3720
   atgtagtttt gtatctgttt tgcagcagcc gccgccgcca tgagcaccaa ctcgtttgat 3780
   ggaagcattg tgagctcata tttgacaacg cgcatgcccc catgggccgg ggtgcgtcag 3840
   aatgtgatgg gctccagcat tgatggtcgc cccgtcctgc ccgcaaactc tactaccttg 3900
   acctacgaga ccgtgtctgg aacgccgttg gagactgcag cctccgccgc cgcttcagcc 3960
   gctgcagcca ccgcccgcgg gattgtgact gactttgctt tcctgagccc gcttgcaagc 4020
   agtgcagctt cccgttcatc cgcccgcgat gacaagttga cggctctttt ggcacaattg 4080
   gattctttga cccgggaact taatgtcgtt tctcagcagc tgttggatct gcgccagcag 4140
   gtttctgccc tgaaggcttc ctcccctccc aatgcggttt aaaacataaa taaaaaacca 4200
   gactctgttt ggatttggat caagcaagtg tcttgctgtc tttatttagg ggttttgcgc 4260
   gcgcggtagg cccgggacca gcggtctcgg tcgttgaggg tcctgtgtat tttttccagg 4320
   acgtggtaaa ggtgactctg gatgttcaga tacatgggca taagcccgtc tctggggtgg 4380
   aggtagcacc actgcagagc ttcatgctgc ggggtggtgt tgtagatgat ccagtcgtag 4440
   caggagcgct gggcgtggtg cctaaaaatg tctttcagta gcaagctgat tgccaggggc 4500
   aggcccttgg tgtaagtgtt tacaaagcgg ttaagctggg atgggtgcat acgtggggat 4560
   atgagatgca tcttggactg tatttttagg ttggctatgt tcccagccat atccctccgg 4620
   ggattcatgt tgtgcagaac caccagcaca gtgtatccgg tgcacttggg aaatttgtca 4680
   tgtagcttag aaggaaatgc gtggaagaac ttggagacgc ccttgtgacc tccaagattt 4740
   tccatgcatt cgtccataat gatggcaatg ggcccacggg cggcggcctg ggcgaagata 4800
   tttctgggat cactaacgtc atagttgtgt tccaggatga gatcgtcata ggccattttt 4860
   acaaagcgcg ggcggagggt gccagactgc ggtataatgg ttccatccgg cccaggggcg 4920
   tagttaccct cacagatttg catttcccac gctttgagtt cagatggggg gatcatgtct 4980
   acctgcgggg cgatgaagaa aacggtttcc ggggtagggg agatcagctg ggaagaaagc 5040
   aggttcctga gcagctgcga cttaccgcag ccggtgggcc cgtaaatcac acctattacc 5100
   ggctgcaact ggtagttaag agagctgcag ctgccgtcat ccctgagcag gggggccact 5160
   tcgttaagca tgtccctgac tcgcatgttt tccctgacca aatccgccag aaggcgctcg 5220
   ccgcccagcg atagcagttc ttgcaaggaa gcaaagtttt tcaacggttt gagaccgtcc 5280
   gccgtaggca tgcttttgag cgtttgacca agcagttcca ggcggtccca cagctcggtc 5340
   acctgctcta cggcatctcg atccagcata tctcctcgtt tcgcgggttg gggcggcttt 5400
   cgctgtacgg cagtagtcgg tgctcgtcca gacgggccag ggtcatgtct ttccacgggc 5460
   gcagggtcct cgtcagcgta gtctgggtca cggtgaaggg gtgcgctccg ggctgcgcgc 5520
   tggccagggt gcgcttgagg ctggtcctgc tggtgctgaa gcgctgccgg tcttcgccct 5580
   gcgcgtcggc caggtagcat ttgaccatgg tgtcatagtc cagcccctcc gcggcgtggc 5640
   ccttggcgcg cagcttgccc ttggaggagg cgccgcacga ggggcagtgc agacttttga 5700
   gggcgtagag cttgggcgcg agaaataccg attccgggga gtaggcatcc gcgccgcagg 5760
   ccccgcagac ggtctcgcat tccacgagcc aggtgagctc tggccgttcg gggtcaaaaa 5820
   ccaggtttcc cccatgcttt ttgatgcgtt tcttacctct ggtttccatg agccggtgtc 5880
   cacgctcggt gacgaaaagg ctgtccgtgt ccccgtatac agacttgaga ggcctgtcct 5940
   cgagcggtgt tccgcggtcc tcctcgtata gaaactcgga ccactctgag acaaaggctc 6000
   gcgtccaggc cagcacgaag gaggctaagt gggaggggta gcggtcgttg tccactaggg 6060
   ggtccactcg ctccagggtg tgaagacaca tgtcgccctc ttcggcatca aggaaggtga 6120
   ttggtttgta ggtgtaggcc acgtgaccgg gtgttcctga aggggggcta taaaaggggg 6180
   tgggggcgcg ttcgtcctca ctctcttccg catcgctgtc tgcgagggcc agctgttggg 6240
   gtgagtactc cctctgaaaa gcgggcatga cttctgcgct aagattgtca gtttccaaaa 6300
   acgaggagga tttgatattc acctggcccg cggtgatgcc tttgagggtg gccgcatcca 6360
   tctggtcaga aaagacaatc tttttgttgt caagcttggt ggcaaacgac ccgtagaggg 6420
   cgttggacag caacttggcg atggagcgca gggtttggtt tttgtcgcga tcggcgcgct 6480
   ccttggccgc gatgtttagc tgcacgtatt cgcgcgcaac gcaccgccat tcgggaaaga 6540
   cggtggtgcg ctcgtcgggc accaggtgca cgcgccaacc gcggttgtgc agggtgacaa 6600
   ggtcaacgct ggtggctacc tctccgcgta ggcgctcgtt ggtccagcag aggcggccgc 6660
   ccttgcgcga gcagaatggc ggtagggggt ctagctgcgt ctcgtccggg gggtctgcgt 6720
   ccacggtaaa gaccccgggc agcaggcgcg cgtcgaagta gtctatcttg catccttgca 6780
   agtctagcgc ctgctgccat gcgcgggcgg caagcgcgcg ctcgtatggg ttgagtgggg 6840
   gaccccatgg catggggtgg gtgagcgcgg aggcgtacat gccgcaaatg tcgtaaacgt 6900
   agaggggctc tctgagtatt ccaagatatg tagggtagca tcttccaccg cggatgctgg 6960
   cgcgcacgta atcgtatagt tcgtgcgagg gagcgaggag gtcgggaccg aggttgctac 7020
   gggcgggctg ctctgctcgg aagactatct gcctgaagat ggcatgtgag ttggatgata 7080
   tggttggacg ctggaagacg ttgaagctgg cgtctgtgag acctaccgcg tcacgcacga 7140
   aggaggcgta ggagtcgcgc agcttgttga ccagctcggc ggtgacctgc acgtctaggg 7200
   cgcagtagtc cagggtttcc ttgatgatgt catacttatc ctgtcccttt tttttccaca 7260
   gctcgcggtt gaggacaaac tcttcgcggt ctttccagta ctcttggatc ggaaacccgt 7320
   cggcctccga acggtaagag cctagcatgt agaactggtt gacggcctgg taggcgcagc 7380
   atcccttttc tacgggtagc gcgtatgcct gcgcggcctt ccggagcgag gtgtgggtga 7440
   gcgcaaaggt gtccctgacc atgactttga ggtactggta tttgaagtca gtgtcgtcgc 7500
   atccgccctg ctcccagagc aaaaagtccg tgcgcttttt ggaacgcgga tttggcaggg 7560
   cgaaggtgac atcgttgaag agtatctttc ccgcgcgagg cataaagttg cgtgtgatgc 7620
   ggaagggtcc cggcacctcg gaacggttgt taattacctg ggcggcgagc acgatctcgt 7680
   caaagccgtt gatgttgtgg cccacaatgt aaagttccaa gaagcgcggg atgcccttga 7740
   tggaaggcaa ttttttaagt tcctcgtagg tgagctcttc aggggagctg agcccgtgct 7800
   ctgaaagggc ccagtctgca agatgagggt tggaagcgac gaatgagctc cacaggtcac 7860
   gggccattag catttgcagg tggtcgcgaa aggtcctaaa ctggcgacct atggccattt 7920
   tttctggggt gatgcagtag aaggtaagcg ggtcttgttc ccagcggtcc catccaaggt 7980
   tcgcggctag gtctcgcgcg gcagtcacta gaggctcatc tccgccgaac ttcatgacca 8040
   gcatgaaggg cacgagctgc ttcccaaagg cccccatcca agtataggtc tctacatcgt 8100
   aggtgacaaa gagacgctcg gtgcgaggat gcgagccgat cgggaagaac tggatctccc 8160
   gccaccaatt ggaggagtgg ctattgatgt ggtgaaagta gaagtccctg cgacgggccg 8220
   aacactcgtg ctggcttttg taaaaacgtg cgcagtactg gcagcggtgc acgggctgta 8280
   catcctgcac gaggttgacc tgacgaccgc gcacaaggaa gcagagtggg aatttgagcc 8340
   cctcgcctgg cgggtttggc tggtggtctt ctacttcggc tgcttgtcct tgaccgtctg 8400
   gctgctcgag gggagttacg gtggatcgga ccaccacgcc gcgcgagccc aaagtccaga 8460
   tgtccgcgcg cggcggtcgg agcttgatga caacatcgcg cagatgggag ctgtccatgg 8520
   tctggagctc ccgcggcgtc aggtcaggcg ggagctcctg caggtttacc tcgcatagac 8580
   gggtcagggc gcgggctaga tccaggtgat acctaatttc caggggctgg ttggtggcgg 8640
   cgtcgatggc ttgcaagagg ccgcatcccc gcggcgcgac tacggtaccg cgcggcgggc 8700
   ggtgggccgc gggggtgtcc ttggatgatg catctaaaag cggtgacgcg ggcgagcccc 8760
   cggaggtagg gggggctccg gacccgccgg gagagggggc aggggcacgt cggcgccgcg 8820
   cgcgggcagg agctggtgct gcgcgcgtag gttgctggcg aacgcgacga cgcggcggtt 8880
   gatctcctga atctggcgcc tctgcgtgaa gacgacgggc ccggtgagct tgaacctgaa 8940
   agagagttcg acagaatcaa tttcggtgtc gttgacggcg gcctggcgca aaatctcctg 9000
   cacgtctcct gagttgtctt gataggcgat ctcggccatg aactgctcga tctcttcctc 9060
   ctggagatct ccgcgtccgg ctcgctccac ggtggcggcg aggtcgttgg aaatgcgggc 9120
   catgagctgc gagaaggcgt tgaggcctcc ctcgttccag acgcggctgt agaccacgcc 9180
   cccttcggca tcgcgggcgc gcatgaccac ctgcgcgaga ttgagctcca cgtgccgggc 9240
   gaagacggcg tagtttcgca ggcgctgaaa gaggtagttg agggtggtgg cggtgtgttc 9300
   tgccacgaag aagtacataa cccagcgtcg caacgtggat tcgttgatat cccccaaggc 9360
   ctcaaggcgc tccatggcct cgtagaagtc cacggcgaag ttgaaaaact gggagttgcg 9420
   cgccgacacg gttaactcct cctccagaag acggatgagc tcggcgacag tgtcgcgcac 9480
   ctcgcgctca aaggctacag gggcctcttc ttcttcttca atctcctctt ccataagggc 9540
   ctccccttct tcttcttctg gcggcggtgg gggagggggg acacggcggc gacgacggcg 9600
   caccgggagg cggtcgacaa agcgctcgat catctccccg cggcgacggc gcatggtctc 9660
   ggtgacggcg cggccgttct cgcgggggcg cagttggaag acgccgcccg tcatgtcccg 9720
   gttatgggtt ggcgggggcc tgccatgcgg cagggatacg gcgctaacga tgcatctcaa 9780
   caattgttgt gtaggtactc cgccgccgag ggacctgagc gagtccgcat cgaccggatc 9840
   ggaaaacctc tcgagaaagg cgtctaacca gtcacagtcg caaggtaggc tgagcaccgt 9900
   ggcgggcggc agcgggcggc ggtcggggtt gtttctggcg gaggtgctgc tgatgatgta 9960
   attaaagtag gcggtcttga gacggcggat ggtcgacaga agcaccatgt ccttgggtcc 10020
   ggcctgctga atgcgcaggc ggtcggccat gccccaggct tcgttttgac atcggcgcag 10080
   gtctttgtag tagtcttgca tgagcctttc taccggcact tcttcttctc cttcctcttg 10140
   tcctgcatct cttgcatcta tcgctgcggc ggcggcggag tttggccgta ggtggcgccc 10200
   tcttcctccc atgcgtgtga ccccgaagcc cctcatcggc tgaagcaggg ctaggtcggc 10260
   gacaacgcgc tcggctaata tggcctgctg cacctgcgtg agggtagact ggaagtcatc 10320
   catgtccaca aagcggtggt atgcgcccgt gttgatggtg taagtgcagt tggccataac 10380
   ggaccagtta acggtctggt gacccggctg cgagagctcg gtgtacctga gacgcgagta 10440
   agccctcgag tcaaatacgt agtcgttgca agtccgcacc aggtactggt atcccaccaa 10500
   aaagtgcggc ggcggctggc ggtagagggg ccagcgtagg gtggccgggg ctccgggggc 10560
   gagatcttcc aacataaggc gatgatatcc gtagatgtac ctggacatcc aggtgatgcc 10620
   ggcggcggtg gtggaggcgc gcggaaagtc gcggacgcgg ttccagatgt tgcgcagcgg 10680
   caaaaagtgc tccatggtcg ggacgctctg gccggtcagg cgcgcgcaat cgttgacgct 10740
   ctagaccgtg caaaaggaga gcctgtaagc gggcactctt ccgtggtctg gtggataaat 10800
   tcgcaagggt atcatggcgg acgaccgggg ttcgagcccc gtatccggcc gtccgccgtg 10860
   atccatgcgg ttaccgcccg cgtgtcgaac ccaggtgtgc gacgtcagac aacgggggag 10920
   tgctcctttt ggcttccttc caggcgcggc ggctgctgcg ctagcttttt tggccactgg 10980
   ccgcgcgcag cgtaagcggt taggctggaa agcgaaagca ttaagtggct cgctccctgt 11040
   agccggaggg ttattttcca agggttgagt cgcgggaccc ccggttcgag tctcggaccg 11100
   gccggactgc ggcgaacggg ggtttgcctc cccgtcatgc aagaccccgc ttgcaaattc 11160
   ctccggaaac agggacgagc cccttttttg cttttcccag atgcatccgg tgctgcggca 11220
   gatgcgcccc cctcctcagc agcggcaaga gcaagagcag cggcagacat gcagggcacc 11280
   ctcccctcct cctaccgcgt caggaggggc gacatccgcg gttgacgcgg cagcagatgg 11340
   tgattacgaa cccccgcggc gccgggcccg gcactacctg gacttggagg agggcgaggg 11400
   cctggcgcgg ctaggagcgc cctctcctga gcggcaccca agggtgcagc tgaagcgtga 11460
   tacgcgtgag gcgtacgtgc cgcggcagaa cctgtttcgc gaccgcgagg gagaggagcc 11520
   cgaggagatg cgggatcgaa agttccacgc agggcgcgag ctgcggcatg gcctgaatcg 11580
   cgagcggttg ctgcgcgagg aggactttga gcccgacgcg cgaaccggga ttagtcccgc 11640
   gcgcgcacac gtggcggccg ccgacctggt aaccgcatac gagcagacgg tgaaccagga 11700
   gattaacttt caaaaaagct ttaacaacca cgtgcgtacg cttgtggcgc gcgaggaggt 11760
   ggctatagga ctgatgcatc tgtgggactt tgtaagcgcg ctggagcaaa acccaaatag 11820
   caagccgctc atggcgcagc tgttccttat agtgcagcac agcagggaca acgaggcatt 11880
   cagggatgcg ctgctaaaca tagtagagcc cgagggccgc tggctgctcg atttgataaa 11940
   catcctgcag agcatagtgg tgcaggagcg cagcttgagc ctggctgaca aggtggccgc 12000
   catcaactat tccatgctta gcctgggcaa gttttacgcc cgcaagatat accatacccc 12060
   ttacgttccc atagacaagg aggtaaagat cgaggggttc tacatgcgca tggcgctgaa 12120
   ggtgcttacc ttgagcgacg acctgggcgt ttatcgcaac gagcgcatcc acaaggccgt 12180
   gagcgtgagc cggcggcgcg agctcagcga ccgcgagctg atgcacagcc tgcaaagggc 12240
   cctggctggc acgggcagcg gcgatagaga ggccgagtcc tactttgacg cgggcgctga 12300
   cctgcgctgg gccccaagcc gacgcgccct ggaggcagct ggggccggac ctgggctggc 12360
   ggtggcaccc gcgcgcgctg gcaacgtcgg cggcgtggag gaatatgacg aggacgatga 12420
   gtacgagcca gaggacggcg agtactaagc ggtgatgttt ctgatcagat gatgcaagac 12480
   gcaacggacc cggcggtgcg ggcggcgctg cagagccagc cgtccggcct taactccacg 12540
   gacgactggc gccaggtcat ggaccgcatc atgtcgctga ctgcgcgcaa tcctgacgcg 12600
   ttccggcagc agccgcaggc caaccggctc tccgcaattc tggaagcggt ggtcccggcg 12660
   cgcgcaaacc ccacgcacga gaaggtgctg gcgatcgtaa acgcgctggc cgaaaacagg 12720
   gccatccggc ccgacgaggc cggcctggtc tacgacgcgc tgcttcagcg cgtggctcgt 12780
   tacaacagcg gcaacgtgca gaccaacctg gaccggctgg tgggggatgt gcgcgaggcc 12840
   gtggcgcagc gtgagcgcgc gcagcagcag ggcaacctgg gctccatggt tgcactaaac 12900
   gccttcctga gtacacagcc cgccaacgtg ccgcggggac aggaggacta caccaacttt 12960
   gtgagcgcac tgcggctaat ggtgactgag acaccgcaaa gtgaggtgta ccagtctggg 13020
   ccagactatt ttttccagac cagtagacaa ggcctgcaga ccgtaaacct gagccaggct 13080
   ttcaaaaact tgcaggggct gtggggggtg cgggctccca caggcgaccg cgcgaccgtg 13140
   tctagcttgc tgacgcccaa ctcgcgcctg ttgctgctgc taatagcgcc cttcacggac 13200
   agtggcagcg tgtcccggga cacataccta ggtcacttgc tgacactgta ccgcgaggcc 13260
   ataggtcagg cgcatgtgga cgagcatact ttccaggaga ttacaagtgt cagccgcgcg 13320
   ctggggcagg aggacacggg cagcctggag gcaaccctaa actacctgct gaccaaccgg 13380
   cggcagaaga tcccctcgtt gcacagttta aacagcgagg aggagcgcat tttgcgctac 13440
   gtgcagcaga gcgtgagcct taacctgatg cgcgacgggg taacgcccag cgtggcgctg 13500
   gacatgaccg cgcgcaacat ggaaccgggc atgtatgcct caaaccggcc gtttatcaac 13560
   cgcctaatgg actacttgca tcgcgcggcc gccgtgaacc ccgagtattt caccaatgcc 13620
   atcttgaacc cgcactggct accgccccct ggtttctaca ccgggggatt cgaggtgccc 13680
   gagggtaacg atggattcct ctgggacgac atagacgaca gcgtgttttc cccgcaaccg 13740
   cagaccctgc tagagttgca acagcgcgag caggcagagg cggcgctgcg aaaggaaagc 13800
   ttccgcaggc caagcagctt gtccgatcta ggcgctgcgg ccccgcggtc agatgctagt 13860
   agcccatttc caagcttgat agggtctctt accagcactc gcaccacccg cccgcgcctg 13920
   ctgggcgagg aggagtacct aaacaactcg ctgctgcagc cgcagcgcga aaaaaacctg 13980
   cctccggcat ttcccaacaa cgggatagag agcctagtgg acaagatgag tagatggaag 14040
   acgtacgcgc aggagcacag ggacgtgcca ggcccgcgcc cgcccacccg tcgtcaaagg 14100
   cacgaccgtc agcggggtct ggtgtgggag gacgatgact cggcagacga cagcagcgtc 14160
   ctggatttgg gagggagtgg caacccgttt gcgcaccttc gccccaggct ggggagaatg 14220
   ttttaaaaaa aaaaaaagca tgatgcaaaa taaaaaactc accaaggcca tggcaccgag 14280
   cgttggtttt cttgtattcc ccttagtatg cggcgcgcgg cgatgtatga ggaaggtcct 14340
   cctccctcct acgagagtgt ggtgagcgcg gcgccagtgg cggcggcgct gggttctccc 14400
   ttcgatgctc ccctggaccc gccgtttgtg cctccgcggt acctgcggcc taccgggggg 14460
   agaaacagca tccgttactc tgagttggca cccctattcg acaccacccg tgtgtacctg 14520
   gtggacaaca agtcaacgga tgtggcatcc ctgaactacc agaacgacca cagcaacttt 14580
   ctgaccacgg tcattcaaaa caatgactac agcccggggg aggcaagcac acagaccatc 14640
   aatcttgacg accggtcgca ctggggcggc gacctgaaaa ccatcctgca taccaacatg 14700
   ccaaatgtga acgagttcat gtttaccaat aagtttaagg cgcgggtgat ggtgtcgcgc 14760
   ttgcctacta aggacaatca ggtggagctg aaatacgagt gggtggagtt cacgctgccc 14820
   gagggcaact actccgagac catgaccata gaccttatga acaacgcgat cgtggagcac 14880
   tacttgaaag tgggcagaca gaacggggtt ctggaaagcg acatcggggt aaagtttgac 14940
   acccgcaact tcagactggg gtttgacccc gtcactggtc ttgtcatgcc tggggtatat 15000
   acaaacgaag ccttccatcc agacatcatt ttgctgccag gatgcggggt ggacttcacc 15060
   cacagccgcc tgagcaactt gttgggcatc cgcaagcggc aacccttcca ggagggcttt 15120
   aggatcacct acgatgatct ggagggtggt aacattcccg cactgttgga tgtggacgcc 15180
   taccaggcga gcttgaaaga tgacaccgaa cagggcgggg gtggcgcagg cggcagcaac 15240
   agcagtggca gcggcgcgga agagaactcc aacgcggcag ccgcggcaat gcagccggtg 15300
   gaggacatga acgatcatgc cattcgcggc gacacctttg ccacacgggc tgaggagaag 15360
   cgcgctgagg ccgaagcagc ggccgaagct gccgcccccg ctgcgcaacc cgaggtcgag 15420
   aagcctcaga agaaaccggt gatcaaaccc ctgacagagg acagcaagaa acgcagttac 15480
   aacctaataa gcaatgacag caccttcacc cagtaccgca gctggtacct tgcatacaac 15540
   tacggcgacc ctcagaccgg aatccgctca tggaccctgc tttgcactcc tgacgtaacc 15600
   tgcggctcgg agcaggtcta ctggtcgttg ccagacatga tgcaagaccc cgtgaccttc 15660
   cgctccacgc gccagatcag caactttccg gtggtgggcg ccgagctgtt gcccgtgcac 15720
   tccaagagct tctacaacga ccaggccgtc tactcccaac tcatccgcca gtttacctct 15780
   ctgacccacg tgttcaatcg ctttcccgag aaccagattt tggcgcgccc gccagccccc 15840
   accatcacca ccgtcagtga aaacgttcct gctctcacag atcacgggac gctaccgctg 15900
   cgcaacagca tcggaggagt ccagcgagtg accattactg acgccagacg ccgcacctgc 15960
   ccctacgttt acaaggccct gggcatagtc tcgccgcgcg tcctatcgag ccgcactttt 16020
   tgagcaagca tgtccatcct tatatcgccc agcaataaca caggctgggg cctgcgcttc 16080
   ccaagcaaga tgtttggcgg ggccaagaag cgctccgacc aacacccagt gcgcgtgcgc 16140
   gggcactacc gcgcgccctg gggcgcgcac aaacgcggcc gcactgggcg caccaccgtc 16200
   gatgacgcca tcgacgcggt ggtggaggag gcgcgcaact acacgcccac gccgccacca 16260
   gtgtccacag tggacgcggc cattcagacc gtggtgcgcg gagcccggcg ctatgctaaa 16320
   atgaagagac ggcggaggcg cgtagcacgt cgccaccgcc gccgacccgg cactgccgcc 16380
   caacgcgcgg cggcggccct gcttaaccgc gcacgtcgca ccggccgacg ggcggccatg 16440
   cgggccgctc gaaggctggc cgcgggtatt gtcactgtgc cccccaggtc caggcgacga 16500
   gcggccgccg cagcagccgc ggccattagt gctatgactc agggtcgcag gggcaacgtg 16560
   tattgggtgc gcgactcggt tagcggcctg cgcgtgcccg tgcgcacccg ccccccgcgc 16620
   aactagattg caagaaaaaa ctacttagac tcgtactgtt gtatgtatcc agcggcggcg 16680
   gcgcgcaacg aagctatgtc caagcgcaaa atcaaagaag agatgctcca ggtcatcgcg 16740
   ccggagatct atggcccccc gaagaaggaa gagcaggatt acaagccccg aaagctaaag 16800
   cgggtcaaaa agaaaaagaa agatgatgat gatgaacttg acgacgaggt ggaactgctg 16860
   cacgctaccg cgcccaggcg acgggtacag tggaaaggtc gacgcgtaaa acgtgttttg 16920
   cgacccggca ccaccgtagt ctttacgccc ggtgagcgct ccacccgcac ctacaagcgc 16980
   gtgtatgatg aggtgtacgg cgacgaggac ctgcttgagc aggccaacga gcgcctcggg 17040
   gagtttgcct acggaaagcg gcataaggac atgctggcgt tgccgctgga cgagggcaac 17100
   ccaacaccta gcctaaagcc cgtaacactg cagcaggtgc tgcccgcgct tgcaccgtcc 17160
   gaagaaaagc gcggcctaaa gcgcgagtct ggtgacttgg cacccaccgt gcagctgatg 17220
   gtacccaagc gccagcgact ggaagatgtc ttggaaaaaa tgaccgtgga acctgggctg 17280
   gagcccgagg tccgcgtgcg gccaatcaag caggtggcgc cgggactggg cgtgcagacc 17340
   gtggacgttc agatacccac taccagtagc accagtattg ccaccgccac agagggcatg 17400
   gagacacaaa cgtccccggt tgcctcagcg gtggcggatg ccgcggtgca ggcggtcgct 17460
   gcggccgcgt ccaagacctc tacggaggtg caaacggacc cgtggatgtt tcgcgtttca 17520
   gccccccggc gcccgcgccg ttcgaggaag tacggcgccg ccagcgcgct actgcccgaa 17580
   tatgccctac atccttccat tgcgcctacc cccggctatc gtggctacac ctaccgcccc 17640
   agaagacgag caactacccg acgccgaacc accactggaa cccgccgccg ccgtcgccgt 17700
   cgccagcccg tgctggcccc gatttccgtg cgcagggtgg ctcgcgaagg aggcaggacc 17760
   ctggtgctgc caacagcgcg ctaccacccc agcatcgttt aaaagccggt ctttgtggtt 17820
   cttgcagata tggccctcac ctgccgcctc cgtttcccgg tgccgggatt ccgaggaaga 17880
   atgcaccgta ggaggggcat ggccggccac ggcctgacgg gcggcatgcg tcgtgcgcac 17940
   caccggcggc ggcgcgcgtc gcaccgtcgc atgcgcggcg gtatcctgcc cctccttatt 18000
   ccactgatcg ccgcggcgat tggcgccgtg cccggaattg catccgtggc cttgcaggcg 18060
   cagagacact gattaaaaac aagttgcatg tggaaaaatc aaaataaaaa gtctggactc 18120
   tcacgctcgc ttggtcctgt aactattttg tagaatggaa gacatcaact ttgcgtctct 18180
   ggccccgcga cacggctcgc gcccgttcat gggaaactgg caagatatcg gcaccagcaa 18240
   tatgagcggt ggcgccttca gctggggctc gctgtggagc ggcattaaaa atttcggttc 18300
   caccgttaag aactatggca gcaaggcctg gaacagcagc acaggccaga tgctgaggga 18360
   taagttgaaa gagcaaaatt tccaacaaaa ggtggtagat ggcctggcct ctggcattag 18420
   cggggtggtg gacctggcca accaggcagt gcaaaataag attaacagta agcttgatcc 18480
   ccgccctccc gtagaggagc ctccaccggc cgtggagaca gtgtctccag aggggcgtgg 18540
   cgaaaagcgt ccgcgccccg acagggaaga aactctggtg acgcaaatag acgagcctcc 18600
   ctcgtacgag gaggcactaa agcaaggcct gcccaccacc cgtcccatcg cgcccatggc 18660
   taccggagtg ctgggccagc acacacccgt aacgctggac ctgcctcccc ccgccgacac 18720
   ccagcagaaa cctgtgctgc caggcccgac cgccgttgtt gtaacccgtc ctagccgcgc 18780
   gtccctgcgc cgcgccgcca gcggtccgcg atcgttgcgg cccgtagcca gtggcaactg 18840
   gcaaagcaca ctgaacagca tcgtgggtct gggggtgcaa tccctgaagc gccgacgatg 18900
   cttctgatag ctaacgtgtc gtatgtgtgt catgtatgcg tccatgtcgc cgccagagga 18960
   gctgctgagc cgccgcgcgc ccgctttcca agatggctac cccttcgatg atgccgcagt 19020
   ggtcttacat gcacatctcg ggccaggacg cctcggagta cctgagcccc gggctggtgc 19080
   agtttgcccg cgccaccgag acgtacttca gcctgaataa caagtttaga aaccccacgg 19140
   tggcgcctac gcacgacgtg accacagacc ggtcccagcg tttgacgctg cggttcatcc 19200
   ctgtggaccg tgaggatact gcgtactcgt acaaggcgcg gttcacccta gctgtgggtg 19260
   ataaccgtgt gctggacatg gcttccacgt actttgacat ccgcggcgtg ctggacaggg 19320
   gccctacttt taagccctac tctggcactg cctacaacgc cctggctccc aagggtgccc 19380
   caaatccttg cgaatgggat gaagctgcta ctgctcttga aataaaccta gaagaagagg 19440
   acgatgacaa cgaagacgaa gtagacgagc aagctgagca gcaaaaaact cacgtatttg 19500
   ggcaggcgcc ttattctggt ataaatatta caaaggaggg tattcaaata ggtgtcgaag 19560
   gtcaaacacc taaatatgcc gataaaacat ttcaacctga acctcaaata ggagaatctc 19620
   agtggtacga aacagaaatt aatcatgcag ctgggagagt cctaaaaaag actaccccaa 19680
   tgaaaccatg ttacggttca tatgcaaaac ccacaaatga aaatggaggg caaggcattc 19740
   ttgtaaagca acaaaatgga aagctagaaa gtcaagtgga aatgcaattt ttctcaacta 19800
   ctgaggcagc cgcaggcaat ggtgataact tgactcctaa agtggtattg tacagtgaag 19860
   atgtagatat agaaacccca gacactcata tttcttacat gcccactatt aaggaaggta 19920
   actcacgaga actaatgggc caacaatcta tgcccaacag gcctaattac attgctttta 19980
   gggacaattt tattggtcta atgtattaca acagcacggg taatatgggt gttctggcgg 20040
   gccaagcatc gcagttgaat gctgttgtag atttgcaaga cagaaacaca gagctttcat 20100
   accagctttt gcttgattcc attggtgata gaaccaggta cttttctatg tggaatcagg 20160
   ctgttgacag ctatgatcca gatgttagaa ttattgaaaa tcatggaact gaagatgaac 20220
   ttccaaatta ctgctttcca ctgggaggtg tgattaatac agagactctt accaaggtaa 20280
   aacctaaaac aggtcaggaa aatggatggg aaaaagatgc tacagaattt tcagataaaa 20340
   atgaaataag agttggaaat aattttgcca tggaaatcaa tctaaatgcc aacctgtgga 20400
   gaaatttcct gtactccaac atagcgctgt atttgcccga caagctaaag tacagtcctt 20460
   ccaacgtaaa aatttctgat aacccaaaca cctacgacta catgaacaag cgagtggtgg 20520
   ctcccgggct agtggactgc tacattaacc ttggagcacg ctggtccctt gactatatgg 20580
   acaacgtcaa cccatttaac caccaccgca atgctggcct gcgctaccgc tcaatgttgc 20640
   tgggcaatgg tcgctatgtg cccttccaca tccaggtgcc tcagaagttc tttgccatta 20700
   aaaacctcct tctcctgccg ggctcataca cctacgagtg gaacttcagg aaggatgtta 20760
   acatggttct gcagagctcc ctaggaaatg acctaagggt tgacggagcc agcattaagt 20820
   ttgatagcat ttgcctttac gccaccttct tccccatggc ccacaacacc gcctccacgc 20880
   ttgaggccat gcttagaaac gacaccaacg accagtcctt taacgactat ctctccgccg 20940
   ccaacatgct ctaccctata cccgccaacg ctaccaacgt gcccatatcc atcccctccc 21000
   gcaactgggc ggctttccgc ggctgggcct tcacgcgcct taagactaag gaaaccccat 21060
   cactgggctc gggctacgac ccttattaca cctactctgg ctctataccc tacctagatg 21120
   gaacctttta cctcaaccac acctttaaga aggtggccat tacctttgac tcttctgtca 21180
   gctggcctgg caatgaccgc ctgcttaccc ccaacgagtt tgaaattaag cgctcagttg 21240
   acggggaggg ttacaacgtt gcccagtgta acatgaccaa agactggttc ctggtacaaa 21300
   tgctagctaa ctataacatt ggctaccagg gcttctatat cccagagagc tacaaggacc 21360
   gcatgtactc cttctttaga aacttccagc ccatgagccg tcaggtggtg gatgatacta 21420
   aatacaagga ctaccaacag gtgggcatcc tacaccaaca caacaactct ggatttgttg 21480
   gctaccttgc ccccaccatg cgcgaaggac aggcctaccc tgctaacttc ccctatccgc 21540
   ttataggcaa gaccgcagtt gacagcatta cccagaaaaa gtttctttgc gatcgcaccc 21600
   tttggcgcat cccattctcc agtaacttta tgtccatggg cgcactcaca gacctgggcc 21660
   aaaaccttct ctacgccaac tccgcccacg cgctagacat gacttttgag gtggatccca 21720
   tggacgagcc cacccttctt tatgttttgt ttgaagtctt tgacgtggtc cgtgtgcacc 21780
   agccgcaccg cggcgtcatc gaaaccgtgt acctgcgcac gcccttctcg gccggcaacg 21840
   ccacaacata aagaagcaag caacatcaac aacagctgcc gccatgggct ccagtgagca 21900
   ggaactgaaa gccattgtca aagatcttgg ttgtgggcca tattttttgg gcacctatga 21960
   caagcgcttt ccaggctttg tttctccaca caagctcgcc tgcgccatag tcaatacggc 22020
   cggtcgcgag actgggggcg tacactggat ggcctttgcc tggaacccgc actcaaaaac 22080
   atgctacctc tttgagccct ttggcttttc tgaccagcga ctcaagcagg tttaccagtt 22140
   tgagtacgag tcactcctgc gccgtagcgc cattgcttct tcccccgacc gctgtataac 22200
   gctggaaaag tccacccaaa gcgtacaggg gcccaactcg gccgcctgtg gactattctg 22260
   ctgcatgttt ctccacgcct ttgccaactg gccccaaact cccatggatc acaaccccac 22320
   catgaacctt attaccgggg tacccaactc catgctcaac agtccccagg tacagcccac 22380
   cctgcgtcgc aaccaggaac agctctacag cttcctggag cgccactcgc cctacttccg 22440
   cagccacagt gcgcagatta ggagcgccac ttctttttgt cacttgaaaa acatgtaaaa 22500
   ataatgtact agagacactt tcaataaagg caaatgcttt tatttgtaca ctctcgggtg 22560
   attatttacc cccacccttg ccgtctgcgc cgtttaaaaa tcaaaggggt tctgccgcgc 22620
   atcgctatgc gccactggca gggacacgtt gcgatactgg tgtttagtgc tccacttaaa 22680
   ctcaggcaca accatccgcg gcagctcggt gaagttttca ctccacaggc tgcgcaccat 22740
   caccaacgcg tttagcaggt cgggcgccga tatcttgaag tcgcagttgg ggcctccgcc 22800
   ctgcgcgcgc gagttgcgat acacagggtt gcagcactgg aacactatca gcgccgggtg 22860
   gtgcacgctg gccagcacgc tcttgtcgga gatcagatcc gcgtccaggt cctccgcgtt 22920
   gctcagggcg aacggagtca actttggtag ctgccttccc aaaaagggcg cgtgcccagg 22980
   ctttgagttg cactcgcacc gtagtggcat caaaaggtga ccgtgcccgg tctgggcgtt 23040
   aggatacagc gcctgcataa aagccttgat ctgcttaaaa gccacctgag cctttgcgcc 23100
   ttcagagaag aacatgccgc aagacttgcc ggaaaactga ttggccggac aggccgcgtc 23160
   gtgcacgcag caccttgcgt cggtgttgga gatctgcacc acatttcggc cccaccggtt 23220
   cttcacgatc ttggccttgc tagactgctc cttcagcgcg cgctgcccgt tttcgctcgt 23280
   cacatccatt tcaatcacgt gctccttatt tatcataatg cttccgtgta gacacttaag 23340
   ctcgccttcg atctcagcgc agcggtgcag ccacaacgcg cagcccgtgg gctcgtgatg 23400
   cttgtaggtc acctctgcaa acgactgcag gtacgcctgc aggaatcgcc ccatcatcgt 23460
   cacaaaggtc ttgttgctgg tgaaggtcag ctgcaacccg cggtgctcct cgttcagcca 23520
   ggtcttgcat acggccgcca gagcttccac ttggtcaggc agtagtttga agttcgcctt 23580
   tagatcgtta tccacgtggt acttgtccat cagcgcgcgc gcagcctcca tgcccttctc 23640
   ccacgcagac acgatcggca cactcagcgg gttcatcacc gtaatttcac tttccgcttc 23700
   gctgggctct tcctcttcct cttgcgtccg cataccacgc gccactgggt cgtcttcatt 23760
   cagccgccgc actgtgcgct tacctccttt gccatgcttg attagcaccg gtgggttgct 23820
   gaaacccacc atttgtagcg ccacatcttc tctttcttcc tcgctgtcca cgattacctc 23880
   tggtgatggc gggcgctcgg gcttgggaga agggcgcttc tttttcttct tgggcgcaat 23940
   ggccaaatcc gccgccgagg tcgatggccg cgggctgggt gtgcgcggca ccagcgcgtc 24000
   ttgtgatgag tcttcctcgt cctcggactc gatacgccgc ctcatccgct tttttggggg 24060
   cgcccgggga ggcggcggcg acggggacgg ggacgacacg tcctccatgg ttgggggacg 24120
   tcgcgccgca ccgcgtccgc gctcgggggt ggtttcgcgc tgctcctctt cccgactggc 24180
   catttccttc tcctataggc agaaaaagat catggagtca gtcgagaaga aggacagcct 24240
   aaccgccccc tctgagttcg ccaccaccgc ctccaccgat gccgccaacg cgcctaccac 24300
   cttccccgtc gaggcacccc cgcttgagga ggaggaagtg attatcgagc aggacccagg 24360
   ttttgtaagc gaagacgacg aggaccgctc agtaccaaca gaggataaaa agcaagacca 24420
   ggacaacgca gaggcaaacg aggaacaagt cgggcggggg gacgaaaggc atggcgacta 24480
   cctagatgtg ggagacgacg tgctgttgaa gcatctgcag cgccagtgcg ccattatctg 24540
   cgacgcgttg caagagcgca gcgatgtgcc cctcgccata gcggatgtca gccttgccta 24600
   cgaacgccac ctattctcac cgcgcgtacc ccccaaacgc caagaaaacg gcacatgcga 24660
   gcccaacccg cgcctcaact tctaccccgt atttgccgtg ccagaggtgc ttgccaccta 24720
   tcacatcttt ttccaaaact gcaagatacc cctatcctgc cgtgccaacc gcagccgagc 24780
   ggacaagcag ctggccttgc ggcagggcgc tgtcatacct gatatcgcct cgctcaacga 24840
   agtgccaaaa atctttgagg gtcttggacg cgacgagaag cgcgcggcaa acgctctgca 24900
   acaggaaaac agcgaaaatg aaagtcactc tggagtgttg gtggaactcg agggtgacaa 24960
   cgcgcgccta gccgtactaa aacgcagcat cgaggtcacc cactttgcct acccggcact 25020
   taacctaccc cccaaggtca tgagcacagt catgagtgag ctgatcgtgc gccgtgcgca 25080
   gcccctggag agggatgcaa atttgcaaga acaaacagag gagggcctac ccgcagttgg 25140
   cgacgagcag ctagcgcgct ggcttcaaac gcgcgagcct gccgacttgg aggagcgacg 25200
   caaactaatg atggccgcag tgctcgttac cgtggagctt gagtgcatgc agcggttctt 25260
   tgctgacccg gagatgcagc gcaagctaga ggaaacattg cactacacct ttcgacaggg 25320
   ctacgtacgc caggcctgca agatctccaa cgtggagctc tgcaacctgg tctcctacct 25380
   tggaattttg cacgaaaacc gccttgggca aaacgtgctt cattccacgc tcaagggcga 25440
   ggcgcgccgc gactacgtcc gcgactgcgt ttacttattt ctatgctaca cctggcagac 25500
   ggccatgggc gtttggcagc agtgcttgga ggagtgcaac ctcaaggagc tgcagaaact 25560
   gctaaagcaa aacttgaagg acctatggac ggccttcaac gagcgctccg tggccgcgca 25620
   cctggcggac atcattttcc ccgaacgcct gcttaaaacc ctgcaacagg gtctgccaga 25680
   cttcaccagt caaagcatgt tgcagaactt taggaacttt atcctagagc gctcaggaat 25740
   cttgcccgcc acctgctgtg cacttcctag cgactttgtg cccattaagt accgcgaatg 25800
   ccctccgccg ctttggggcc actgctacct tctgcagcta gccaactacc ttgcctacca 25860
   ctctgacata atggaagacg tgagcggtga cggtctactg gagtgtcact gtcgctgcaa 25920
   cctatgcacc ccgcaccgct ccctggtttg caattcgcag ctgcttaacg aaagtcaaat 25980
   tatcggtacc tttgagctgc agggtccctc gcctgacgaa aagtccgcgg ctccggggtt 26040
   gaaactcact ccggggctgt ggacgtcggc ttaccttcgc aaatttgtac ctgaggacta 26100
   ccacgcccac gagattaggt tctacgaaga ccaatcccgc ccgcctaatg cggagcttac 26160
   cgcctgcgtc attacccagg gccacattct tggccaattg caagccatca acaaagcccg 26220
   ccaagagttt ctgctacgaa agggacgggg ggtttacttg gacccccagt ccggcgagga 26280
   gctcaaccca atccccccgc cgccgcagcc ctatcagcag cagccgcggg cccttgcttc 26340
   ccaggatggc acccaaaaag aagctgcagc tgccgccgcc acccacggac gaggaggaat 26400
   actgggacag tcaggcagag gaggttttgg acgaggagga ggaggacatg atggaagact 26460
   gggagagcct agacgaggaa gcttccgagg tcgaagaggt gtcagacgaa acaccgtcac 26520
   cctcggtcgc attcccctcg ccggcgcccc agaaatcggc aaccggttcc agcatggcta 26580
   caacctccgc tcctcaggcg ccgccggcac tgcccgttcg ccgacccaac cgtagatggg 26640
   acaccactgg aaccagggcc ggtaagtcca agcagccgcc gccgttagcc caagagcaac 26700
   aacagcgcca aggctaccgc tcatggcgcg ggcacaagaa cgccatagtt gcttgcttgc 26760
   aagactgtgg gggcaacatc tccttcgccc gccgctttct tctctaccat cacggcgtgg 26820
   ccttcccccg taacatcctg cattactacc gtcatctcta cagcccatac tgcaccggcg 26880
   gcagcggcag caacagcagc ggccacacag aagcaaaggc gaccggatag caagactctg 26940
   acaaagccca agaaatccac agcggcggca gcagcaggag gaggagcgct gcgtctggcg 27000
   cccaacgaac ccgtatcgac ccgcgagctt agaaacagga tttttcccac tctgtatgct 27060
   atatttcaac agagcagggg ccaagaacaa gagctgaaaa taaaaaacag gtctctgcga 27120
   tccctcaccc gcagctgcct gtatcacaaa agcgaagatc agcttcggcg cacgctggaa 27180
   gacgcggagg ctctcttcag taaatactgc gcgctgactc ttaaggacta gtttcgcgcc 27240
   ctttctcaaa tttaagcgcg aaaactacgt catctccagc ggccacaccc ggcgccagca 27300
   cctgttgtca gcgccattat gagcaaggaa attcccacgc cctacatgtg gagttaccag 27360
   ccacaaatgg gacttgcggc tggagctgcc caagactact caacccgaat aaactacatg 27420
   agcgcgggac cccacatgat atcccgggtc aacggaatac gcgcccaccg aaaccgaatt 27480
   ctcctggaac aggcggctat taccaccaca cctcgtaata accttaatcc ccgtagttgg 27540
   cccgctgccc tggtgtacca ggaaagtccc gctcccacca ctgtggtact tcccagagac 27600
   gcccaggccg aagttcagat gactaactca ggggcgcagc ttgcgggcgg ctttcgtcac 27660
   agggtgcggt cgcccgggca gggtataact cacctgacaa tcagagggcg aggtattcag 27720
   ctcaacgacg agtcggtgag ctcctcgctt ggtctccgtc cggacgggac atttcagatc 27780
   ggcggcgccg gccgctcttc attcacgcct cgtcaggcaa tcctaactct gcagacctcg 27840
   tcctctgagc cgcgctctgg aggcattgga actctgcaat ttattgagga gtttgtgcca 27900
   tcggtctact ttaacccctt ctcgggacct cccggccact atccggatca atttattcct 27960
   aactttgacg cggtaaagga ctcggcggac ggctacgact gaatgttaag tggagaggca 28020
   gagcaactgc gcctgaaaca cctggtccac tgtcgccgcc acaagtgctt tgcccgcgac 28080
   tccggtgagt tttgctactt tgaattgccc gaggatcata tcgagggccc ggcgcacggc 28140
   gtccggctta ccgcccaggg agagcttgcc cgtagcctga ttcgggagtt tacccagcgc 28200
   cccctgctag ttgagcggga caggggaccc tgtgttctca ctgtgatttg caactgtcct 28260
   aaccctggat tacatcaaga tctttgttgc catctctgtg ctgagtataa taaatacaga 28320
   aattaaaata tactggggct cctatcgcca tcctgtaaac gccaccgtct tcacccgccc 28380
   aagcaaacca aggcgaacct tacctggtac ttttaacatc tctccctctg tgatttacaa 28440
   cagtttcaac ccagacggag tgagtctacg agagaacctc tccgagctca gctactccat 28500
   cagaaaaaac accaccctcc ttacctgccg ggaacgtacg agtgcgtcac cggccgctgc 28560
   accacaccta ccgcctgacc gtaaaccaga ctttttccgg acagacctca ataactctgt 28620
   ttaccagaac aggaggtgag cttagaaaac ccttagggta ttaggccaaa ggcgcagcta 28680
   ctgtggggtt tatgaacaat tcaagcaact ctacgggcta ttctaattca ggtttctcta 28740
   gaatcggggt tggggttatt ctctgtcttg tgattctctt tattcttata ctaacgcttc 28800
   tctgcctaag gctcgccgcc tgctgtgtgc acatttgcat ttattgtcag ctttttaaac 28860
   gctggggtcg ccacccaaga tgattaggta cataatccta ggtttactca cccttgcgtc 28920
   agcccacggt accacccaaa aggtggattt taaggagcca gcctgtaatg ttacattcgc 28980
   agctgaagct aatgagtgca ccactcttat aaaatgcacc acagaacatg aaaagctgct 29040
   tattcgccac aaaaacaaaa ttggcaagta tgctgtttat gctatttggc agccaggtga 29100
   cactacagag tataatgtta cagttttcca gggtaaaagt cataaaactt ttatgtatac 29160
   ttttccattt tatgaaatgt gcgacattac catgtacatg agcaaacagt ataagttgtg 29220
   gcccccacaa aattgtgtgg aaaacactgg cactttctgc tgcactgcta tgctaattac 29280
   agtgctcgct ttggtctgta ccctactcta tattaaatac aaaagcagac gcagctttat 29340
   tgaggaaaag aaaatgcctt aatttactaa gttacaaagc taatgtcacc actaactgct 29400
   ttactcgctg cttgcaaaac aaattcaaaa agttagcatt ataattagaa taggatttaa 29460
   accccccggt catttcctgc tcaataccat tcccctgaac aattgactct atgtgggata 29520
   tgctccagcg ctacaacctt gaagtcaggc ttcctggatg tcagcatctg actttggcca 29580
   gcacctgtcc cgcggatttg ttccagtcca actacagcga cccaccctaa cagagatgac 29640
   caacacaacc aacgcggccg ccgctaccgg acttacatct accacaaata caccccaagt 29700
   ttctgccttt gtcaataact gggataactt gggcatgtgg tggttctcca tagcgcttat 29760
   gtttgtatgc cttattatta tgtggctcat ctgctgccta aagcgcaaac gcgcccgacc 29820
   acccatctat agtcccatca ttgtgctaca cccaaacaat gatggaatcc atagattgga 29880
   cggactgaaa cacatgttct tttctcttac agtatgatta aatgagacat gattcctcga 29940
   gtttttatat tactgaccct tgttgcgctt ttttgtgcgt gctccacatt ggctgcggtt 30000
   tctcacatcg aagtagactg cattccagcc ttcacagtct atttgcttta cggatttgtc 30060
   accctcacgc tcatctgcag cctcatcact gtggtcatcg cctttatcca gtgcattgac 30120
   tgggtctgtg tgcgctttgc atatctcaga caccatcccc agtacaggga caggactata 30180
   gctgagcttc ttagaattct ttaattatga aatttactgt gacttttctg ctgattattt 30240
   gcaccctatc tgcgttttgt tccccgacct ccaagcctca aagacatata tcatgcagat 30300
   tcactcgtat atggaatatt ccaagttgct acaatgaaaa aagcgatctt tccgaagcct 30360
   ggttatatgc aatcatctct gttatggtgt tctgcagtac catcttagcc ctagctatat 30420
   atccctacct tgacattggc tggaacgcaa tagatgccat gaaccaccca actttccccg 30480
   cgcccgctat gcttccactg caacaagttg ttgccggcgg ctttgtccca gccaatcagc 30540
   ctcgcccacc ttctcccacc cccactgaaa tcagctactt taatctaaca ggaggagatg 30600
   actgacaccc tagatctaga aatggacgga attattacag agcagcgcct gctagaaaga 30660
   cgcagggcag cggccgagca acagcgcatg aatcaagagc tccaagacat ggttaacttg 30720
   caccagtgca aaaggggtat cttttgtctc gtaaagcagg ccaaagtcac ctacgacagt 30780
   aataccaccg gacaccgcct tagctacaag ttgccaacca agcgtcagaa attggtggtc 30840
   atggtgggag aaaagcccat taccataact cagcactcgg tagaaaccga aggctgcatt 30900
   cactcacctt gtcaaggacc tgaggatctc tgcaccctta ttaagaccct gtgcggtctc 30960
   aaagatctta ttccctttaa ctaataaaaa aaaataataa agcatcactt acttaaaatc 31020
   agttagcaaa tttctgtcca gtttattcag cagcacctcc ttgccctcct cccagctctg 31080
   gtattgcagc ttcctcctgg ctgcaaactt tctccacaat ctaaatggaa tgtcagtttc 31140
   ctcctgttcc tgtccatccg cacccactat cttcatgttg ttgcagatga agcgcgcaag 31200
   accgtctgaa gataccttca accccgtgta tccatatgac acggaaaccg gtcctccaac 31260
   tgtgcctttt cttactcctc cctttgtatc ccccaatggg tttcaagaga gtccccctgg 31320
   ggtactctct ttgcgcctat ccgaacctct agttacctcc aatggcatgc ttgcgctcaa 31380
   aatgggcaac ggcctctctc tggacgaggc cggcaacctt acctcccaaa atgtaaccac 31440
   tgtgagccca cctctccgag gagacaagtc aaacataaac ctggaaatat ctgcacccct 31500
   cacagttacc tcagaagccc taactgtggc tgccgccgca cctctaatgg tcgcgggcaa 31560
   cacactcacc atgcaatcac aggccccgct aaccgtgcac gactccaaac ttagcattgc 31620
   cacccaagga cccctcacag tgtcagaagg aaagctagcc ctgcaaacat caggccccct 31680
   caccaccacc gatagcagta cccttactat cactgcctca ccccctctaa ctactgccac 31740
   tggtagcttg ggcattgact tgaaagagcc catttataca caaaatggaa aactaggact 31800
   aaagtacggg gctcctttgc atgtaacaga cgacctaaac actttgaccg tagcaactgg 31860
   tccaggtgtg actattaata atacttcctt gcaaactaaa gttactggag ccttgggttt 31920
   tgattcacaa ggcaatatgc aacttaatgt agcaggagga ctaaggattg attctcaaaa 31980
   cagacgcctt atacttgatg ttagttatcc gtttgatgct caaaaccaac taaatctaag 32040
   actaggacag ggccctcttt ttataaactc agcccacaac ttggatatta actacaacaa 32100
   aggcctttac ttgtttacag cttcaaacaa ttccaaaaag cttgaggtta acctaagcac 32160
   tgccaagggg ttgatgtttg acgctacagc catagccatt aatgcaggag atgggcttga 32220
   atttggttca cctaatgcac caaacacaaa tcccctcaaa acaaaaattg gccatggcct 32280
   agaatttgat tcaaacaagg ctatggttcc taaactagga actggcctta gttttgacag 32340
   cacaggtgcc attacagtag gaaacaaaaa taatgataag ctaactttgt ggaccacacc 32400
   agctccatct cctaactgta gactaaatgc agagaaagat gctaaactca ctttggtctt 32460
   aacaaaatgt ggcagtcaaa tacttgctac agtttcagtt ttggctgtta aaggcagttt 32520
   ggctccaata tctggaacag ttcaaagtgc tcatcttatt ataagatttg acgaaaatgg 32580
   agtgctacta aacaattcct tcctggaccc agaatattgg aactttagaa atggagatct 32640
   tactgaaggc acagcctata caaacgctgt tggatttatg cctaacctat cagcttatcc 32700
   aaaatctcac ggtaaaactg ccaaaagtaa cattgtcagt caagtttact taaacggaga 32760
   caaaactaaa cctgtaacac taaccattac actaaacggt acacaggaaa caggagacac 32820
   aactccaagt gcatactcta tgtcattttc atgggactgg tctggccaca actacattaa 32880
   tgaaatattt gccacatcct cttacacttt ttcatacatt gcccaagaat aaagaatcgt 32940
   ttgtgttatg tttcaacgtg tttatttttc aattgtacta agcggtgatg tttctgatca 33000
   gccaccatgg gagtgctaaa attcaagcac atctttttca gaagctttgt taaatcaagt 33060
   ggagtatccc agatagtttt caccttcctt ctgattccat gttgcttgac tctgaatttc 33120
   agagcacctc ctgttattcc aaatgtgcct ttcctctggg cctggaatgc cccaagtgaa 33180
   ttttgtcttg gaaaatttga tgagccacta gatatgagcc tcttctcttt cataggaagc 33240
   ccccgaataa acgccaccgg gcaaggtgtt acaatatttt atgttgatag acttggctac 33300
   tatccttaca tagattcaat cacaggagta actgtgaatg gaggaatccc ccagaagatt 33360
   tccttacaag accatctgga caaagctaag aaagacatta cattttatat gccagtagac 33420
   aatttgggaa tggctgttat tgactgggaa gaatggagac ccacttgggc aagaaactgg 33480
   aaacctaaag atgtttacaa gaataggtct attgaattgg ttcagcaaca aaatgtacaa 33540
   cttagtctca cagaggccac tgagaaagca aaacaagaat ttgaaaaggc agggaaggat 33600
   ttcctggtag agactataaa attgggaaaa ttacttcggc caaatcactt gtggggttat 33660
   tatctttttc cggattgtta caaccatcac tataagaaac ccggttacaa tggaagttgc 33720
   ttcaatgtag aaataaaaag aaatgatgat ctcagctggt tgtggaatga aagcactgct 33780
   ctttacccat ccatttattt gaacactcag cagtctcctg tagctgctac actctatgtg 33840
   cgcaatcgag ttcgggaagc catcagagtt tccaaaatac ctgatgcaaa aagtccactt 33900
   ccggtttttg catatacccg catagttttt actgatcaag ttttgaaatt cctttctcaa 33960
   gatgaacttg tgtatacatt tggcgaaact gttgctctgg gtgcttctgg aattgtaata 34020
   tggggaaccc tcagtataat gcgaagtatg aaatcttgct tgctcctaga caattacatg 34080
   gagactatac tgaatcctta cataatcaac gtcacactag cagccaaaat gtgtagccaa 34140
   gtgctttgcc aggagcaagg agtgtgtata aggaaaaact ggaattcaag tgactatctt 34200
   cacctcaacc cagataattt tgctattcaa cttgagaaag gtggaaagtt cacagtacgt 34260
   ggaaaaccga cacttgaaga cctggagcaa ttttctgaaa aattttattg cagctgttat 34320
   agcaccttga gttgtaagga gaaagctgat gtaaaagaca ctgatgctgt tgatgtgtgt 34380
   attgctgatg gtgtctgtat agatgctttt ctaaaacctc ccatggagac agaagaacct 34440
   caaattttct acaatgcttc accctccaca ctatcttaat aaactttatt tttcaattgc 34500
   agaaaatttc aagtcatttt tcattcagta gtatagcccc accaccacat agcttataca 34560
   gatcaccgta ccttaatcaa actcacagaa ccctagtatt caacctgcca cctccctccc 34620
   aacacacaga gtacacagtc ctttctcccc ggctggcctt aaaaagcatc atatcatggg 34680
   taacagacat attcttaggt gttatattcc acacggtttc ctgtcgagcc aaacgctcat 34740
   cagtgatatt aataaactcc ccgggcagct cacttaagtt catgtcgctg tccagctgct 34800
   gagccacagg ctgctgtcca acttgcggtt gcttaacggg cggcgaagga gaagtccacg 34860
   cctacatggg ggtagagtca taatcgtgca tcaggatagg gcggtggtgc tgcagcagcg 34920
   cgcgaataaa ctgctgccgc cgccgctccg tcctgcagga atacaacatg gcagtggtct 34980
   cctcagcgat gattcgcacc gcccgcagca taaggcgcct tgtcctccgg gcacagcagc 35040
   gcaccctgat ctcacttaaa tcagcacagt aactgcagca cagcaccaca atattgttca 35100
   aaatcccaca gtgcaaggcg ctgtatccaa agctcatggc ggggaccaca gaacccacgt 35160
   ggccatcata ccacaagcgc aggtagatta agtggcgacc cctcataaac acgctggaca 35220
   taaacattac ctcttttggc atgttgtaat tcaccacctc ccggtaccat ataaacctct 35280
   gattaaacat ggcgccatcc accaccatcc taaaccagct ggccaaaacc tgcccgccgg 35340
   ctatacactg cagggaaccg ggactggaac aatgacagtg gagagcccag gactcgtaac 35400
   catggatcat catgctcgtc atgatatcaa tgttggcaca acacaggcac acgtgcatac 35460
   acttcctcag gattacaagc tcctcccgcg ttagaaccat atcccaggga acaacccatt 35520
   cctgaatcag cgtaaatccc acactgcagg gaagacctcg cacgtaactc acgttgtgca 35580
   ttgtcaaagt gttacattcg ggcagcagcg gatgatcctc cagtatggta gcgcgggttt 35640
   ctgtctcaaa aggaggtaga cgatccctac tgtacggagt gcgccgagac aaccgagatc 35700
   gtgttggtcg tagtgtcatg ccaaatggaa cgccggacgt agtcatattt cctgaagcaa 35760
   aaccaggtgc gggcgtgaca aacagatctg cgtctccggt ctcgccgctt agatcgctct 35820
   gtgtagtagt tgtagtatat ccactctctc aaagcatcca ggcgccccct ggcttcgggt 35880
   tctatgtaaa ctccttcatg cgccgctgcc ctgataacat ccaccaccgc agaataagcc 35940
   acacccagcc aacctacaca ttcgttctgc gagtcacaca cgggaggagc gggaagagct 36000
   ggaagaacca tgtttttttt tttattccaa aagattatcc aaaacctcaa aatgaagatc 36060
   tattaagtga acgcgctccc ctccggtggc gtggtcaaac tctacagcca aagaacagat 36120
   aatggcattt gtaagatgtt gcacaatggc ttccaaaagg caaacggccc tcacgtccaa 36180
   gtggacgtaa aggctaaacc cttcagggtg aatctcctct ataaacattc cagcaccttc 36240
   aaccatgccc aaataattct catctcgcca ccttctcaat atatctctaa gcaaatcccg 36300
   aatattaagt ccggccattg taaaaatctg ctccagagcg ccctccacct tcagcctcaa 36360
   gcagcgaatc atgattgcaa aaattcaggt tcctcacaga cctgtataag attcaaaagc 36420
   ggaacattaa caaaaatacc gcgatcccgt aggtcccttc gcagggccag ctgaacataa 36480
   tcgtgcaggt ctgcacggac cagcgcggcc acttccccgc caggaaccat gacaaaagaa 36540
   cccacactga ttatgacacg catactcgga gctatgctaa ccagcgtagc cccgatgtaa 36600
   gcttgttgca tgggcggcga tataaaatgc aaggtgctgc tcaaaaaatc aggcaaagcc 36660
   tcgcgcaaaa aagaaagcac atcgtagtca tgctcatgca gataaaggca ggtaagctcc 36720
   ggaaccacca cagaaaaaga caccattttt ctctcaaaca tgtctgcggg tttctgcata 36780
   aacacaaaat aaaataacaa aaaaacattt aaacattaga agcctgtctt acaacaggaa 36840
   aaacaaccct tataagcata agacggacta cggccatgcc ggcgtgaccg taaaaaaact 36900
   ggtcaccgtg attaaaaagc accaccgaca gctcctcggt catgtccgga gtcataatgt 36960
   aagactcggt aaacacatca ggttgattca catcggtcag tgctaaaaag cgaccgaaat 37020
   agcccggggg aatacatacc cgcaggcgta gagacaacat tacagccccc ataggaggta 37080
   taacaaaatt aataggagag aaaaacacat aaacacctga aaaaccctcc tgcctaggca 37140
   aaatagcacc ctcccgctcc agaacaacat acagcgcttc cacagcggca gccataacag 37200
   tcagccttac cagtaaaaaa gaaaacctat taaaaaaaca ccactcgaca cggcaccagc 37260
   tcaatcagtc acagtgtaaa aaagggccaa gtgcagagcg agtatatata ggactaaaaa 37320
   atgacgtaac ggttaaagtc cacaaaaaac acccagaaaa ccgcacgcga acctacgccc 37380
   agaaacgaaa gccaaaaaac ccacaacttc ctcaaatcgt cacttccgtt ttcccacgtt 37440
   acgtcacttc ccattttaag aaaactacaa ttcccaacac atacaagtta ctccgcccta 37500
   aaacctacgt cacccgcccc gttcccacgc cccgcgccac gtcacaaact ccaccccctc 37560
   attatcatat tggcttcaat ccaaaataag gtatattatt gatgatg 37607
<210> 4
   <211> 36141
   <212> DNA
   <213> Artificial Sequence
<220>
   <223> Complete sequence of ICOVIR15
<400> 4
   catcatcaat aatatacctt attttgcatc atcaataata taccttattt tggattgaag 60
   ccaatatgat aatgaggggg tggagtttgt gacgtggcgc ggggcgtggg aacggggcgg 120
   gtgacgtagt agtgtggcgg aagtgtgatg ttgcaagtgt ggcggaacac atgtaagcga 180
   cggatgtggc aaaagtgacg tttttggtgt gcgccggtgt acacaggaag tgacaatttt 240
   cgcgcggttt taggcggatg ttgtagtaaa tttgggcgta accgagtaag atttggccat 300
   tttcgcggga aaactgaata agaggaagtg aaatctgaat aattttgtgt tactcatagc 360
   gcgtaatatt tgtctagggc cgcggggact ttgaccgttt acgtggagac tcgcccaggt 420
   gtttttctca ggtgttttcc gcgtactcgg cggctcgtgg ctctttcgcg gcaaaaagga 480
   tttggcgcgt aaaagtggtt cgaagtactc ggcggctcgt ggctctttcg cggcaaaaag 540
   gatttggcgc gtaaaagtgg ttcgaagtac gtcgaccaca aaccccgccc agcgtcttgt 600
   cattggcgtc gacgctgtac ggggtcaaag ttggcgtttt attattatag tcagctgacg 660
   tgtagtgtat ttatacccgg tgagttcctc aagaggccac tcttgagtgc cagcgagtag 720
   agttttctcc tccgagccgc tccgacaccg ggactgaaaa tgagacatat tatctgccac 780
   ggaggtgtta ttaccgaaga aatggccgcc agtcttttgg accagctgat cgaagaggta 840
   ctggctgata atcttccacc tcctagccat tttgaaccac ctacccttca cgaactgtat 900
   gatttagacg tgacggcccc cgaagatccc aacgaggagg cggtttcgca gatttttccc 960
   gactctgtaa tgttggcggt gcaggaaggg attgacttac tcacttttcc gccggcgccc 1020
   ggttctccgg agccgcctca cctttcccgg cagcccgagc agccggagca gagagccttg 1080
   ggtccggttt ctatgccaaa ccttgtaccg gaggtgatcg atccacccag tgacgacgag 1140
   gatgaagagg gtgaggagtt tgtgttagat tatgtggagc accccgggca cggttgcagg 1200
   tcttgtcatt atcaccggag gaatacgggg gacccagata ttatgtgttc gctttgctat 1260
   atgaggacct gtggcatgtt tgtctacagt aagtgaaaat tatgggcagt gggtgataga 1320
   gtggtgggtt tggtgtggta attttttttt taatttttac agttttgtgg tttaaagaat 1380
   tttgtattgt gattttttta aaaggtcctg tgtctgaacc tgagcctgag cccgagccag 1440
   aaccggagcc tgcaagacct acccgccgtc ctaaaatggc gcctgctatc ctgagacgcc 1500
   cgacatcacc tgtgtccaga gaatgcaata gtagtacgga tagctgtgac tccggtcctt 1560
   ctaacacacc tcctgagata cacccggtgg tcccgctgtg ccccattaaa ccagttgccg 1620
   tgagagttgg tgggcgtcgc caggctgtgg aatgtatcga ggacttgctt aacgagcctg 1680
   ggcaaccttt ggacttgagc tgtaaacgcc ccaggccata aggtgtaaac ctgtgattgc 1740
   gtgtgtggtt aacgcctttg tttgctgaat gagttgatgt aagtttaata aagggtgaga 1800
   taatgtttaa cttgcatggc gtgttaaatg gggcggggct taaagggtat ataatgcgcc 1860
   gtgggctaat cttggttaca tctgacctca tggaggcttg ggagtgtttg gaagattttt 1920
   ctgctgtgcg taacttgctg gaacagagct ctaacagtac ctcttggttt tggaggtttc 1980
   tgtggggctc atcccaggca aagttagtct gcagaattaa ggaggattac aagtgggaat 2040
   ttgaagagct tttgaaatcc tgtggtgagc tgtttgattc tttgaatctg ggtcaccagg 2100
   cgcttttcca agagaaggtc atcaagactt tggatttttc cacaccgggg cgcgctgcgg 2160
   ctgctgttgc ttttttgagt tttataaagg ataaatggag cgaagaaacc catctgagcg 2220
   gggggtacct gctggatttt ctggccatgc atctgtggag agcggttgtg agacacaaga 2280
   atcgcctgct actgttgtct tccgtccgcc cggcgataat accgacggag gagcagcagc 2340
   agcagcagga ggaagccagg cggcggcggc aggagcagag cccatggaac ccgagagccg 2400
   gcctggaccc tcgggaatga atgttgtaca ggtggctgaa ctgtatccag aactgagacg 2460
   cattttgaca attacagagg atgggcaggg gctaaagggg gtaaagaggg agcggggggc 2520
   ttgtgaggct acagaggagg ctaggaatct agcttttagc ttaatgacca gacaccgtcc 2580
   tgagtgtatt acttttcaac agatcaagga taattgcgct aatgagcttg atctgctggc 2640
   gcagaagtat tccatagagc agctgaccac ttactggctg cagccagggg atgattttga 2700
   ggaggctatt agggtatatg caaaggtggc acttaggcca gattgcaagt acaagatcag 2760
   caaacttgta aatatcagga attgttgcta catttctggg aacggggccg aggtggagat 2820
   agatacggag gatagggtgg cctttagatg tagcatgata aatatgtggc cgggggtgct 2880
   tggcatggac ggggtggtta ttatgaatgt aaggtttact ggccccaatt ttagcggtac 2940
   ggttttcctg gccaatacca accttatcct acacggtgta agcttctatg ggtttaacaa 3000
   tacctgtgtg gaagcctgga ccgatgtaag ggttcggggc tgtgcctttt actgctgctg 3060
   gaagggggtg gtgtgtcgcc ccaaaagcag ggcttcaatt aagaaatgcc tctttgaaag 3120
   gtgtaccttg ggtatcctgt ctgagggtaa ctccagggtg cgccacaatg tggcctccga 3180
   ctgtggttgc ttcatgctag tgaaaagcgt ggctgtgatt aagcataaca tggtatgtgg 3240
   caactgcgag gacagggcct ctcagatgct gacctgctcg gacggcaact gtcacctgct 3300
   gaagaccatt cacgtagcca gccactctcg caaggcctgg ccagtgtttg agcataacat 3360
   actgacccgc tgttccttgc atttgggtaa caggaggggg gtgttcctac cttaccaatg 3420
   caatttgagt cacactaaga tattgcttga gcccgagagc atgtccaagg tgaacctgaa 3480
   cggggtgttt gacatgacca tgaagatctg gaaggtgctg aggtacgatg agacccgcac 3540
   caggtgcaga ccctgcgagt gtggcggtaa acatattagg aaccagcctg tgatgctgga 3600
   tgtgaccgag gagctgaggc ccgatcactt ggtgctggcc tgcacccgcg ctgagtttgg 3660
   ctctagcgat gaagatacag attgaggtac tgaaatgtgt gggcgtggct taagggtggg 3720
   aaagaatata taaggtgggg gtcttatgta gttttgtatc tgttttgcag cagccgccgc 3780
   cgccatgagc accaactcgt ttgatggaag cattgtgagc tcatatttga caacgcgcat 3840
   gcccccatgg gccggggtgc gtcagaatgt gatgggctcc agcattgatg gtcgccccgt 3900
   cctgcccgca aactctacta ccttgaccta cgagaccgtg tctggaacgc cgttggagac 3960
   tgcagcctcc gccgccgctt cagccgctgc agccaccgcc cgcgggattg tgactgactt 4020
   tgctttcctg agcccgcttg caagcagtgc agcttcccgt tcatccgccc gcgatgacaa 4080
   gttgacggct cttttggcac aattggattc tttgacccgg gaacttaatg tcgtttctca 4140
   gcagctgttg gatctgcgcc agcaggtttc tgccctgaag gcttcctccc ctcccaatgc 4200
   ggtttaaaac ataaataaaa aaccagactc tgtttggatt tggatcaagc aagtgtcttg 4260
   ctgtctttat ttaggggttt tgcgcgcgcg gtaggcccgg gaccagcggt ctcggtcgtt 4320
   gagggtcctg tgtatttttt ccaggacgtg gtaaaggtga ctctggatgt tcagatacat 4380
   gggcataagc ccgtctctgg ggtggaggta gcaccactgc agagcttcat gctgcggggt 4440
   ggtgttgtag atgatccagt cgtagcagga gcgctgggcg tggtgcctaa aaatgtcttt 4500
   cagtagcaag ctgattgcca ggggcaggcc cttggtgtaa gtgtttacaa agcggttaag 4560
   ctgggatggg tgcatacgtg gggatatgag atgcatcttg gactgtattt ttaggttggc 4620
   tatgttccca gccatatccc tccggggatt catgttgtgc agaaccacca gcacagtgta 4680
   tccggtgcac ttgggaaatt tgtcatgtag cttagaagga aatgcgtgga agaacttgga 4740
   gacgcccttg tgacctccaa gattttccat gcattcgtcc ataatgatgg caatgggccc 4800
   acgggcggcg gcctgggcga agatatttct gggatcacta acgtcatagt tgtgttccag 4860
   gatgagatcg tcataggcca tttttacaaa gcgcgggcgg agggtgccag actgcggtat 4920
   aatggttcca tccggcccag gggcgtagtt accctcacag atttgcattt cccacgcttt 4980
   gagttcagat ggggggatca tgtctacctg cggggcgatg aagaaaacgg tttccggggt 5040
   aggggagatc agctgggaag aaagcaggtt cctgagcagc tgcgacttac cgcagccggt 5100
   gggcccgtaa atcacaccta ttaccgggtg caactggtag ttaagagagc tgcagctgcc 5160
   gtcatccctg agcagggggg ccacttcgtt aagcatgtcc ctgactcgca tgttttccct 5220
   gaccaaatcc gccagaaggc gctcgccgcc cagcgatagc agttcttgca aggaagcaaa 5280
   gtttttcaac ggtttgagac cgtccgccgt aggcatgctt ttgagcgttt gaccaagcag 5340
   ttccaggcgg tcccacagct cggtcacctg ctctacggca tctcgatcca gcatatctcc 5400
   tcgtttcgcg ggttggggcg gctttcgctg tacggcagta gtcggtgctc gtccagacgg 5460
   gccagggtca tgtctttcca cgggcgcagg gtcctcgtca gcgtagtctg ggtcacggtg 5520
   aaggggtgcg ctccgggctg cgcgctggcc agggtgcgct tgaggctggt cctgctggtg 5580
   ctgaagcgct gccggtcttc gccctgcgcg tcggccaggt agcatttgac catggtgtca 5640
   tagtccagcc cctccgcggc gtggcccttg gcgcgcagct tgcccttgga ggaggcgccg 5700
   cacgaggggc agtgcagact tttgagggcg tagagcttgg gcgcgagaaa taccgattcc 5760
   ggggagtagg catccgcgcc gcaggccccg cagacggtct cgcattccac gagccaggtg 5820
   agctctggcc gttcggggtc aaaaaccagg tttcccccat gctttttgat gcgtttctta 5880
   cctctggttt ccatgagccg gtgtccacgc tcggtgacga aaaggctgtc cgtgtccccg 5940
   tatacagact tgagaggcct gtcctcgagc ggtgttccgc ggtcctcctc gtatagaaac 6000
   tcggaccact ctgagacaaa ggctcgcgtc caggccagca cgaaggaggc taagtgggag 6060
   gggtagcggt cgttgtccac tagggggtcc actcgctcca gggtgtgaag acacatgtcg 6120
   ccctcttcgg catcaaggaa ggtgattggt ttgtaggtgt aggccacgtg accgggtgtt 6180
   cctgaagggg ggctataaaa gggggtgggg gcgcgttcgt cctcactctc ttccgcatcg 6240
   ctgtctgcga gggccagctg ttggggtgag tactccctct gaaaagcggg catgacttct 6300
   gcgctaagat tgtcagtttc caaaaacgag gaggatttga tattcacctg gcccgcggtg 6360
   atgcctttga gggtggccgc atccatctgg tcagaaaaga caatcttttt gttgtcaagc 6420
   ttggtggcaa acgacccgta gagggcgttg gacagcaact tggcgatgga gcgcagggtt 6480
   tggtttttgt cgcgatcggc gcgctccttg gccgcgatgt ttagctgcac gtattcgcgc 6540
   gcaacgcacc gccattcggg aaagacggtg gtgcgctcgt cgggcaccag gtgcacgcgc 6600
   caaccgcggt tgtgcagggt gacaaggtca acgctggtgg ctacctctcc gcgtaggcgc 6660
   tcgttggtcc agcagaggcg gccgcccttg cgcgagcaga atggcggtag ggggtctagc 6720
   tgcgtctcgt ccggggggtc tgcgtccacg gtaaagaccc cgggcagcag gcgcgcgtcg 6780
   aagtagtcta tcttgcatcc ttgcaagtct agcgcctgct gccatgcgcg ggcggcaagc 6840
   gcgcgctcgt atgggttgag tgggggaccc catggcatgg ggtgggtgag cgcggaggcg 6900
   tacatgccgc aaatgtcgta aacgtagagg ggctctctga gtattccaag atatgtaggg 6960
   tagcatcttc caccgcggat gctggcgcgc acgtaatcgt atagttcgtg cgagggagcg 7020
   aggaggtcgg gaccgaggtt gctacgggcg ggctgctctg ctcggaagac tatctgcctg 7080
   aagatggcat gtgagttgga tgatatggtt ggacgctgga agacgttgaa gctggcgtct 7140
   gtgagaccta ccgcgtcacg cacgaaggag gcgtaggagt cgcgcagctt gttgaccagc 7200
   tcggcggtga cctgcacgtc tagggcgcag tagtccaggg tttccttgat gatgtcatac 7260
   ttatcctgtc cctttttttt ccacagctcg cggttgagga caaactcttc gcggtctttc 7320
   cagtactctt ggatcggaaa cccgtcggcc tccgaacggt aagagcctag catgtagaac 7380
   tggttgacgg cctggtaggc gcagcatccc ttttctacgg gtagcgcgta tgcctgcgcg 7440
   gccttccgga gcgaggtgtg ggtgagcgca aaggtgtccc tgaccatgac tttgaggtac 7500
   tggtatttga agtcagtgtc gtcgcatccg ccctgctccc agagcaaaaa gtccgtgcgc 7560
   tttttggaac gcggatttgg cagggcgaag gtgacatcgt tgaagagtat ctttcccgcg 7620
   cgaggcataa agttgcgtgt gatgcggaag ggtcccggca cctcggaacg gttgttaatt 7680
   acctgggcgg cgagcacgat ctcgtcaaag ccgttgatgt tgtggcccac aatgtaaagt 7740
   tccaagaagc gcgggatgcc cttgatggaa ggcaattttt taagttcctc gtaggtgagc 7800
   tcttcagggg agctgagccc gtgctctgaa agggcccagt ctgcaagatg agggttggaa 7860
   gcgacgaatg agctccacag gtcacgggcc attagcattt gcaggtggtc gcgaaaggtc 7920
   ctaaactggc gacctatggc cattttttct ggggtgatgc agtagaaggt aagcgggtct 7980
   tgttcccagc ggtcccatcc aaggttcgcg gctaggtctc gcgcggcagt cactagaggc 8040
   tcatctccgc cgaacttcat gaccagcatg aagggcacga gctgcttccc aaaggccccc 8100
   atccaagtat aggtctctac atcgtaggtg acaaagagac gctcggtgcg aggatgcgag 8160
   ccgatcggga agaactggat ctcccgccac caattggagg agtggctatt gatgtggtga 8220
   aagtagaagt ccctgcgacg ggccgaacac tcgtgctggc ttttgtaaaa acgtgcgcag 8280
   tactggcagc ggtgcacggg ctgtacatcc tgcacgaggt tgacctgacg accgcgcaca 8340
   aggaagcaga gtgggaattt gagcccctcg cctggcgggt ttggctggtg gtcttctact 8400
   tcggctgctt gtccttgacc gtctggctgc tcgaggggag ttacggtgga tcggaccacc 8460
   acgccgcgcg agcccaaagt ccagatgtcc gcgcgcggcg gtcggagctt gatgacaaca 8520
   tcgcgcagat gggagctgtc catggtctgg agctcccgcg gcgtcaggtc aggcgggagc 8580
   tcctgcaggt ttacctcgca tagacgggtc agggcgcggg ctagatccag gtgataccta 8640
   atttccaggg gctggttggt ggcggcgtcg atggcttgca agaggccgca tccccgcggc 8700
   gcgactacgg taccgcgcgg cgggcggtgg gccgcggggg tgtccttgga tgatgcatct 8760
   aaaagcggtg acgcgggcga gcccccggag gtaggggggg ctccggaccc gccgggagag 8820
   ggggcagggg cacgtcggcg ccgcgcgcgg gcaggagctg gtgctgcgcg cgtaggttgc 8880
   tggcgaacgc gacgacgcgg cggttgatct cctgaatctg gcgcctctgc gtgaagacga 8940
   cgggcccggt gagcttgagc ctgaaagaga gttcgacaga atcaatttcg gtgtcgttga 9000
   cggcggcctg gcgcaaaatc tcctgcacgt ctcctgagtt gtcttgatag gcgatctcgg 9060
   ccatgaactg ctcgatctct tcctcctgga gatctccgcg tccggctcgc tccacggtgg 9120
   cggcgaggtc gttggaaatg cgggccatga gctgcgagaa ggcgttgagg cctccctcgt 9180
   tccagacgcg gctgtagacc acgccccctt cggcatcgcg ggcgcgcatg accacctgcg 9240
   cgagattgag ctccacgtgc cgggcgaaga cggcgtagtt tcgcaggcgc tgaaagaggt 9300
   agttgagggt ggtggcggtg tgttctgcca cgaagaagta cataacccag cgtcgcaacg 9360
   tggattcgtt gatatccccc aaggcctcaa ggcgctccat ggcctcgtag aagtccacgg 9420
   cgaagttgaa aaactgggag ttgcgcgccg acacggttaa ctcctcctcc agaagacgga 9480
   tgagctcggc gacagtgtcg cgcacctcgc gctcaaaggc tacaggggcc tcttcttctt 9540
   cttcaatctc ctcttccata agggcctccc cttcttcttc ttctggcggc ggtgggggag 9600
   gggggacacg gcggcgacga cggcgcaccg ggaggcggtc gacaaagcgc tcgatcatct 9660
   ccccgcggcg acggcgcatg gtctcggtga cggcgcggcc gttctcgcgg gggcgcagtt 9720
   ggaagacgcc gcccgtcatg tcccggttat gggttggcgg ggggctgcca tgcggcaggg 9780
   atacggcgct aacgatgcat ctcaacaatt gttgtgtagg tactccgccg ccgagggacc 9840
   tgagcgagtc cgcatcgacc ggatcggaaa acctctcgag aaaggcgtct aaccagtcac 9900
   agtcgcaagg taggctgagc accgtggcgg gcggcagcgg gcggcggtcg gggttgtttc 9960
   tggcggaggt gctgctgatg atgtaattaa agtaggcggt cttgagacgg cggatggtcg 10020
   acagaagcac catgtccttg ggtccggcct gctgaatgcg caggcggtcg gccatgcccc 10080
   aggcttcgtt ttgacatcgg cgcaggtctt tgtagtagtc ttgcatgagc ctttctaccg 10140
   gcacttcttc ttctccttcc tcttgtcctg catctcttgc atctatcgct gcggcggcgg 10200
   cggagtttgg ccgtaggtgg cgccctcttc ctcccatgcg tgtgaccccg aagcccctca 10260
   tcggctgaag cagggctagg tcggcgacaa cgcgctcggc taatatggcc tgctgcacct 10320
   gcgtgagggt agactggaag tcatccatgt ccacaaagcg gtggtatgcg cccgtgttga 10380
   tggtgtaagt gcagttggcc ataacggacc agttaacggt ctggtgaccc ggctgcgaga 10440
   gctcggtgta cctgagacgc gagtaagccc tcgagtcaaa tacgtagtcg ttgcaagtcc 10500
   gcaccaggta ctggtatccc accaaaaagt gcggcggcgg ctggcggtag aggggccagc 10560
   gtagggtggc cggggctccg ggggcgagat cttccaacat aaggcgatga tatccgtaga 10620
   tgtacctgga catccaggtg atgccggcgg cggtggtgga ggcgcgcgga aagtcgcgga 10680
   cgcggttcca gatgttgcgc agcggcaaaa agtgctccat ggtcgggacg ctctggccgg 10740
   tcaggcgcgc gcaatcgttg acgctctaga ccgtgcaaaa ggagagcctg taagcgggca 10800
   ctcttccgtg gtctggtgga taaattcgca agggtatcat ggcggacgac cggggttcga 10860
   gccccgtatc cggccgtccg ccgtgatcca tgcggttacc gcccgcgtgt cgaacccagg 10920
   tgtgcgacgt cagacaacgg gggagtgctc cttttggctt ccttccaggc gcggcggctg 10980
   ctgcgctagc ttttttggcc actggccgcg cgcagcgtaa gcggttaggc tggaaagcga 11040
   aagcattaag tggctcgctc cctgtagccg gagggttatt ttccaagggt tgagtcgcgg 11100
   gacccccggt tcgagtctcg gaccggccgg actgcggcga acgggggttt gcctccccgt 11160
   catgcaagac cccgcttgca aattcctccg gaaacaggga cgagcccctt ttttgctttt 11220
   cccagatgca tccggtgctg cggcagatgc gcccccctcc tcagcagcgg caagagcaag 11280
   agcagcggca gacatgcagg gcaccctccc ctcctcctac cgcgtcagga ggggcgacat 11340
   ccgcggttga cgcggcagca gatggtgatt acgaaccccc gcggcgccgg gcccggcact 11400
   acctggactt ggaggagggc gagggcctgg cgcggctagg agcgccctct cctgagcggt 11460
   acccaagggt gcagctgaag cgtgatacgc gtgaggcgta cgtgccgcgg cagaacctgt 11520
   ttcgcgaccg cgagggagag gagcccgagg agatgcggga tcgaaagttc cacgcagggc 11580
   gcgagctgcg gcatggcctg aatcgcgagc ggttgctgcg cgaggaggac tttgagcccg 11640
   acgcgcgaac cgggattagt cccgcgcgcg cacacgtggc ggccgccgac ctggtaaccg 11700
   catacgagca gacggtgaac caggagatta actttcaaaa aagctttaac aaccacgtgc 11760
   gtacgcttgt ggcgcgcgag gaggtggcta taggactgat gcatctgtgg gactttgtaa 11820
   gcgcgctgga gcaaaaccca aatagcaagc cgctcatggc gcagctgttc cttatagtgc 11880
   agcacagcag ggacaacgag gcattcaggg atgcgctgct aaacatagta gagcccgagg 11940
   gccgctggct gctcgatttg ataaacatcc tgcagagcat agtggtgcag gagcgcagct 12000
   tgagcctggc tgacaaggtg gccgccatca actattccat gcttagcctg ggcaagtttt 12060
   acgcccgcaa gatataccat accccttacg ttcccataga caaggaggta aagatcgagg 12120
   ggttctacat gcgcatggcg ctgaaggtgc ttaccttgag cgacgacctg ggcgtttatc 12180
   gcaacgagcg catccacaag gccgtgagcg tgagccggcg gcgcgagctc agcgaccgcg 12240
   agctgatgca cagcctgcaa agggccctgg ctggcacggg cagcggcgat agagaggccg 12300
   agtcctactt tgacgcgggc gctgacctgc gctgggcccc aagccgacgc gccctggagg 12360
   cagctggggc cggacctggg ctggcggtgg cacccgcgcg cgctggcaac gtcggcggcg 12420
   tggaggaata tgacgaggac gatgagtacg agccagagga cggcgagtac taagcggtga 12480
   tgtttctgat cagatgatgc aagacgcaac ggacccggcg gtgcgggcgg cgctgcagag 12540
   ccagccgtcc ggccttaact ccacggacga ctggcgccag gtcatggacc gcatcatgtc 12600
   gctgactgcg cgcaatcctg acgcgttccg gcagcagccg caggccaacc ggctctccgc 12660
   aattctggaa gcggtggtcc cggcgcgcgc aaaccccacg cacgagaagg tgctggcgat 12720
   cgtaaacgcg ctggccgaaa acagggccat ccggcccgac gaggccggcc tggtctacga 12780
   cgcgctgctt cagcgcgtgg ctcgttacaa cagcggcaac gtgcagacca acctggaccg 12840
   gctggtgggg gatgtgcgcg aggccgtggc gcagcgtgag cgcgcgcagc agcagggcaa 12900
   cctgggctcc atggttgcac taaacgcctt cctgagtaca cagcccgcca acgtgccgcg 12960
   gggacaggag gactacacca actttgtgag cgcactgcgg ctaatggtga ctgagacacc 13020
   gcaaagtgag gtgtaccagt ctgggccaga ctattttttc cagaccagta gacaaggcct 13080
   gcagaccgta aacctgagcc aggctttcaa aaacttgcag gggctgtggg gggtgcgggc 13140
   tcccacaggc gaccgcgcga ccgtgtctag cttgctgacg cccaactcgc gcctgttgct 13200
   gctgctaata gcgcccttca cggacagtgg cagcgtgtcc cgggacacat acctaggtca 13260
   cttgctgaca ctgtaccgcg aggccatagg tcaggcgcat gtggacgagc atactttcca 13320
   ggagattaca agtgtcagcc gcgcgctggg gcaggaggac acgggcagcc tggaggcaac 13380
   cctaaactac ctgctgacca accggcggca gaagatcccc tcgttgcaca gtttaaacag 13440
   cgaggaggag cgcattttgc gctacgtgca gcagagcgtg agccttaacc tgatgcgcga 13500
   cggggtaacg cccagcgtgg cgctggacat gaccgcgcgc aacatggaac cgggcatgta 13560
   tgcctcaaac cggccgttta tcaaccgcct aatggactac ttgcatcgcg cggccgccgt 13620
   gaaccccgag tatttcacca atgccatctt gaacccgcac tggctaccgc cccctggttt 13680
   ctacaccggg ggattcgagg tgcccgaggg taacgatgga ttcctctggg acgacataga 13740
   cgacagcgtg ttttccccgc aaccgcagac cctgctagag ttgcaacagc gcgagcaggc 13800
   agaggcggcg ctgcgaaagg aaagcttccg caggccaagc agcttgtccg atctaggcgc 13860
   tgcggccccg cggtcagatg ctagtagccc atttccaagc ttgatagggt ctcttaccag 13920
   cactcgcacc acccgcccgc gcctgctggg cgaggaggag tacctaaaca actcgctgct 13980
   gcagccgcag cgcgaaaaaa acctgcctcc ggcatttccc aacaacggga tagagagcct 14040
   agtggacaag atgagtagat ggaagacgta cgcgcaggag cacagggacg tgccaggccc 14100
   gcgcccgccc acccgtcgtc aaaggcacga ccgtcagcgg ggtctggtgt gggaggacga 14160
   tgactcggca gacgacagca gcgtcctgga tttgggaggg agtggcaacc cgtttgcgca 14220
   ccttcgcccc aggctgggga gaatgtttta aaaaaaaaaa agcatgatgc aaaataaaaa 14280
   actcaccaag gccatggcac cgagcgttgg ttttcttgta ttccccttag tatgcggcgc 14340
   gcggcgatgt atgaggaagg tcctcctccc tcctacgaga gtgtggtgag cgcggcgcca 14400
   gtggcggcgg cgctgggttc tcccttcgat gctcccctgg acccgccgtt tgtgcctccg 14460
   cggtacctgc ggcctaccgg ggggagaaac agcatccgtt actctgagtt ggcaccccta 14520
   ttcgacacca cccgtgtgta cctggtggac aacaagtcaa cggatgtggc atccctgaac 14580
   taccagaacg accacagcaa ctttctgacc acggtcattc aaaacaatga ctacagcccg 14640
   ggggaggcaa gcacacagac catcaatctt gacgaccggt cgcactgggg cggcgacctg 14700
   aaaaccatcc tgcataccaa catgccaaat gtgaacgagt tcatgtttac caataagttt 14760
   aaggcgcggg tgatggtgtc gcgcttgcct actaaggaca atcaggtgga gctgaaatac 14820
   gagtgggtgg agttcacgct gcccgagggc aactactccg agaccatgac catagacctt 14880
   atgaacaacg cgatcgtgga gcactacttg aaagtgggca gacagaacgg ggttctggaa 14940
   agcgacatcg gggtaaagtt tgacacccgc aacttcagac tggggtttga ccccgtcact 15000
   ggtcttgtca tgcctggggt atatacaaac gaagccttcc atccagacat cattttgctg 15060
   ccaggatgcg gggtggactt cacccacagc cgcctgagca acttgttggg catccgcaag 15120
   cggcaaccct tccaggaggg ctttaggatc acctacgatg atctggaggg tggtaacatt 15180
   cccgcactgt tggatgtgga cgcctaccag gcgagcttga aagatgacac cgaacagggc 15240
   gggggtggcg caggcggcag caacagcagt ggcagcggcg cggaagagaa ctccaacgcg 15300
   gcagccgcgg caatgcagcc ggtggaggac atgaacgatc atgccattcg cggcgacacc 15360
   tttgccacac gggctgagga gaagcgcgct gaggccgaag cagcggccga agctgccgcc 15420
   cccgctgcgc aacccgaggt cgagaagcct cagaagaaac cggtgatcaa acccctgaca 15480
   gaggacagca agaaacgcag ttacaaccta ataagcaatg acagcacctt cacccagtac 15540
   cgcagctggt accttgcata caactacggc gaccctcaga ccggaatccg ctcatggacc 15600
   ctgctttgca ctcctgacgt aacctgcggc tcggagcagg tctactggtc gttgccagac 15660
   atgatgcaag accccgtgac cttccgctcc acgcgccaga tcagcaactt tccggtggtg 15720
   ggcgccgagc tgttgcccgt gcactccaag agcttctaca acgaccaggc cgtctactcc 15780
   caactcatcc gccagtttac ctctctgacc cacgtgttca atcgctttcc cgagaaccag 15840
   attttggcgc gcccgccagc ccccaccatc accaccgtca gtgaaaacgt tcctgctctc 15900
   acagatcacg ggacgctacc gctgcgcaac agcatcggag gagtccagcg agtgaccatt 15960
   actgacgcca gacgccgcac ctgcccctac gtttacaagg ccctgggcat agtctcgccg 16020
   cgcgtcctat cgagccgcac tttttgagca agcatgtcca tccttatatc gcccagcaat 16080
   aacacaggct ggggcctgcg cttcccaagc aagatgtttg gcggggccaa gaagcgctcc 16140
   gaccaacacc cagtgcgcgt gcgcgggcac taccgcgcgc cctggggcgc gcacaaacgc 16200
   ggccgcactg ggcgcaccac cgtcgatgac gccatcgacg cggtggtgga ggaggcgcgc 16260
   aactacacgc ccacgccgcc accagtgtcc acagtggacg cggccattca gaccgtggtg 16320
   cgcggagccc ggcgctatgc taaaatgaag agacggcgga ggcgcgtagc acgtcgccac 16380
   cgccgccgac ccggcactgc cgcccaacgc gcggcggcgg ccctgcttaa ccgcgcacgt 16440
   cgcaccggcc gacgggcggc catgcgggcc gctcgaaggc tggccgcggg tattgtcact 16500
   gtgcccccca ggtccaggcg acgagcggcc gccgcagcag ccgcggccat tagtgctatg 16560
   actcagggtc gcaggggcaa cgtgtattgg gtgcgcgact cggttagcgg cctgcgcgtg 16620
   cccgtgcgca cccgcccccc gcgcaactag attgcaagaa aaaactactt agactcgtac 16680
   tgttgtatgt atccagcggc ggcggcgcgc aacgaagcta tgtccaagcg caaaatcaaa 16740
   gaagagatgc tccaggtcat cgcgccggag atctatggcc ccccgaagaa ggaagagcag 16800
   gattacaagc cccgaaagct aaagcgggtc aaaaagaaaa agaaagatga tgatgatgaa 16860
   cttgacgacg aggtggaact gctgcacgct accgcgccca ggcgacgggt acagtggaaa 16920
   ggtcgacgcg taaaacgtgt tttgcgaccc ggcaccaccg tagtctttac gcccggtgag 16980
   cgctccaccc gcacctacaa gcgcgtgtat gatgaggtgt acggcgacga ggacctgctt 17040
   gagcaggcca acgagcgcct cggggagttt gcctacggaa agcggcataa ggacatgctg 17100
   gcgttgccgc tggacgaggg caacccaaca cctagcctaa agcccgtaac actgcagcag 17160
   gtgctgcccg cgcttgcacc gtccgaagaa aagcgcggcc taaagcgcga gtctggtgac 17220
   ttggcaccca ccgtgcagct gatggtaccc aagcgccagc gactggaaga tgtcttggaa 17280
   aaaatgaccg tggaacctgg gctggagccc gaggtccgcg tgcggccaat caagcaggtg 17340
   gcgccgggac tgggcgtgca gaccgtggac gttcagatac ccactaccag tagcaccagt 17400
   attgccaccg ccacagaggg catggagaca caaacgtccc cggttgcctc agcggtggcg 17460
   gatgccgcgg tgcaggcggt cgctgcggcc gcgtccaaga cctctacgga ggtgcaaacg 17520
   gacccgtgga tgtttcgcgt ttcagccccc cggcgcccgc gcggttcgag gaagtacggc 17580
   gccgccagcg cgctactgcc cgaatatgcc ctacatcctt ccattgcgcc tacccccggc 17640
   tatcgtggct acacctaccg ccccagaaga cgagcaacta cccgacgccg aaccaccact 17700
   ggaacccgcc gccgccgtcg ccgtcgccag cccgtgctgg ccccgatttc cgtgcgcagg 17760
   gtggctcgcg aaggaggcag gaccctggtg ctgccaacag cgcgctacca ccccagcatc 17820
   gtttaaaagc cggtctttgt ggttcttgca gatatggccc tcacctgccg cctccgtttc 17880
   ccggtgccgg gattccgagg aagaatgcac cgtaggaggg gcatggccgg ccacggcctg 17940
   acgggcggca tgcgtcgtgc gcaccaccgg cggcggcgcg cgtcgcaccg tcgcatgcgc 18000
   ggcggtatcc tgcccctcct tattccactg atcgccgcgg cgattggcgc cgtgcccgga 18060
   attgcatccg tggccttgca ggcgcagaga cactgattaa aaacaagttg catgtggaaa 18120
   aatcaaaata aaaagtctgg actctcacgc tcgcttggtc ctgtaactat tttgtagaat 18180
   ggaagacatc aactttgcgt ctctggcccc gcgacacggc tcgcgcccgt tcatgggaaa 18240
   ctggcaagat atcggcacca gcaatatgag cggtggcgcc ttcagctggg gctcgctgtg 18300
   gagcggcatt aaaaatttcg gttccaccgt taagaactat ggcagcaagg cctggaacag 18360
   cagcacaggc cagatgctga gggataagtt gaaagagcaa aatttccaac aaaaggtggt 18420
   agatggcctg gcctctggca ttagcggggt ggtggacctg gccaaccagg cagtgcaaaa 18480
   taagattaac agtaagcttg atccccgccc tcccgtagag gagcctccac cggccgtgga 18540
   gacagtgtct ccagaggggc gtggcgaaaa gcgtccgcgc cccgacaggg aagaaactct 18600
   ggtgacgcaa atagacgagc ctccctcgta cgaggaggca ctaaagcaag gcctgcccac 18660
   cacccgtccc atcgcgccca tggctaccgg agtgctgggc cagcacacac ccgtaacgct 18720
   ggacctgcct ccccccgccg acacccagca gaaacctgtg ctgccaggcc cgaccgccgt 18780
   tgttgtaacc cgtcctagcc gcgcgtccct gcgccgcgcc gccagcggtc cgcgatcgtt 18840
   gcggcccgta gccagtggca actggcaaag cacactgaac agcatcgtgg gtctgggggt 18900
   gcaatccctg aagcgccgac gatgcttctg aatagctaac gtgtcgtatg tgtgtcatgt 18960
   atgcgtccat gtcgccgcca gaggagctgc tgagccgccg cgcgcccgct ttccaagatg 19020
   gctacccctt cgatgatgcc gcagtggtct tacatgcaca tctcgggcca ggacgcctcg 19080
   gagtacctga gccccgggct ggtgcagttt gcccgcgcca ccgagacgta cttcagcctg 19140
   aataacaagt ttagaaaccc cacggtggcg cctacgcacg acgtgaccac agaccggtcc 19200
   cagcgtttga cgctgcggtt catccctgtg gaccgtgagg atactgcgta ctcgtacaag 19260
   gcgcggttca ccctagctgt gggtgataac cgtgtgctgg acatggcttc cacgtacttt 19320
   gacatccgcg gcgtgctgga caggggccct acttttaagc cctactctgg cactgcctac 19380
   aacgccctgg ctcccaaggg tgccccaaat ccttgcgaat gggatgaagc tgctactgct 19440
   cttgaaataa acctagaaga agaggacgat gacaacgaag acgaagtaga cgagcaagct 19500
   gagcagcaaa aaactcacgt atttgggcag gcgccttatt ctggtataaa tattacaaag 19560
   gagggtattc aaataggtgt cgaaggtcaa acacctaaat atgccgataa aacatttcaa 19620
   cctgaacctc aaataggaga atctcagtgg tacgaaactg aaattaatca tgcagctggg 19680
   agagtcctta aaaagactac cccaatgaaa ccatgttacg gttcatatgc aaaacccaca 19740
   aatgaaaatg gagggcaagg cattcttgta aagcaacaaa atggaaagct agaaagtcaa 19800
   gtggaaatgc aatttttctc aactactgag gcgaccgcag gcaatggtga taacttgact 19860
   cctaaagtgg tattgtacag tgaagatgta gatatagaaa ccccagacac tcatatttct 19920
   tacatgccca ctattaagga aggtaactca cgagaactaa tgggccaaca atctatgccc 19980
   aacaggccta attacattgc ttttagggac aattttattg gtctaatgta ttacaacagc 20040
   acgggtaata tgggtgttct ggcgggccaa gcatcgcagt tgaatgctgt tgtagatttg 20100
   caagacagaa acacagagct ttcataccag cttttgcttg attccattgg tgatagaacc 20160
   aggtactttt ctatgtggaa tcaggctgtt gacagctatg atccagatgt tagaattatt 20220
   gaaaatcatg gaactgaaga tgaacttcca aattactgct ttccactggg aggtgtgatt 20280
   aatacagaga ctcttaccaa ggtaaaacct aaaacaggtc aggaaaatgg atgggaaaaa 20340
   gatgctacag aattttcaga taaaaatgaa ataagagttg gaaataattt tgccatggaa 20400
   atcaatctaa atgccaacct gtggagaaat ttcctgtact ccaacatagc gctgtatttg 20460
   cccgacaagc taaagtacag tccttccaac gtaaaaattt ctgataaccc aaacacctac 20520
   gactacatga acaagcgagt ggtggctccc gggttagtgg actgctacat taaccttgga 20580
   gcacgctggt cccttgacta tatggacaac gtcaacccat ttaaccacca ccgcaatgct 20640
   ggcctgcgct accgctcaat gttgctgggc aatggtcgct atgtgccctt ccacatccag 20700
   gtgcctcaga agttctttgc cattaaaaac ctccttctcc tgccgggctc atacacctac 20760
   gagtggaact tcaggaagga tgttaacatg gttctgcaga gctccctagg aaatgaccta 20820
   agggttgacg gagccagcat taagtttgat agcatttgcc tttacgccac cttcttcccc 20880
   atggcccaca acaccgcctc cacgcttgag gccatgctta gaaacgacac caacgaccag 20940
   tcctttaacg actatctctc cgccgccaac atgctctacc ctatacccgc caacgctacc 21000
   aacgtgccca tatccatccc ctcccgcaac tgggcggctt tccgcggctg ggccttcacg 21060
   cgccttaaga ctaaggaaac cccatcactg ggctcgggct acgaccctta ttacacctac 21120
   tctggctcta taccctacct agatggaacc ttttacctca accacacctt taagaaggtg 21180
   gccattacct ttgactcttc tgtcagctgg cctggcaatg accgcctgct tacccccaac 21240
   gagtttgaaa ttaagcgctc agttgacggg gagggttaca acgttgccca gtgtaacatg 21300
   accaaagact ggttcctggt acaaatgcta gctaactaca acattggcta ccagggcttc 21360
   tatatcccag agagctacaa ggaccgcatg tactccttct ttagaaactt ccagcccatg 21420
   agccgtcagg tggtggatga tactaaatac aaggactacc aacaggtggg catcctacac 21480
   caacacaaca actctggatt tgttggctac cttgccccca ccatgcgcga aggacaggcc 21540
   taccctgcta acttccccta tccgcttata ggcaagaccg cagttgacag cattacccag 21600
   aaaaagtttc tttgcgatcg caccctttgg cgcatcccat tctccagtaa ctttatgtcc 21660
   atgggcgcac tcacagacct gggccaaaac cttctctacg ccaactccgc ccacgcgcta 21720
   gacatgactt ttgaggtgga tcccatggac gagcccaccc ttctttatgt tttgtttgaa 21780
   gtctttgacg tggtccgtgt gcaccggccg caccgcggcg tcatcgaaac cgtgtacctg 21840
   cgcacgccct tctcggccgg caacgccaca acataaagaa gcaagcaaca tcaacaacag 21900
   ctgccgccat gggctccagt gagcaggaac tgaaagccat tgtcaaagat cttggttgtg 21960
   ggccatattt tttgggcacc tatgacaagc gctttccagg ctttgtttct ccacacaagc 22020
   tcgcctgcgc catagtcaat acggccggtc gcgagactgg gggcgtacac tggatggcct 22080
   ttgcctggaa cccgcactca aaaacatgct acctctttga gccctttggc ttttctgacc 22140
   agcgactcaa gcaggtttac cagtttgagt acgagtcact cctgcgccgt agcgccattg 22200
   cttcttcccc cgaccgctgt ataacgctgg aaaagtccac ccaaagcgta caggggccca 22260
   actcggccgc ctgtggacta ttctgctgca tgtttctcca cgcctttgcc aactggcccc 22320
   aaactcccat ggatcacaac cccaccatga accttattac cggggtaccc aactccatgc 22380
   tcaacagtcc ccaggtacag cccaccctgc gtcgcaacca ggaacagctc tacagcttcc 22440
   tggagcgcca ctcgccctac ttccgcagcc acagtgcgca gattaggagc gccacttctt 22500
   tttgtcactt gaaaaacatg taaaaataat gtactagaga cactttcaat aaaggcaaat 22560
   gcttttattt gtacactctc gggtgattat ttacccccac ccttgccgtc tgcgccgttt 22620
   aaaaatcaaa ggggttctgc cgcgcatcgc tatgcgccac tggcagggac acgttgcgat 22680
   actggtgttt agtgctccac ttaaactcag gcacaaccat ccgcggcagc tcggtgaagt 22740
   tttcactcca caggctgcgc accatcacca acgcgtttag caggtcgggc gccgatatct 22800
   tgaagtcgca gttggggcct ccgccctgcg cgcgcgagtt gcgatacaca gggttgcagc 22860
   actggaacac tatcagcgcc gggtggtgca cgctggccag cacgctcttg tcggagatca 22920
   gatccgcgtc caggtcctcc gcgttgctca gggcgaacgg agtcaacttt ggtagctgcc 22980
   ttcccaaaaa gggcgcgtgc ccaggctttg agttgcactc gcaccgtagt ggcatcaaaa 23040
   ggtgaccgtg cccggtctgg gcgttaggat acagcgcctg cataaaagcc ttgatctgct 23100
   taaaagccac ctgagccttt gcgccttcag agaagaacat gccgcaagac ttgccggaaa 23160
   actgattggc cggacaggcc gcgtcgtgca cgcagcacct tgcgtcggtg ttggagatct 23220
   gcaccacatt tcggccccac cggttcttca cgatcttggc cttgctagac tgctccttca 23280
   gcgcgcgctg cccgttttcg ctcgtcacat ccatttcaat cacgtgctcc ttatttatca 23340
   taatgcttcc gtgtagacac ttaagctcgc cttcgatctc agcgcagcgg tgcagccaca 23400
   acgcgcagcc cgtgggctcg tgatgcttgt aggtcacctc tgcaaacgac tgcaggtacg 23460
   cctgcaggaa tcgccccatc atcgtcacaa aggtcttgtt gctggtgaag gtcagctgca 23520
   acccgcggtg ctcctcgttc agccaggtct tgcatacggc cgccagagct tccacttggt 23580
   caggcagtag tttgaagttc gcctttagat cgttatccac gtggtacttg tccatcagcg 23640
   cgcgcgcagc ctccatgccc ttctcccacg cagacacgat cggcacactc agcgggttca 23700
   tcaccgtaat ttcactttcc gcttcgctgg gctcttcctc ttcctcttgc gtccgcatac 23760
   cacgcgccac tgggtcgtct tcattcagcc gccgcactgt gcgcttacct cctttgccat 23820
   gcttgattag caccggtggg ttgctgaaac ccaccatttg tagcgccaca tcttctcttt 23880
   cttcctcgct gtccacgatt acctctggtg atggcgggcg ctcgggcttg ggagaagggc 23940
   gcttcttttt cttcttgggc gcaatggcca aatccgccgc cgaggtcgat ggccgcgggc 24000
   tgggtgtgcg cggcaccagc gcgtcttgtg atgagtcttc ctcgtcctcg gactcgatac 24060
   gccgcctcat ccgctttttt gggggcgccc ggggaggcgg cggcgacggg gacggggacg 24120
   acacgtcctc catggttggg ggacgtcgcg ccgcaccgcg tccgcgctcg ggggtggttt 24180
   cgcgctgctc ctcttcccga ctggccattt ccttctccta taggcagaaa aagatcatgg 24240
   agtcagtcga gaagaaggac agcctaaccg ccccctctga gttcgccacc accgcctcca 24300
   ccgatgccgc caacgcgcct accaccttcc ccgtcgaggc acccccgctt gaggaggagg 24360
   aagtgattat cgagcaggac ccaggttttg taagcgaaga cgacgaggac cgctcagtac 24420
   caacagagga taaaaagcaa gaccaggaca acgcagaggc aaacgaggaa caagtcgggc 24480
   ggggggacga aaggcatggc gactacctag atgtgggaga cgacgtgctg ttgaagcatc 24540
   tgcagcgcca gtgcgccatt atctgcgacg cgttgcaaga gcgcagcgat gtgcccctcg 24600
   ccatagcgga tgtcagcctt gcctacgaac gccacctatt ctcaccgcgc gtacccccca 24660
   aacgccaaga aaacggcaca tgcgagccca acccgcgcct caacttctac cccgtatttg 24720
   ccgtgccaga ggtgcttgcc acctatcaca tctttttcca aaactgcaag atacccctat 24780
   cctgccgtgc caaccgcagc cgagcggaca agcagctggc cttgcggcag ggcgctgtca 24840
   tacctgatat cgcctcgctc aacgaagtgc caaaaatctt tgagggtctt ggacgcgacg 24900
   agaagcgcgc ggcaaacgct ctgcaacagg aaaacagcga aaatgaaagt cactctggag 24960
   tgttggtgga actcgagggt gacaacgcgc gcctagccgt actaaaacgc agcatcgagg 25020
   tcacccactt tgcctacccg gcacttaacc taccccccaa ggtcatgagc acagtcatga 25080
   gtgagctgat cgtgcgccgt gcgcagcccc tggagaggga tgcaaatttg caagaacaaa 25140
   cagaggaggg cctacccgca gttggcgacg agcagctagc gcgctggctt caaacgcgcg 25200
   agcctgccga cttggaggag cgacgcaaac taatgatggc cgcagtgctc gttaccgtgg 25260
   agcttgagtg catgcagcgg ttctttgctg acccggagat gcagcgcaag ctagaggaaa 25320
   cattgcacta cacctttcga cagggctacg tacgccaggc ctgcaagatc tccaacgtgg 25380
   agctctgcaa cctggtctcc taccttggaa ttttgcacga aaaccgcctt gggcaaaacg 25440
   tgcttcattc cacgctcaag ggcgaggcgc gccgcgacta cgtccgcgac tgcgtttact 25500
   tatttctatg ctacacctgg cagacggcca tgggcgtttg gcagcagtgc ttggaggagt 25560
   gcaacctcaa ggagctgcag aaactgctaa agcaaaactt gaaggaccta tggacggcct 25620
   tcaacgagcg ctccgtggcc gcgcacctgg cggacatcat tttccccgaa cgcctgctta 25680
   aaaccctgca acagggtctg ccagacttca ccagtcaaag catgttgcag aactttagga 25740
   actttatcct agagcgctca ggaatcttgc ccgccacctg ctgtgcactt cctagcgact 25800
   ttgtgcccat taagtaccgc gaatgccctc cgccgctttg gggccactgc taccttctgc 25860
   agctagccaa ctaccttgcc taccactctg acataatgga agacgtgagc ggtgacggtc 25920
   tactggagtg tcactgtcgc tgcaacctat gcaccccgca ccgctccctg gtttgcaatt 25980
   cgcagctgct taacgaaagt caaattatcg gtacctttga gctgcagggt ccctcgcctg 26040
   acgaaaagtc cgcggctccg gggttgaaac tcactccggg gctgtggacg tcggcttacc 26100
   ttcgcaaatt tgtacctgag gactaccacg cccacgagat taggttctac gaagaccaat 26160
   cccgcccgcc aaatgcggag cttaccgcct gcgtcattac ccagggccac attcttggcc 26220
   aattgcaagc catcaacaaa gcccgccaag agtttctgct acgaaaggga cggggggttt 26280
   acttggaccc ccagtccggc gaggagctca acccaatccc cccgccgccg cagccctatc 26340
   agcagcagcc gcgggccctt gcttcccagg atggcaccca aaaagaagct gcagctgccg 26400
   ccgccaccca cggacgagga ggaatactgg gacagtcagg cagaggaggt tttggacgag 26460
   gaggaggagg acatgatgga agactgggag agcctagacg aggaagcttc cgaggtcgaa 26520
   gaggtgtcag acgaaacacc gtcaccctcg gtcgcattcc cctcgccggc gccccagaaa 26580
   tcggcaaccg gttccagcat ggctacaacc tccgctcctc aggcgccgcc ggcactgccc 26640
   gttcgccgac ccaaccgtag atgggacacc actggaacca gggccggtaa gtccaagcag 26700
   ccgccgccgt tagcccaaga gcaacaacag cgccaaggct accgctcatg gcgcgggcac 26760
   aagaacgcca tagttgcttg cttgcaagac tgtgggggca acatctcctt cgcccgccgc 26820
   tttcttctct accatcacgg cgtggccttc ccccgtaaca tcctgcatta ctaccgtcat 26880
   ctctacagcc catactgcac cggcggcagc ggcagcggca gcaacagcag cggccacaca 26940
   gaagcaaagg cgaccggata gcaagactct gacaaagccc aagaaatcca cagcggcggc 27000
   agcagcagga ggaggagcgc tgcgtctggc gcccaacgaa cccgtatcga cccgcgagct 27060
   tagaaacagg atttttccca ctctgtatgc tatatttcaa cagagcaggg gccaagaaca 27120
   agagctgaaa ataaaaaaca ggtctctgcg atccctcacc cgcagctgcc tgtatcacaa 27180
   aagcgaagat cagcttcggc gcacgctgga agacgcggag gctctcttca gtaaatactg 27240
   cgcgctgact cttaaggact agtttcgcgc cctttctcaa atttaagcgc gaaaactacg 27300
   tcatctccag cggccacacc cggcgccagc acctgtcgtc agcgccatta tgagcaagga 27360
   aattcccacg ccctacatgt ggagttacca gccacaaatg ggacttgcgg ctggagctgc 27420
   ccaagactac tcaacccgaa taaactacat gagcgcggga ccccacatga tatcccgggt 27480
   caacggaatc cgcgcccacc gaaaccgaat tctcttggaa caggcggcta ttaccaccac 27540
   acctcgtaat aaccttaatc cccgtagttg gcccgctgcc ctggtgtacc aggaaagtcc 27600
   cgctcccacc actgtggtac ttcccagaga cgcccaggcc gaagttcaga tgactaactc 27660
   aggggcgcag cttgcgggcg gctttcgtca cagggtgcgg tcgcccgggc agggtataac 27720
   tcacctgaca atcagagggc gaggtattca gctcaacgac gagtcggtga gctcctcgct 27780
   tggtctccgt ccggacggga catttcagat cggcggcgcc ggccgtcctt cattcacgcc 27840
   tcgtcaggca atcctaactc tgcagacctc gtcctctgag ccgcgctctg gaggcattgg 27900
   aactctgcaa tttattgagg agtttgtgcc atcggtctac tttaacccct tctcgggacc 27960
   tcccggccac tatccggatc aatttattcc taactttgac gcggtaaagg actcggcgga 28020
   cggctacgac tgaatgttaa gtggagaggc agagcaactg cgcctgaaac acctggtcca 28080
   ctgtcgccgc cacaagtgct ttgcccgcga ctccggtgag ttttgctact ttgaattgcc 28140
   cgaggatcat atcgagggcc cggcgcacgg cgtccggctt accgcccagg gagagcttgc 28200
   ccgtagcctg attcgggagt ttacccagcg ccccctgcta gttgagcggg acaggggacc 28260
   ctgtgttctc actgtgattt gcaactgtcc taaccttgga ttacatcaag atctttgttg 28320
   ccatctctgt gctgagtata ataaatacag aaattaaaat atactggggc tcctatcgcc 28380
   atcctgtaaa cgccaccgtc ttcacccgcc caagcaaacc aaggcgaacc ttacctggta 28440
   cttttaacat ctctccctct gtgatttaca acagtttcaa cccagacgga gtgagtctac 28500
   gagagaacct ctccgagctc agctactcca tcagaaaaaa caccaccctc cttacctgcc 28560
   gggaacgtac gagtgcgtca ccggccgctg caccacacct accgcctgac cgtaaaccag 28620
   actttttccg gacagacctc aataactctg tttaccagaa caggaggtga gcttagaaaa 28680
   cccttagggt attaggccaa aggcgcagct actgtggggt ttatgaacaa ttcaagcaac 28740
   tctacgggct attctaattc aggtttctct agaatcgggg ttggggttat tctctgtctt 28800
   gtgattctct ttattcttat actaacgctt ctctgcctaa ggctcgccgc ctgctgtgtg 28860
   cacatttgca tttattgtca gctttttaaa cgctggggtc gccacccaag atgattaggt 28920
   acataatcct aggtttactc acccttgcgt cagcccacgg taccacccaa aaggtggatt 28980
   ttaaggagcc agcctgtaat gttacattcg cagctgaagc taatgagtgc accactctta 29040
   taaaatgcac cacagaacat gaaaagctgc ttattcgcca caaaaacaaa attggcaagt 29100
   atgctgttta tgctatttgg cagccaggtg acactacaga gtataatgtt acagttttcc 29160
   agggtaaaag tcataaaact tttatgtata cttttccatt ttatgaaatg tgcgacatta 29220
   ccatgtacat gagcaaacag tataagttgt ggcccccaca aaattgtgtg gaaaacactg 29280
   gcactttctg ctgcactgct atgctaatta cagtgctcgc tttggtctgt accctactct 29340
   atattaaata caaaagcaga cgcagcttta ttgaggaaaa gaaaatgcct taatttacta 29400
   agttacaaag ctaatgtcac cactaactgc tttactcgct gcttgcaaaa caaattcaaa 29460
   aagttagcat tataattaga ataggattta aaccccccgg tcatttcctg ctcaatacca 29520
   ttcccctgaa caattgactc tatgtgggat atgctccagc gctacaacct tgaagtcagg 29580
   cttcctggat gtcagcatct gactttggcc agcacctgtc ccgcggattt gttccagtcc 29640
   aactacagcg acccacccta acagagatga ccaacacaac caacgcggcc gccgctaccg 29700
   gacttacatc taccacaaat acaccccaag tttctgcctt tgtcaataac tgggataact 29760
   tgggcatgtg gtggttctcc atagcgctta tgtttgtatg ccttattatt atgtggctca 29820
   tctgctgcct aaagcgcaaa cgcgcccgac cacccatcta tagtcccatc attgtgctac 29880
   acccaaacaa tgatggaatc catagattgg acggactgaa acacatgttc ttttctctta 29940
   cagtatgatt aaatgagaca tgattcctcg agtttttata ttactgaccc ttgttgcgct 30000
   tttttgtgcg tgctccacat tggctgcggt ttctcacatc gaagtagact gcattccagc 30060
   cttcacagtc tatttgcttt acggatttgt caccctcacg ctcatctgca gcctcatcac 30120
   tgtggtcatc gcctttatcc agtgcattga ctgggtctgt gtgcgctttg catatctcag 30180
   acaccatccc cagtacaggg acaggactat agctgagctt cttagaattc tttaattatg 30240
   aaatttactg tgacttttct gctgattatt tgcaccctat ctgcgttttg ttccccgacc 30300
   tccaagcctc aaagacatat atcatgcaga ttcactcgta tatggaatat tccaagttgc 30360
   tacaatgaaa aaagcgatct ttccgaagcc tggttatatg caatcatctc tgttatggtg 30420
   ttctgcagta ccatcttagc cctagctata tatccctacc ttgacattgg ctggaaacga 30480
   atagatgcca tgaaccaccc aactttcccc gcgcccgcta tgcttccact gcaacaagtt 30540
   gttgccggcg gctttgtccc agccaatcag cctcgcccca cttctcccac ccccactgaa 30600
   atcagctact ttaatctaac aggaggagat gactgacacc ctagatctag aaatggacgg 30660
   aattattaca gagcagcgcc tgctagaaag acgcagggca gcggccgagc aacagcgcat 30720
   gaatcaagag ctccaagaca tggttaactt gcaccagtgc aaaaggggta tcttttgtct 30780
   ggtaaagcag gccaaagtca cctacgacag taataccacc ggacaccgcc ttagctacaa 30840
   gttgccaacc aagcgtcaga aattggtggt catggtggga gaaaagccca ttaccataac 30900
   tcagcactcg gtagaaaccg aaggctgcat tcactcacct tgtcaaggac ctgaggatct 30960
   ctgcaccctt attaagaccc tgtgcggtct caaagatctt attcccttta actaataaaa 31020
   aaaaataata aagcatcact tacttaaaat cagttagcaa atttctgtcc agtttattca 31080
   gcagcacctc cttgccctcc tcccagctct ggtattgcag cttcctcctg gctgcaaact 31140
   ttctccacaa tctaaatgga atgtcagttt cctcctgttc ctgtccatcc gcacccacta 31200
   tcttcatgtt gttgcagatg aagcgcgcaa gaccgtctga agataccttc aaccccgtgt 31260
   atccatatga cacggaaacc ggtcctccaa ctgtgccttt tcttactcct ccctttgtat 31320
   cccccaatgg gtttcaagag agtccccctg gggtactctc tttgcgccta tccgaacctc 31380
   tagttacctc caatggcatg cttgcgctca aaatgggcaa cggcctctct ctggacgagg 31440
   ccggcaacct tacctcccaa aatgtaacca ctgtgagccc acctctcaaa aaaaccaagt 31500
   caaacataaa cctggaaata tctgcacccc tcacagttac ctcagaagcc ctaactgtgg 31560
   ctgccgccgc acctctaatg gtcgcgggca acacactcac catgcaatca caggccccgc 31620
   taaccgtgca cgactccaaa cttagcattg ccacccaagg acccctcaca gtgtcagaag 31680
   gaaagctagc cctgcaaaca tcaggccccc tcaccaccac cgatagcagt acccttacta 31740
   tcactgcctc accccctcta actactgcca ctggtagctt gggcattgac ttgaaagagc 31800
   ccatttatac acaaaatgga aaactaggac taaagtacgg ggctcctttg catgtaacag 31860
   acgacctaaa cactttgacc gtagcaactg gtccaggtgt gactattaat aatacttcct 31920
   tgcaaactaa agttactgga gccttgggtt ttgattcaca aggcaatatg caacttaatg 31980
   tagcaggagg actaaggatt gattctcaaa acagacgcct tatacttgat gttagttatc 32040
   cgtttgatgc tcaaaaccaa ctaaatctaa gactaggaca gggccctctt tttataaact 32100
   cagcccacaa cttggatatt aactacaaca aaggccttta cttgtttaca gcttcaaaca 32160
   attccaaaaa gcttgaggtt aacctaagca ctgccaaggg gttgatgttt gacgctacag 32220
   ccatagccat taatgcagga gatgggcttg aatttggttc acctaatgca ccaaacacaa 32280
   atcccctcaa aacaaaaatt ggccatggcc tagaatttga ttcaaacaag gctatggttc 32340
   ctaaactagg aactggcctt agttttgaca gcacaggtgc cattacagta ggaaacaaaa 32400
   ataatgataa gctaactttg tggaccacac cagctccatc tcctaactgt agactaaatg 32460
   cagagaaaga tgctaaactc actttggtct taacaaaatg tggcagtcaa atacttgcta 32520
   cagtttcagt tttggctgtt aaaggcagtt tggctccaat atctggaaca gttcaaagtg 32580
   ctcatcttat tataagattt gacgaaaatg gagtgctact aaacaattcc ttcctggacc 32640
   cagaatattg gaactttaga aatggagatc ttactgaagg cacagcctat acaaacgctg 32700
   ttggatttat gcctaaccta tcagcttatc caaaatctca cggtaaaact gccaaaagta 32760
   acattgtcag tcaagtttac ttaaacggag acaaaactaa acctgtaaca ctaacgatca 32820
   cactaaacgg tacacaggaa acaggagaca caacttgtga ctgccgcgga gactgtttct 32880
   gcccatctgc atactctatg tcattttcat gggactggtc tggccacaac tacattaatg 32940
   aaatatttgc cacatcctct tacacttttt catacattgc ccaagaataa agaatcgttt 33000
   gtgttatgtt tcaacgtgtt tatttttcaa ttgcagaaaa tttcaagtca tttttcattc 33060
   agtagtatag ccccaccacc acatagctta tacagatcac cgtaccttaa tcaaactcac 33120
   agaaccctag tattcaacct gccacctccc tcccaacaca cagagtacac agtcctttct 33180
   ccccggctgg ccttaaaaag catcatatca tgggtaacag acatattctt aggtgttata 33240
   ttccacacgg tttcctgtcg agccaaacgc tcatcagtga tattaataaa ctccccgggc 33300
   agctcactta agttcatgtc gctgtccagc tgctgagcca caggctgctg tccaacttgc 33360
   ggttgcttaa cgggcggcga aggagaagtc cacgcctaca tgggggtaga gtcataatcg 33420
   tgcatcagga tagggcggtg gtgctgcagc agcgcgcgaa taaactgctg ccgccgccgc 33480
   tccgtcctgc aggaatacaa catggcagtg gtctcctcag cgatgattcg caccgcccgc 33540
   agcataaggc gccttgtcct ccgggcacag cagcgcaccc tgatctcact taaatcagca 33600
   cagtaactgc agcacagcac cacaatattg ttcaaaatcc cacagtgcaa ggcgctgtat 33660
   ccaaagctca tggcggggac cacagaaccc acgtggccat cataccacaa gcgcaggtag 33720
   attaagtggc gacccctcat aaacacgctg gacataaaca ttacctcttt tggcatgttg 33780
   taattcacca cctcccggta ccatataaac ctctgattaa acatggcgcc atccaccacc 33840
   atcctaaacc agctggccaa aacctgcccg ccggctatac actgcaggga accgggactg 33900
   gaacaatgac agtggagagc ccaggactcg taaccatgga tcatcatgct cgtcatgata 33960
   tcaatgttgg cacaacacag gcacacgtgc atacacttcc tcaggattac aagctcctcc 34020
   cgcgttagaa ccatatccca gggaacaacc cattcctgaa tcagcgtaaa tcccacactg 34080
   cagggaagac ctcgcacgta actcacgttg tgcattgtca aagtgttaca ttcgggcagc 34140
   agcggatgat cctccagtat ggtagcgcgg gtttctgtct caaaaggagg tagacgatcc 34200
   ctactgtacg gagtgcgccg agacaaccga gatcgtgttg gtcgtagtgt catgccaaat 34260
   ggaacgccgg acgtagtcat atttcctgaa gcaaaaccag gtgcgggcgt gacaaacaga 34320
   tctgcgtctc cggtctcgcc gcttagatcg ctctgtgtag tagttgtagt atatccactc 34380
   tctcaaagca tccaggcgcc ccctggcttc gggttctatg taaactcctt catgcgccgc 34440
   tgccctgata acatccacca ccgcagaata agccacaccc agccaaccta cacattcgtt 34500
   ctgcgagtca cacacgggag gagcgggaag agctggaaga accatgtttt tttttttatt 34560
   ccaaaagatt atccaaaacc tcaaaatgaa gatctattaa gtgaacgcgc tcccctccgg 34620
   tggcgtggtc aaactctaca gccaaagaac agataatggc atttgtaaga tgttgcacaa 34680
   tggcttccaa aaggcaaacg gccctcacgt ccaagtggac gtaaaggcta aacccttcag 34740
   ggtgaatctc ctctataaac attccagcac cttcaaccat gcccaaataa ttctcatctc 34800
   gccaccttct caatatatct ctaagcaaat cccgaatatt aagtccggcc attgtaaaaa 34860
   tctgctccag agcgccctcc accttcagcc tcaagcagcg aatcatgatt gcaaaaattc 34920
   aggttcctca cagacctgta taagattcaa aagcggaaca ttaacaaaaa taccgcgatc 34980
   ccgtaggtcc cttcgcaggg ccagctgaac ataatcgtgc aggtctgcac ggaccagcgc 35040
   ggccacttcc ccgccaggaa ccatgacaaa agaacccaca ctgattatga cacgcatact 35100
   cggagctatg ctaaccagcg tagccccgat gtaagcttgt tgcatgggcg gcgatataaa 35160
   atgcaaggtg ctgctcaaaa aatcaggcaa agcctcgcgc aaaaaagaaa gcacatcgta 35220
   gtcatgctca tgcagataaa ggcaggtaag ctccggaacc accacagaaa aagacaccat 35280
   ttttctctca aacatgtctg cgggtttctg cataaacaca aaataaaata acaaaaaaac 35340
   atttaaacat tagaagcctg tcttacaaca ggaaaaacaa cccttataag cataagacgg 35400
   actacggcca tgccggcgtg accgtaaaaa aactggtcac cgtgattaaa aagcaccacc 35460
   gacagctcct cggtcatgtc cggagtcata atgtaagact cggtaaacac atcaggttga 35520
   ttcacatcgg tcagtgctaa aaagcgaccg aaatagcccg ggggaataca tacccgcagg 35580
   cgtagagaca acattacagc ccccatagga ggtataacaa aattaatagg agagaaaaac 35640
   acataaacac ctgaaaaacc ctcctgccta ggcaaaatag caccctcccg ctccagaaca 35700
   acatacagcg cttccacagc ggcagccata acagtcagcc ttaccagtaa aaaagaaaac 35760
   ctattaaaaa aacaccactc gacacggcac cagctcaatc agtcacagtg taaaaaaggg 35820
   ccaagtgcag agcgagtata tataggacta aaaaatgacg taacggttaa agtccacaaa 35880
   aaacacccag aaaaccgcac gcgaacctac gcccagaaac gaaagccaaa aaacccacaa 35940
   cttcctcaaa tcgtcacttc cgttttccca cgttacgtca cttcccattt taagaaaact 36000
   acaattccca acacatacta gttactccgc cctaaaacct acgtcacccg ccccgttccc 36060
   acgccccgcg ccacgtcaca aactccaccc cctcattatc atattggctt caatccaaaa 36120
   taaggtatat tattgatgat g 36141
<210> 5
   <211> 50
   <212> DNA
   <213> Artificial Sequence
<220>
   <223> SplF oligonucleotide
<400> 5
   gtacgtcgac cacaaacccc gcccagcgtc ttgtcattgg cgtcgacgct 50
<210> 6
   <211> 50
   <212> DNA
   <213> Artificial Sequence
<220>
   <223> SplR oligonucleotide
<400> 6
   gtacagcgtc gacgccaatg acaagacgct gggcggggtt tgtggtcgac 50
<210> 7
   <211> 63
   <212> DNA
   <213> Artificial Sequence
<220>
   <223> E2FF2 oligonucleotide
<400> 7
   gtacgtcggc ggctcgtggc tctttcgcgg caaaaaggat ttggcgcgta aaagtggttc 60
   gaa 63
<210> 8
   <211> 60
   <212> DNA
   <213> Artificial Sequence
<220>
   <223> E2FR2 oligonucleotide
<400> 8
   gtacttcgaa ccacttttac gcgccaaatc ctttttgccg cgaaagagcc acgagccgcc 60
<210> 9
   <211> 576
   <212> PRT
   <213> Artificial Sequence
<220>
   <223> Amino acid sequence, amino acids 1-582 of the modified version of the fibre of adenovirus serotype 5 in which RGDK modification has been introduced.
<400> 9
   Met Lys Arg Ala Arg Pro Ser Glu Asp Thr Phe Asn Pro Val Tyr Pro
   1               5                   10                  15
   Tyr Asp Thr Glu Thr Gly Pro Pro Thr Val Pro Phe Leu Thr Pro Pro
               20                  25                  30
   Phe Val Ser Pro Asn Gly Phe Gln Glu Ser Pro Pro Gly Val Leu Ser
           35                  40                  45
   Leu Arg Leu Ser Glu Pro Leu Val Thr Ser Asn Gly Met Leu Ala Leu
       50                  55                  60
   Lys Met Gly Asn Gly Leu Ser Leu Asp Glu Ala Gly Asn Leu Thr Ser
   65                  70                  75                  80
   Gln Asn Val Thr Thr Val Ser Pro Pro Leu Arg Gly Asp Lys Ser Asn
                   85                  90                  95
   Ile Asn Leu Glu Ile Ser Ala Pro Leu Thr Val Thr Ser Glu Ala Leu
               100                 105                 110
   Thr Val Ala Ala Ala Ala Pro Leu Met Val Ala Gly Asn Thr Leu Thr
           115                 120                 125
   Met Gly Ser Gln Ala Pro Leu Thr Val His Asp Ser Lys Leu Ser Ile
       130                 135                 140
   Ala Thr Gln Gly Pro Leu Thr Val Ser Glu Gly Lys Leu Ala Leu Gln
   145                 150                 155                 160
   Thr Ser Gly Pro Leu Thr Thr Thr Asp Ser Ser Thr Leu Thr Ile Thr
                   165                 170                 175
   Ala Ser Pro Pro Leu Thr Thr Ala Thr Gly Ser Leu Gly Ile Asp Leu
               180                 185                 190
   Lys Glu Pro Ile Tyr Thr Gln Asn Gly Lys Leu Gly Leu Lys Tyr Gly
           195                 200                 205
   Ala Pro Leu His Val Thr Asp Asp Leu Asn Thr Leu Thr Val Ala Thr
       210                 215                 220
   Gly Pro Gly Val Thr Ile Asn Asn Thr Ser Leu Gly Thr Lys Val Thr
   225                 230                 235                 240
   Gly Ala Leu Gly Phe Asp Ser Gln Gly Asn Met Gln Leu Asn Val Ala
                   245                 250                 255
   Gly Gly Leu Arg Ile Asp Ser Gln Asn Arg Arg Leu Ile Leu Asp Val
               260                 265                 270
   Ser Tyr Pro Phe Asp Ala Gln Asn Gln Leu Asn Leu Arg Leu Gly Gln
           275                 280                 285
   Gly Pro Leu Phe Ile Asn Ser Ala His Asn Leu Asp Ile Asn Tyr Asn
       290                 295                 300
   Lys Gly Leu Tyr Leu Phe Thr Ala Ser Asn Asn Ser Lys Lys Leu Glu
   305                 310                 315                 320
   Val Asn Leu Ser Thr Ala Lys Gly Leu Met Phe Asp Ala Thr Ala Ile
                   325                 330                 335
   Ala Ile Asn Ala Gly Asp Gly Leu Glu Phe Gly Ser Pro Asn Ala Pro
               340                 345                 350
   Asn Thr Asn Pro Leu Lys Thr Lys Ile Ile Gly His Gly Leu Glu Phe
           355                 360                 365
   Asp Ser Asn Lys Ala Met Val Pro Lys Leu Gly Thr Gly Leu Ser Phe
   370 375 380
   Asp Ser Thr Gly Ala Ile Thr Val Gly Asn Lys Asn Asn Asp Lys Leu
   385 390 395 400
   Thr Leu Trp Thr Thr Pro Ala Pro Ser Pro Asn Cys Asp Leu Asn Ala
   405 410 415
   Glu Lys Asp Ala Lys Leu Thr Leu Val Leu Thr Lys Cys Gly Ser Gln
   420 425 430
   Ile Leu Ala Thr Val Ser Val Leu Ala Val Lys Gly Ser Leu Ala Pro
   435 440 445
   Ile Ser Gly Thr Val Gln Ser Ala His Leu Ile Ile Arg Phe Asp Glu
   450 455 460
   Asn Gly Val Leu Leu Asn Asn Ser Phe Leu Asp Pro Glu Tyr Trp Asn
   465 470 475 480
   Phe Arg Asn Gly Asp Leu Thr Glu Gly Thr Ala Tyr Thr Asn Ala Val
   485 490 495
   Gly Phe Met Pro Asn Leu Ser Ala Tyr Pro Lys Ser His Gly Lys Thr
   500 505 510
   Ala Lys Ser Asn Ile Val Ser Gln Val Tyr Leu Asn Gly Asp Lys Thr
   515 520 525
   Lys Pro Val Thr Leu Thr Ile Thr Leu Asn Gly Thr Gln Glu Thr Gly
   530 535 540
   Asp Thr Thr Pro Ser Ala Tyr Ser Met Ser Phe Ser Trp Asp Trp Ser
   545 550 555 560
   Gly His Asn Tyr Ile Asn Glu Ile Phe Ala Thr Ser Ser Tyr Thr Phe
   565 570 575
<210> 10
   <211> 9
   <212> PRT
   <213> Artificial Sequence
<220>
   <223> RGD-4C peptdie
<400> 10
   Cys Asp Cys Arg Gly Asp Cys Phe Cys
   1               5
<210> 11
   <211> 23
   <212> DNA
   <213> Artificial Sequence
<220>
   <223> Forward primer for CRX
<400> 11
   aggtggctct gaagatcaat ctg 23
<210> 12
   <211> 20
   <212> DNA
   <213> Artificial Sequence
<220>
   <223> Reverse primer for CRX
<400> 12
   ttagccctcc ggttcttgaa 20
<210> 13
   <211> 15
   <212> DNA
   <213> Artificial Sequence
<220>
   <223> Probe for CRX
<400> 13
   ctgagtccag ggttc 15
<210> 14
   <211> 24
   <212> DNA
   <213> Artificial Sequence
<220>
   <223> Forward primer for TBP
<400> 14
   gaacatcatg gatcagaaca acag 24
<210> 15
   <211> 23
   <212> DNA
   <213> Artificial Sequence
<220>
   <223> Reverse primer for TBP
<400> 15
   attggtgttc tgaataggct gtg 23
<210> 16
   <211> 25
   <212> DNA
   <213> Artificial Sequence
<220>
   <223> Probe for TBP
<400> 16
   ctgccacctt acgctcaggg cttgg 25
<210> 17
   <211> 37634
   <212> DNA
   <213> Artificial Sequence
<220>
   <223> Complete sequence of ICOVIR17
<400> 17